# EUROPEAN PATENT SPECIFICATION

(11) **EP 3 359 572 B1**
(45) Date of publication and mention of the grant of the patent: **27.11.2024**
(21) Application number: 16790464.8
(22) Date of filing: 06.10.2016
(51) Int. Cl.: C07K 16/28, A61P 25/00

(54) **METHOD FOR TREATING MULTIPLE SCLEROSIS**
VERFAHREN ZUR BEHANDLUNG VON MULTIPLER SKLEROSE
MÉTHODE DE TRAITEMENT DE LA SCLÉROSE EN PLAQUES

(30) Priority: 06.10.2015 US 201562238103 P; 07.10.2015 US 201562238674 P; 28.12.2015 US 201562271985 P; 16.02.2016 US 201662296049 P; 14.04.2016 US 201662322734 P; 27.05.2016 US 201662342633 P; 27.06.2016 US 201662355299 P
(43) Date of publication of application: 15.08.2018
(73) Proprietor: F. Hoffmann-La Roche AG, 4070 Basel (CH)
(72) Inventor: LEPPERT, David, 4104 Oberwil (CH); LI-KWAI-CHEUNG, Anne-Marie, 4070 Basel (CH); LIBONATI, Michele, South San Francisco, CA 94080-4990 (US); MASTERMAN, Donna, South San Francisco, CA 94080-4990 (US); PFEFEN, Jean-Paul, 4070 Basel (CH); SMITH, Craig, South San Francisco, CA 94080-4990 (US); WEISSKOPF, Algirdas Jonas, Kakarieka, 4070 Basel (CH); ZHANG, Jiameng, South San Francisco, CA 94080-4990 (US); CHIN, Peter, S., South San Francisco, CA 94080-4990 (US); GARREN, Hideki, 4070 Basel (CH)
(74) Representative: Vossius & Partner Patentanwälte Rechtsanwälte mbB
(86) International application number: PCT/US2016/055841
(87) International publication number: WO 2017/062682

(56) References cited:
- ROMMER PAULUS S ET AL: "Rituximab for secondary progressive multiple sclerosis: a case series", CNS DRUGS, ADIS INTERNATIONAL, AUCKLAND, NZ, vol. 25, no. 7, 29 June 2011 (2011-06-29), pages 607 - 613, XP008157975, ISSN: 1172-7047, DOI: 10.2165/11589390-000000000-00000
- SABEEN LULU ET AL: "Humoral-Targeted Immunotherapies in Multiple Sclerosis", NEUROTHERAPEUTICS, vol. 10, no. 1, 1 January 2013 (2013-01-01), US, pages 34 - 43, XP055333733, ISSN: 1933-7213, DOI: 10.1007/s13311-012-0164-3
- ZHANG JIAMENG ET AL: "EDSS variability before randomization may limit treatment discovery in primary progressive MS", MULTIPLE SCLEROSIS JOURNAL, vol. 19, no. 6, 1 May 2013 (2013-05-01), US, pages 775 - 781, XP055841737, ISSN: 1352-4585, Retrieved from the Internet <URL:https://journals.sagepub.com/doi/pdf/10.1177/1352458512459685> DOI: 10.1177/1352458512459685
- ONTANEDA DANIEL ET AL: "Progressive multiple sclerosis", CURRENT OPINION IN NEUROLOGY, RAPID SCIENCE PUBLISHERS, LONDON, GB, vol. 28, no. 3, 1 June 2015 (2015-06-01), pages 237 - 243, XP009192987, ISSN: 1350-7540, DOI: 10.1097/WCO.0000000000000195
- ZHANG JIAMENG ET AL: "EDSS variability before randomization may limit treatment discovery in primary progressive MS", MULTIPLE SCLEROSIS JOURNAL, vol. 19, no. 6, 1 May 2013 (2013-05-01), US, pages 775 - 781, XP055841737, ISSN: 1352-4585, Retrieved from the Internet <URL:https://journals.sagepub.com/doi/pdf/10.1177/1352458512459685> DOI: 10.1177/1352458512459685
- GENENTECH ET AL: "Genentech: Press Releases | Phase II Study Showed Ocrelizumab Maintained Significant Reduction in Disease Activity for Multiple Sclerosis Patients for Almost Two Years", 19 October 2011 (2011-10-19), XP055203108, Retrieved from the Internet <URL:http://www.gene.com/media/press-releases/13627/2011-10-19/phase-ii-study-showed-ocrelizumab-mainta> [retrieved on 20150717]
- L. KAPPOS ET AL.: "Ocrelizumab in relapsing-remitting multiple sclerosis: a phase 2, randomised, placebo-controlled, multicentre trial", LANCET, 19 November 2011 (2011-11-19), pages 1779 - 87, XP055333854, Retrieved from the Internet <URL:http://www.sciencedirect.com/science/article/pii/S0140673611616498/pdfft?md5=158b2ed9ca46655cb0f78883fa5517c2&pid=1-s2.0-S0140673611616498-main.pdf> [retrieved on 20170110], DOI: 10.1016/S0140-
- J. L. JONES ET AL: "Improvement in disability after alemtuzumab treatment of multiple sclerosis is associated with neuroprotective autoimmunity", BRAIN., vol. 133, no. 8, 21 July 2010 (2010-07-21), GB, pages 2232 - 2247, XP055334857, ISSN: 0006-8950, DOI: 10.1093/brain/awq176
- SIDDHARAMA PAWATE ET AL: "Newer Agents in the Treatment of Multiple Sclerosis", NEUROLOGIST, vol. 19, no. 4, 1 April 2015 (2015-04-01), US, pages 104 - 117, XP055333727, ISSN: 1074-7931, DOI: 10.1097/NRL.0000000000000020
- F HOFFMANN-LA ROCHE: "Media Release: Roche's ocrelizumab first investigational medicine to show positive pivotal study results in both relapsing and primary progressive forms of multiple sclerosis", 8 October 2015 (2015-10-08), XP055334454, Retrieved from the Internet <URL:http://www.roche.com/dam/jcr:8e278040-1e67-4e30-9ef7-972833f56c51/en/med-cor-2015-10-08-e.pdf> [retrieved on 20170111]

## Description

### FIELD OF THE INVENTION

The present invention concerns an anti-CD20 antibody, which is ocrelizumab, for use in a method of improving functional ability in a human patient having multiple sclerosis, as further defined in the claims.

### BACKGROUND OF THE INVENTION

Multiple Sclerosis (MS) is an inflammatory and demyelinating degenerative disease of the human central nervous system (CNS). It is a worldwide disease that affects approximately 300,000 persons in the United States; it is a disease of young adults, with 70%-80% having onset between 20 and 40 years old (Anderson et al. Ann Neurology 31(3):333-6 (1992); Noonan et al. Neurology 58:136-8 (2002)). MS is a heterogeneous disorder based on clinical course, magnetic resonance imaging (MRI) scan assessment, and pathology analysis of biopsy and autopsy material (Lucchinetti et al. Ann Neurol 47:707-17 (2000)). The disease manifests itself in a large number of possible combinations of deficits, including spinal cord, brainstem, cranial nerve, cerebellar, cerebral, and cognitive syndromes. Progressive disability is the fate of most patients with MS, especially when a 25-year perspective is included. Half of MS patients require a cane to walk within 15 years of disease onset. MS is a major cause of neurologic disability in young and middle-aged adults and, until the past decade, has had no known beneficial treatments. MS is difficult to diagnose because of the non-specific clinical findings, which led to the development of highly structured diagnostic criteria that include several technological advances, consisting of MRI scans, evoked potentials, and cerebrospinal fluid (CSF) studies. All diagnostic criteria rely upon the general principles of scattered lesions in the central white matter occurring at different times and not explained by other etiologies such as infection, vascular disorder, or autoimmune disorder (McDonald et al. Ann Neurol 50:121-7 (2001)). MS has four patterns of disease: relapsing-remitting MS (RRMS; 80%-85% of cases at onset), primary progressive MS (PPMS; 10%-15% at onset), progressive relapsing MS (PRMS; 5% at onset); and secondary progressive MS (SPMS) (Kremenchutzky et al. Brain 122 (Pt 10):1941-50 (1999); Confavreux et al. N Engl J Med 343(20):1430-8 (2000)). An estimated 50% of patients with RRMS will develop SPMS in 10 years, and up to 90% of RRMS patients will eventually develop SPMS (Weinshenker et al. Brain 112(Pt 1): 133-46 (1989)).

Several disease modifying drugs in five classes are approved in the United States for the treatment of RRMS, whereas no drugs have been approved for PPMS. The RRMS treatments include the following: interferon class, IFN-beta-1a (REBIF^{®}, Extavia, AVONEX^{®} and PLEGRIDY^{™} and IFN-beta-1b (BETASERON^{®}); glatiramer acetate (COPAXONE^{®}), a polypeptide; natalizumab (TYSABRI^{®}), alemtuzumab (LEMTRADA^{®}), both monoclonal antibodies; dimethyl fumarate (TECFIDERA^{®}) and fingolimod (GILENYA^{®}), both small molecules, and mitoxantrone (NOVANTRONE^{®}), a cytotoxic agent; teriflunomide (AUBAGIO^{®}). Other drugs with an aim of disease modification have been used with varying degrees of success, including methotrexate, cyclophosphamide, azathioprine, and intravenous (IV) immunoglobulin. Current therapies lack sufficient efficacy to quell early disease or are not used in early MS due to associated side effects (Hartung et al. (2011) Expert Rev Neurother. 11, 351-62; Freedman et al., Mult. Scler. Relat. Disord. 3(2):147-55, 2014). Thus, there remains a need for highly effective therapies with an acceptable benefit-risk profile that can be given early in the course of disease to decrease the long-term consequences of accumulating disability and improve the quality of life of patients.

### BRIEF SUMMARY OF THE INVENTION

The present invention relates to an anti-CD20 antibody for use in a method of improving functional ability in a human patient having multiple sclerosis comprising administering to the patient an effective amount of the anti-CD20 antibody, wherein the patient has improvement in functional ability after treatment; wherein the anti-CD20 antibody is ocrelizumab; wherein the multiple sclerosis is a primary progressive multiple sclerosis (PPMS) with T1 gadolinium staining lesions; and wherein the improvement in functional ability is measured by the Timed 25-Foot Walk (T-25FW) test or EDSS score.

The invention is defined by the appended claims. Any references in the description to methods of treatment refer to the compounds, pharmaceutical compositions and medicaments of the present invention for use in a method for treatment of the human (or animal) body by therapy (or for diagnosis).

In some embodiments, the patient has 12-week confirmed disability improvement after treatment. In some embodiments, the patient has 24-week confirmed disability improvement after treatment. In some embodiments, the improvement in functional ability in the patient is sustained for at least 12 weeks. In some embodiments, the improvement in functional ability in the patient is sustained for at least 24 weeks. As explained above, the improvement in functional ability is measured by the Timed 25-Foot Walk (T-25FW) test or EDSS score. In some embodiments, the improvement in functional ability is measured by the Timed 25-Foot Walk (T-25FW) test and EDSS score.

In accordance with the above, the invention relates to an anti-CD20 antibody for use in a method of improving functional ability in a human patient having multiple sclerosis, as further defined herein above and in the claims, wherein the patient has T1 gadolinium staining lesions at baseline.

In some embodiments, the anti-CD20 antibody is administered to the patient to provide an initial anti-CD20 antibody exposure followed by a second anti-CD20 antibody exposure, wherein the first and second exposures are each about 600 mg of the antibody, and wherein the interval between the first exposure and the second exposure is about 20-24 weeks or about 5-6 months. In some embodiments, the anti-CD20 antibody is administered to the patient to provide a third anti-CD20 antibody exposure, wherein the third exposure is about 600 mg of the antibody, and wherein the interval between the second exposure and the third exposure is about 20-24 weeks or about 5-6 months. In some embodiments, the anti-CD20 antibody is administered to the patient to provide a fourth anti-CD20 antibody exposure, wherein the fourth exposure is about 600 mg of the antibody, and wherein the interval between the third exposure and the fourth exposure is about 20-24 weeks or about 5-6 months.

In some embodiments, the first exposure comprises a first dose and a second dose of the anti-CD20 antibody, wherein each dose is about 300 mg and the first dose and the second dose are separated by about two weeks or about 14 days (such as 13 days or 15 days). In some embodiments "about 14 days" refers to a variation of 1 day before or after the 14^{th} day. In some embodiments, the second, third, and/or fourth exposures comprise a single dose of about 600 mg. In some embodiments, the initial exposure and second, third, and/or fourth additional exposures comprise a first dose and a second dose of the anti-CD20 antibody, wherein each dose is about 300 mg and the first dose and the second dose are separated by about two weeks or about 14 days (such as 13 days or 15 days).

In some embodiments, the patient has improved functional ability after one, two, three, and/or four exposures of the anti-CD20 antibody.

In certain embodiments, the patient maintains the ability to mount a humoral response to an antigen during treatment. In certain embodiments, the antigen is a mumps antigen, a rubella antigen, a varicella antigen, an S. pneumonia antigen, a tetanus toxoid antigen, a pneumococcal antigen, or an influenza antigen.

In certain embodiments, the patient is premedicated prior to infusion with the anti-CD20 antibody. In certain embodiments, the patient is premedicated with methylprednisolone (or an equivalent) approximately 30 minutes prior to each infusion of anti-CD20 antibody. In certain embodiments, the patient is premedicated with 100 mg IV methylprednisolone (or an equivalent) approximately 30 minutes prior to each infusion of anti-CD20 antibody. In certain embodiments, the patient is additionally (or alternatively) premedicated with an antihistaminic drug (e.g. diphenhydramine) approximately 30-60 minutes before each infusion of anti-CD20 antibody. In certain embodiments, the patient is additionally (or alternatively) premedicated with an antipyretic (e.g. acetaminophen/paracetamol).

In some embodiments, a second medicament is administered with the initial exposure or later exposures, wherein the anti-CD20 antibody is a first medicament. In some embodiments, the second medicament is selected from the group consisting of an interferon, glatiramer acetate, a cytotoxic agent, chemotherapeutic agent, mitoxantrone, methotrexate, cyclophosphamide, chlorambucil, azathioprine, gamma globulin, Campath, anti-CD4, cladribine, corticosteroid, mycophenolate mofetil (MMF), cyclosporine, cholesterol-lowering drug of the statin class, estradiol, testosterone, hormone replacement drug, a TNF inhibitor, disease modifying anti-rheumatic drug (DMARD), non-steroidal anti-inflammatory drug (NSAID), levothyroxine, cyclosporin A, somatostatin analogue, cytokine or cytokine receptor antagonist, antimetabolite, immunosuppressive agent, integrin antagonist or antibody, LFA-1 antibody, efalizumab, alpha 4 integrin antibody, natalizumab, and another B-cell surface marker antibody.

As explained above, the multiple sclerosis is a primary progressive multiple sclerosis (PPMS).

As explained before, the invention relates to an anti-CD20 antibody for use in a method of improving functional ability in a human patient having multiple sclerosis, wherein the multiple sclerosis is a primary progressive multiple sclerosis (PPMS) with T1 gadolinium staining lesions, as further defined herein above and in the claims. In line with this, in certain embodiments, the patient is selected for treatment based upon having PPMS. In certain embodiments, treatment is based upon the patient having PPMS. In certain embodiments, the patient is diagnosed with PPMS prior to treatment.

In some embodiments, the anti-CD20 antibody is administered to the patient to provide an initial anti-CD20 antibody exposure followed by one or more additional anti-CD20 antibody exposures, wherein each exposure is about 600 mg of the antibody, and each exposure is provided to the patient as one or two doses of the anti-CD20 antibody, and wherein the interval between each exposure is about 20-24 weeks or about 5-6 months. In some embodiments, "about 20-24 weeks" refers to a time point between 20 weeks and 24 weeks. In some embodiments, "about 20-24 weeks" refers to a variation of a week or 7 days before or after the 24^{th} week. In some embodiments, "about 5-6 months" refers to a time point between 5 and 6 months.

In some embodiments, the first exposure comprises a first dose and a second dose of the anti-CD20 antibody, wherein each dose is about 300 mg and the first dose and the second dose are separated by about two weeks or about 14 days (such as 13 days or 15 days). In some embodiments "about 14 days" refers to a variation of 1 day before or after the 14^{th} day. In some embodiments, the second, third, and/or fourth exposures comprise a single dose of about 600 mg. In some embodiments, the initial exposure and second, third, and/or fourth additional exposures comprise a first dose and a second dose of the anti-CD20 antibody, wherein each dose is about 300 mg and the first dose and the second dose are separated by about two weeks or about 14 days (such as 13 days or 15 days).

In certain embodiments according to (or as applied to) any of the embodiments above, the anti-CD20 antibody is administered to the patient to provide an initial anti-CD20 antibody exposure followed by a second anti-CD20 antibody exposure, wherein the first and second exposures are each about 600 mg of the antibody, and wherein the interval between the first exposure and the second exposure is about 20-24 weeks or about 5-6 months. In certain embodiments according to (or as applied to) any of the embodiments above, the anti-CD20 antibody is administered to the patient to provide a third anti-CD20 antibody exposure, wherein the third exposure is about 600 mg of the antibody, and wherein the interval between the second exposure and the third exposure is about 20-24 weeks or about 5-6 months. In certain embodiments according to (or as applied to) any of the embodiments above, the anti-CD20 antibody is administered to the patient to provide a fourth anti-CD20 antibody exposure, wherein the fourth exposure is about 600 mg of the antibody, and wherein the interval between the third exposure and the fourth exposure is about 20-24 weeks or about 5-6 months.

In some embodiments according to (or as applied to) any of the embodiments above, the first exposure comprises a first dose and a second dose of the anti-CD20 antibody, wherein each dose is about 300 mg and the first dose and the second dose are separated by about two weeks or about 14 days (such as 13 days or 15 days). In some embodiments "about 14 days" refers to a variation of 1 day before or after the 14^{th} day. In certain embodiments according to (or as applied to) any of the embodiments above, the first and second doses are administered intravenously. In certain embodiments according to (or as applied to) any of the embodiments above, the first and second doses each comprise 250 ml of anti-CD20 antibody at a concentration of about 1.2 mg/ml. In certain embodiments according to (or as applied to) any of the embodiments above, the first and second doses are each infused at a rate of 30 ml/hour. In certain embodiments according to (or as applied to) any of the embodiments above, the infusion rate of the first and second doses can be increased in 30 ml/hour increments to a maximum rate of 180 ml/hr. In certain embodiments according to (or as applied to) any of the embodiments above, the first and second doses are each be given over approximately 2.5 hours.

In some embodiments, the second, third, and/or fourth exposures comprise a single dose of about 600 mg. In certain embodiments according to (or as applied to) any of the embodiments above, the second, third, and/or fourth exposures are administered intravenously. In certain embodiments according to (or as applied to) any of the embodiments above, the second, third, and/or fourth exposures each comprise 500 ml of anti-CD20 antibody at a concentration of about 1.2 mg/ml. In certain embodiments according to (or as applied to) any of the embodiments above, the second, third, and/or fourth exposures are each infused at a rate of 40 ml/hour. In certain embodiments according to (or as applied to) any of the embodiments above, the infusion rate of the second, third, and/or fourth exposures can be increased in 40 ml/hour increments to a maximum rate of 200 ml/hr. In certain embodiments according to (or as applied to) any of the embodiments above, the first and second doses are each be given over approximately 3.5 hours.

In some embodiments, the initial exposure and second, third, and/or fourth additional exposures comprise a first dose and a second dose of the anti-CD20 antibody, wherein each dose is about 300 mg and the first dose and the second dose are separated by about two weeks or about 14 days (such as 13 days or 15 days). In some embodiments "about 14 days" refers to a variation of 1 day before or after the 14^{th} day. In certain embodiments according to (or as applied to) any of the embodiments above, the first and second doses are administered intravenously. In certain embodiments according to (or as applied to) any of the embodiments above, the first and second doses each comprise 250 ml of anti-CD20 antibody at a concentration of about 1.2 mg/ml. In certain embodiments according to (or as applied to) any of the embodiments above, the first and second doses are each infused at a rate of 30 ml/hour. In certain embodiments according to (or as applied to) any of the embodiments above, the infusion rate of the first and second doses can be increased in 30 ml/hour increments to a maximum rate of 180 ml/hr. In certain embodiments according to (or as applied to) any of the embodiments above, the first and second doses are each be given over approximately 2.5 hours.

In certain embodiments, the patient receives at least 2, 3, 4, or more than 4 anti-CD20 antibody exposures.

In some embodiments, the anti-CD20 antibody is administered to the patient to provide one or more additional anti-CD20 antibody exposure after the fourth exposure, wherein the one or more additional exposure is about 600 mg of the antibody, and wherein the interval between the fourth exposure and the additional exposure is about 20-24 weeks or about 5-6 months. In some embodiments, "about 20-24 weeks" refers to a time point between 20 weeks and 24 weeks. In some embodiments, "about 20-24 weeks" refers to a variation of a week or 7 days before or after the 24^{th} week. In some embodiments, "about 5-6 months" refers to a time point between 5 and 6 months. In some embodiments, the interval between each of the additional exposures following the fourth exposure is 20-24 weeks or about 5-6 months. In some embodiments, the one or more exposures comprise a first dose and a second dose of the anti-CD20 antibody, wherein each dose is about 300 mg and the first dose and the second dose are separated by about 14 days (such as 13 days or 15 days). In some embodiments "about 14 days" refers to a variation of 1 day before or after the 14^{th} day. In some embodiments, the one or more exposures comprise a first dose and a second dose of the anti-CD20 antibody, wherein each dose is about 300 mg and the first dose and the second dose are separated by about 14 days (such as 13 days or 15 days).

As explained above, the anti-CD20 antibody to be used in accordance with the invention is ocrelizumab. In some embodiments, the anti-CD20 antibody is in a pharmaceutically acceptable composition. In some embodiments, the anti-CD20 antibody is in a formulation comprising 30 mg/mL antibody, 20 mM Sodium Acetate, 106 mM Trehalose, 0.02% polysorbate 20, pH 5.3. In some embodiments, the antibody in the formulation is stored at about 2-8°C at 300 mg/vial. In some embodiments, the antibody is diluted in saline (0.9% sodium chloride) in an IV bag for administration by infusion.

In some embodiments, the anti-CD20 antibody is administered intravenously. In some embodiments, the anti-CD20 antibody is administered intravenously for each antibody exposure. In some embodiments, the antibody is administered subcutaneously. In some embodiments, the anti-CD20 antibody is administered subcutaneously for each antibody exposure.

In certain embodiments, a reduction or decrease or improvement after administration of the anti-CD20 antibody can be compared to a baseline level, to a level in untreated patient(s), and/or to a level in patient(s), e.g., such as mean, average, or median level of a group of patients, receiving a different treatment (such as interferon beta-1a or REBIF^{®}).

### BRIEF DESCRIPTION OF THE DRAWINGS

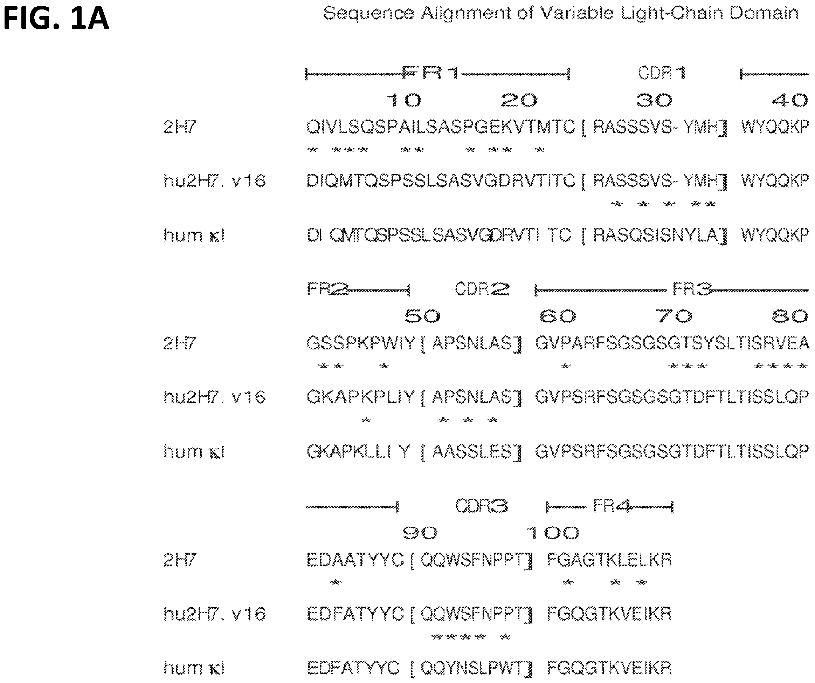

FIG. 1A is a sequence alignment comparing the amino acid sequences of the light chain variable domain (V_{L}) of each of murine 2H7 (SEQ ID NO:1), humanized 2H7.v16 variant (SEQ ID NO:2), and the human kappa light chain subgroup I (SEQ ID NO:3). The CDRs of V_{L} of 2H7 and hu2H7.v16 are as follows: CDR1 (SEQ ID NO:4), CDR2 (SEQ ID NO:5), and CDR3 (SEQ ID NO:6).

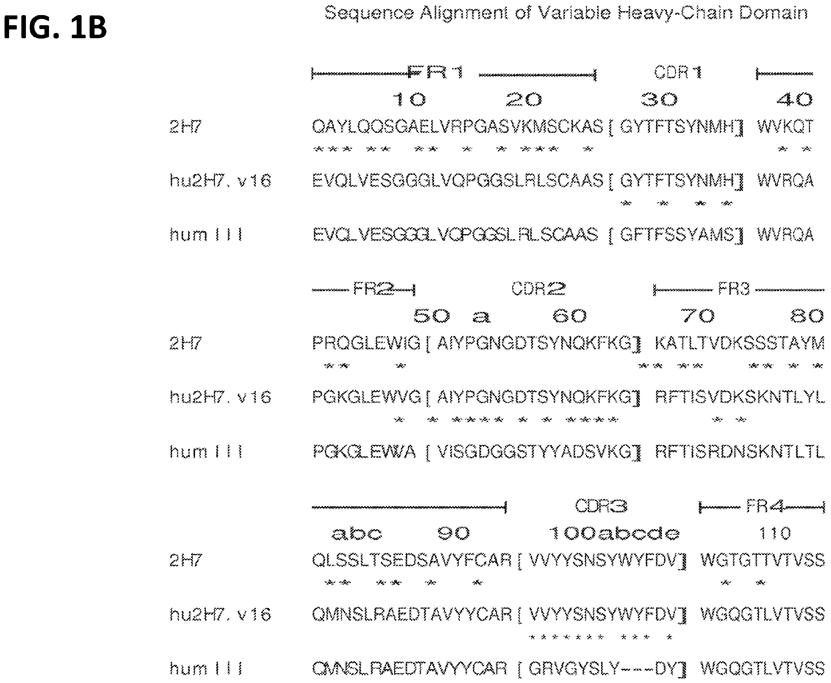

**FIG. 1B** is a sequence alignment comparing the amino acid sequences of the heavy chain variable domain (V_{H}) of each of murine 2H7 (SEQ ID NO:7), humanized 2H7.v16 variant (SEQ ID NO: 8), and the human consensus sequence of the heavy chain subgroup III (SEQ ID NO:9). The CDRs of V_{H} of 2H7 and hu2H7.v16 are as follows: CDR1 (SEQ ID NO: 10), CDR2 (SEQ ID NO:11), and CDR3 (SEQ ID NO:12).
In **FIG. 1A** and **FIG. 1B****,** the CDR1, CDR2 and CDR3 in each chain are enclosed within brackets, flanked by the framework regions, FR1-FR4, as indicated. 2H7 refers to the murine 2H7 antibody. The asterisks in between two rows of sequences indicate the positions that are different between the two sequences. Residue numbering is according to Kabat et al. Sequences of Immunological Interest, 5th Ed. Public Health Service, National Institutes of Health, Bethesda, Md. (1991), with insertions shown as a, b, c, d, and e.
**FIG. 2** shows the amino acid sequence of the mature 2H7.v16 light chain (SEQ ID NO: 13)
**FIG. 3** shows the amino acid sequence of the mature 2H7.v16 heavy chain (SEQ ID NO: 14).
**FIG. 4** shows the amino acid sequence of the mature 2H7.v31 heavy chain (SEQ ID NO: 15). The L chain of 2H7.v31 is the same as for 2H7.v16.

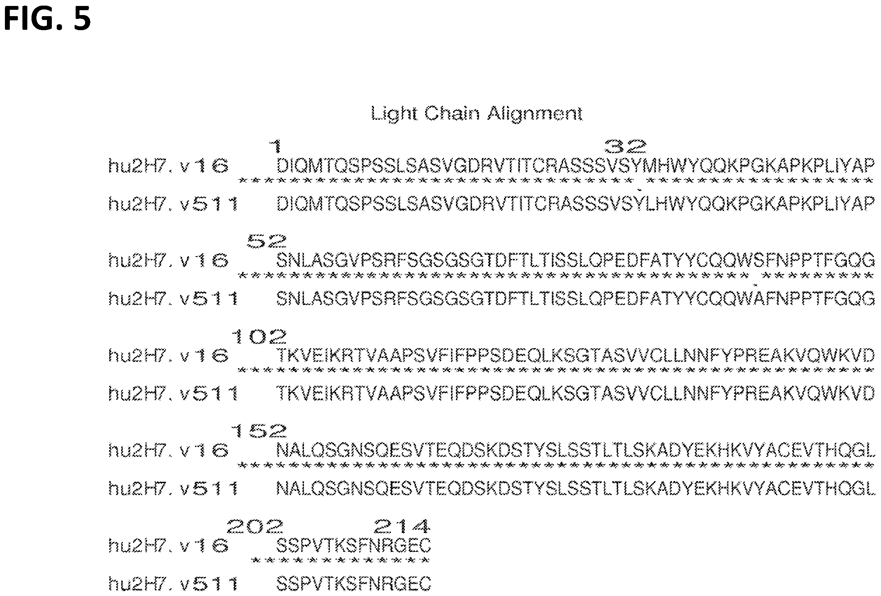

**FIG. 5** shows an alignment of the mature 2H7.v16 and 2H7.v511 light chains (SEQ ID NOS. 13 and 16, respectively), with Kabat variable domain residue numbering and Eu constant domain residue numbering.

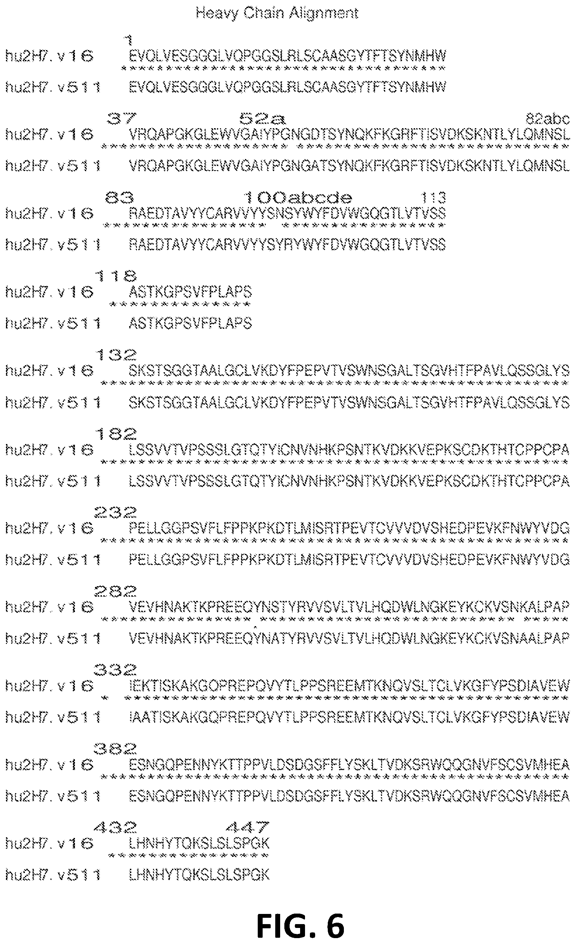

**FIG. 6** shows an alignment of the mature 2H7.v16 and 2H7.v511 heavy chains (SEQ ID NOS. 14 and 17, respectively), with Kabat variable domain residue numbering and Eu constant domain residue numbering.
**FIG. 7** shows an overview of the study design for two identical Phase III studies (i.e., STUDY I and STUDY II) of ocrelizumab in comparison with interferon beta-1a (REBIF^{®}) in patients with relapsing multiple sclerosis.
**FIG. 8** shows the hierarchical statistical analysis plan for STUDY I and STUDY II.
**FIG. 9** shows the patient disposition in STUDY I and STUDY II.
**FIG. 10A** shows the Annualized Relapse Rate (ARR) at 96 weeks for patients treated with ocrelizumab compared to patients treated with interferon beta-1a in STUDY I.
**FIG. 10B** shows the Annualized Relapse Rate (ARR) at 96 weeks for patients treated with ocrelizumab compared to patients treated with interferon beta-1a in STUDY II.
**FIG. 11** shows time to onset of Confirmed Disability Progression (CDP) for at least 12 weeks over a 96 week period in patients treated with ocrelizumab compared to patients treated with interferon beta-1a (pooled data from STUDY I and STUDY II).
**FIG. 12A** shows time to onset of Confirmed Disability Progression for at least 12 weeks over a 96 week period in patients treated with ocrelizumab compared to patients treated with interferon beta-1a in STUDY I.
**FIG. 12B** shows time to onset of Confirmed Disability Progression for at least 12 weeks over a 96 week period in patients treated with ocrelizumab compared to patients treated with interferon beta-1a in STUDY II.
**FIG. 13** shows time to onset of Confirmed Disability Progression for at least 24 weeks over a 96 week period in patients treated with ocrelizumab compared to patients treated with interferon beta-1a (pooled data from STUDY I and STUDY II).
**FIG. 14A** shows time to onset of Confirmed Disability Progression for at least 24 weeks over a 96 week period in patients treated with ocrelizumab compared to patients treated with interferon beta-1a in STUDY I.
**FIG. 14B** shows time to onset of Confirmed Disability Progression for at least 24 weeks over a 96 week period in patients treated with ocrelizumab compared to patients treated with interferon beta-1a in STUDY II.
**FIG. 15A** shows proportion of patients (with baseline EDSS score of 2.0 or more) with confirmed disability improvement (i.e., CDI) for at least 12 weeks in patients receiving ocrelizumab compared to patients receiving interferon beta-1a (the pooled data from STUDY I and STUDY II).
**FIG. 15B** shows proportion of patients (with baseline EDSS score of 2.0 or more) with confirmed disability improvement (i.e., CDI) for at least 24 weeks in patients receiving ocrelizumab compared to patients receiving interferon beta-1a (the pooled data from STUDY I and STUDY II).
**FIG. 16A** shows proportion of patients (with baseline EDSS score of 2.0 or more) with confirmed disability improvement (i.e., CDI) for at least 12 weeks in patients receiving ocrelizumab compared to patients receiving interferon beta-1a in STUDY I.
**FIG. 16B** shows the proportion of patients (with baseline EDSS score of 2.0 or more) with confirmed disability improvement (i.e., CDI) for at least 12 weeks was in patients receiving ocrelizumab compared to patients receiving interferon beta-1a in STUDY II.
**FIG. 16C** shows the change in Multiple Sclerosis Functional Composite score from baseline to Week 96 patients receiving ocrelizumab compared to patients receiving interferon beta-1a in STUDY I.
**FIG. 16D** shows the change in Multiple Sclerosis Functional Composite score from baseline to Week 96 patients receiving ocrelizumab compared to patients receiving interferon beta-1a in STUDY II.
**FIG. 17A** shows the total number of T1 gadolinium-enhancing lesions detected at Week 24, Week 48, and Week 96 in patients receiving ocrelizumab compared to patients receiving interferon beta-1a in STUDY I.
**FIG. 17B** shows the total number of T1 gadolinium-enhancing lesions detected at Week 24, Week 48, and Week 96 in patients receiving ocrelizumab compared to patients receiving interferon beta-1a in STUDY II.
**FIG. 18A** shows the mean total number of T1 gadolinium-enhancing lesions in patients receiving ocrelizumab at Week 24, at Week 48, and at week 96 compared to patients receiving IFN β-1a in STUDY I.
**FIG. 18B** shows the mean total number of T1 gadolinium-enhancing lesions in patients receiving ocrelizumab at Week 24, at Week 48, and at week 96 compared to patients receiving IFN β-1a in STUDY II.
**FIG. 19A** shows the total number of new and/or enlarging hyperintense T2 lesions detected at Week 24, Week 48, and Week 96 in patients receiving ocrelizumab compared to patients receiving interferon beta-1a in STUDY I and STUDY II.
**FIG. 19B** shows the total number of new and/or enlarging hyperintense T2 lesions detected at Week 24, Week 48, and Week 96 in patients receiving ocrelizumab compared to patients receiving interferon beta-1a in STUDY II.
**FIG. 19C** shows the mean number of new and/or enlarging hyperintense T2 lesions detected at Week 24, Week 48, and Week 96 in patients receiving ocrelizumab compared to patients receiving interferon beta-1a in STUDY I.
**FIG. 19D** shows the mean number of new and/or enlarging hyperintense T2 lesions detected at Week 24, Week 48, and Week 96 in patients receiving ocrelizumab compared to patients receiving interferon beta-1a in STUDY II.
**FIG. 20A** shows the rate of brain volume loss from Week 24 to Week 96 in patients receiving ocrelizumab compared to patients receiving interferon beta-1a in STUDY I.
**FIG. 20B** shows the rate of brain volume loss from Week 24 to Week 96 in patients receiving ocrelizumab compared to patients receiving interferon beta-1a in STUDY II.
**FIG. 21A** shows the rate of brain volume loss from baseline to Week 96 in patients receiving ocrelizumab compared to patients receiving interferon beta-1a in STUDY I.
**FIG. 21B** shows the rate of brain volume loss from baseline to Week 96 in patients receiving ocrelizumab compared to patients receiving interferon beta-1a in STUDY II.
**FIG. 22A** shows the total number of new T1 hypointense lesions per MRI scan at Week 24, Week 48, and Week 96 in patients receiving ocrelizumab compared to patients receiving interferon beta-1a in STUDY I.
**FIG. 22B** shows the total number of new T1 hypointense lesions per MRI scan at Week 24, Week 48, and Week 96 in patients receiving ocrelizumab compared to patients receiving interferon beta-1a in STUDY II.
**FIG. 23A** shows infusion related reactions for patients receiving IFN β-1a in STUDY I and STUDY II (pooled).
**FIG. 23B** shows infusion related reactions for patients receiving ocrelizumab in STUDY I and STUDY II (pooled).
**FIG. 24** shows the study design for a Phase III study of Ocrelizumab in patients with primary progressive multiple sclerosis (PPMS).
**FIG. 25** provides a statistical hierarchy of the Phase III study of Ocrelizumab in patients with PPMS.
**FIG. 26** shows patient disposition at the clinical cut-off date for the Phase III PPMS study.
**FIG. 27** shows time to onset of Confirmed Disability Progression for at least 12 weeks in patients treated with ocrelizumab compared to patients receiving placebo for the Phase III PPMS study.
**FIG. 28** shows time to onset of Confirmed Disability Progression for at least 24 weeks in patients treated with ocrelizumab compared to patients receiving placebo for the Phase III PPMS study.
**FIG. 29** shows the rate of decline in walking speed, as measured by the Timed 25-Foot Walk, in patients receiving 600mg ocrelizumab compared to patients receiving placebo from baseline to Week 120 for the Phase III PPMS study.
**FIG. 30** shows the rate of decline in walking speed at Week 120 relative to baseline in patients receiving 600mg ocrelizumab compared to patients receiving placebo for the Phase III PPMS study.
**FIG. 31** shows the percent change of whole brain volume from Week 24 to Week 96 in patients receiving 600mg ocrelizumab compared to patients receiving placebo for the Phase III PPMS study.
**FIG. 32** shows the change in T2 lesion volume in in patients receiving 600mg ocrelizumab compared to patients receiving placebo for the Phase III PPMS study.
**FIG. 33** shows infusion related reactions (IRR) in patients receiving 600mg ocrelizumab compared to patients receiving placebo by exposure and severity until the clinical cut-off date for the Phase III PPMS study.
**FIG. 34A** shows the proportion of patients with no evidence of disease activity (NEDA) taking IFN β-1a compared to the proportion of patients with NEDA taking ocrelizumab in STUDY I.
**FIG. 34B** shows the proportion of patients with no evidence of disease activity (NEDA) taking IFN β-1a compared to the proportion of patients with NEDA taking ocrelizumab in STUDY II.
**FIG. 35A** shows the reduction in the percent change from baseline walking time in the timed 25-Foot Walk test to Week 120 in patients with Gd⁺ lesions at baseline receiving ocrelizumab vs. patients with Gd⁺ lesions at baseline receiving placebo.
**FIG. 35B** shows the reduction in the percent change from baseline walking time in the timed 25-Foot Walk test to Week 120 in patients without Gd⁺ lesions at baseline receiving ocrelizumab vs. patients without Gd⁺ lesions at baseline receiving placebo.
**FIG. 35C** shows the reduction in the percent change from baseline walking time in the timed 25-Foot Walk test to Week 120 in patients in the overall study population receiving ocrelizumab vs. patients in the overall study population receiving placebo.
**FIG. 36A** shows the difference of whole brain volume loss from Week 24 to Week 120 in ocrelizumab-treated patients with T1 Gd⁺ lesions at baseline as compared to patients with T1 Gd⁺ lesions at baseline receiving placebo.
**FIG. 36B** shows the difference of whole brain volume loss from Week 24 to Week 120 in ocrelizumab-treated patients without T1 Gd⁺ lesions at baseline as compared to patients without T1 Gd⁺ lesions at baseline receiving placebo.
**FIG. 36C** shows the rate of whole brain volume loss from Week 24 to Week 120 in ocrelizumab-treated patients in the overall study population as compared to patients in the overall study population receiving placebo.
**FIG. 37A** shows the difference in T2 lesion volume from baseline to week 120 in patients receiving ocrelizumab vs. patients receiving placebo.
**FIG. 37B** shows the difference in T2 lesion volume from baseline to week 120 in patients with T1 Gd⁺ lesions at baseline receiving ocrelizumab vs. patients with T1 Gd⁺ lesions at baseline receiving placebo.
**FIG. 37C** shows the difference in T2 lesion volume from baseline to week 120 in patients without T1 Gd⁺ lesions at baseline receiving ocrelizumab vs. patients without T1 Gd⁺ lesions at baseline receiving placebo.
**FIG. 38** shows the difference in reported improvement in change in quality of life between patients receiving ocrelizumab and patients receiving IFN β-1a, as measured by Short Form-36 (SF-36) Physical Component Summary (PCS).
**FIG. 39A** shows the difference between changes in EDSS score from baseline and Week 96 in patients receiving ocrelizumab vs. patients receiving IFN β-1a in STUDY I.
**FIG. 39B** shows the difference between changes in EDSS score from baseline and Week 96 in patients receiving ocrelizumab vs. patients receiving IFN β-1a in STUDY II.
**FIG. 40A** shows the change in SF-36 Physical Component Summary score from base line to Week 120 in patients in the overall study population receiving 600mg ocrelizumab compared to patients in the overall study population receiving placebo.
**FIG. 40B** shows the change in SF-36 Physical Component Summary score from base line to Week 120 in patients with T1 gadolinium-enhancing lesions at baseline receiving 600mg ocrelizumab compared to patients with T1 gadolinium-enhancing lesions at baseline receiving placebo.
**FIG. 40C** shows the change in SF-36 Physical Component Summary score from base line to Week 120 in patients without T1 gadolinium-enhancing lesions at baseline receiving 600mg ocrelizumab compared to patients without T1 gadolinium-enhancing lesions at baseline receiving placebo.
**FIG. 41** shows time to onset of Confirmed Composite Disability Progression for at least 12 weeks in patients treated with ocrelizumab compared to patients receiving placebo for the Phase III PPMS study.
**FIG. 42** shows time to onset of Confirmed Composite Disability Progression for at least 24 weeks in patients treated with ocrelizumab compared to patients receiving placebo for the Phase III PPMS study.
**FIG. 43** shows the annualized protocol-defined relapse rate by Week 96 in the following subgroups: active inadequate responders, active treatment-naive patients, highly active inadequate responders, and highly active treatment naive patients.
**FIG. 44** shows the time to onset of CDP for at least 12 weeks in the following subgroups: active inadequate responders, active treatment-naive patients, highly active inadequate responders, and highly active treatment naive patients.
**FIG. 45** shows the time to onset of CDP for at least 24 weeks in the following subgroups: active inadequate responders, active treatment-naive patients, highly active inadequate responders, and highly active treatment naive patients.
**FIG. 46** shows the proportion of patients in the following subgroups who have CDI for at least 12 weeks: active inadequate responders, active treatment-naive patients, highly active inadequate responders, and highly active treatment naive patients.
**FIG. 47** shows the total number of T1 gadolinium-enhancing lesions as detected by brain MRI in the following subgroups: active inadequate responders, active treatment-naive patients, highly active inadequate responders, and highly active treatment naive patients.

### Detailed Description of the Invention

### I. Definitions

A "B-cell" is a lymphocyte that matures within the bone marrow, and includes a naive B cell, memory B cell, or effector B cell (plasma cells). The B-cell herein may be a normal or non-malignant B cell.

A "B-cell surface marker" or "B-cell surface antigen" herein is an antigen expressed on the surface of a B cell that can be targeted with an antibody that binds thereto. Exemplary B-cell surface markers include the CD10, CD19, CD20, CD21, CD22, CD23, CD24, CD37, CD40, CD53, CD72, CD73, CD74, CDw75, CDw76, CD77, CDw78, CD79a, CD79b, CD80, CD81, CD82, CD83, CDw84, CD85 and CD86 leukocyte surface markers (for descriptions, *see* The Leukocyte Antigen Facts Book, 2nd Edition. 1997, ed. Barclay et al. Academic Press, Harcourt Brace & Co., New York). Other B-cell surface markers include RP105, FcRH2, B-cell CR2, CCR6, P2X5, HLA-DOB, CXCR5, FCER2, BR3, Btig, NAG14, SLGC16270, FcRH1, IRTA2, ATWD578, FcRH3, IRTA1, FcRH6, BCMA, and 239287. The B-cell surface marker of particular interest herein is preferentially expressed on B cells compared to other non-B-cell tissues of a mammal and may be expressed on both precursor B cells and mature B cells. The preferred B-cell surface marker herein is CD20.

The "CD20" antigen, or "CD20," is an about 35-kDa, non-glycosylated phosphoprotein found on the surface of greater than 90% of B cells from peripheral blood or lymphoid organs. CD20 is present on both normal B cells as well as malignant B cells, but is not expressed on stem cells. Other names for CD20 in the literature include "B-lymphocyte-restricted antigen" and "Bp35". The CD20 antigen is described in Clark et al. Proc. Natl. Acad. Sci. (USA) 82:1766 (1985), for example.

An "antibody antagonist" herein is an antibody that, upon binding to a B cell surface marker on B cells, destroys or depletes B cells in a mammal and/or interferes with one or more B-cell functions, *e*.*g*. by reducing or preventing a humoral response elicited by the B cell. The antibody antagonist preferably is able to deplete B cells (*i.e.* reduce circulating B-cell levels) in a mammal treated therewith. Such depletion may be achieved via various mechanisms such antibody-dependent cell-mediated cytotoxicity (ADCC) and/or complement dependent cytotoxicity (CDC), inhibition of B-cell proliferation and/or induction of B-cell death *(e.g.* via apoptosis).

"Antibody-dependent cell-mediated cytotoxicity" and "ADCC" refer to a cell-mediated reaction in which nonspecific cytotoxic cells that express Fc receptors (FcRs) *(e.g.* Natural Killer (NK) cells, neutrophils, and macrophages) recognize bound antibody on a target cell and subsequently cause lysis of the target cell. The primary cells for mediating ADCC, NK cells, express FcyRIII only, whereas monocytes express FcyRI, FcyRII and FcyRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. PNAS (USA) 95:652-656 (1998).

"Human effector cells" are leukocytes that express one or more FcRs and perform effector functions. In some embodiments, the cells express at least FcyRIII and carry out ADCC effector function. Examples of human leukocytes that mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. In some embodiments, the FcR is a native sequence human FcR. Moreover, a preferred FcR is one that binds an IgG antibody (a gamma receptor) and includes receptors of the FcyRI, FcyRII, and Fcy RIII subclasses, including allelic variants and alternatively spliced forms of these receptors. FcyRII receptors include FcyRIIA (an "activating receptor") and FcyRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain. *(see* Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

"Complement dependent cytotoxicity" or "CDC" refers to the ability of a molecule to lyse a target in the presence of complement. The complement activation pathway is initiated by the binding of the first component of the complement system (C1q) to a molecule *(e.g.* an antibody) complexed with a cognate antigen. To assess complement activation, a CDC assay, *e.g.* as described in Gazzano-Santoro et al., J. Immunol. Methods 202:163 (1996) may be performed.

"Growth inhibitory" antibodies are those that prevent or reduce proliferation of a cell expressing an antigen to which the antibody binds. For example, the antibody may prevent or reduce proliferation of B cells *in vitro* and/or *in vivo.*

Antibodies that "induce apoptosis" are those that induce programmed cell death, *e.g.* of a B cell, as determined by standard apoptosis assays, such as binding of annexin V, fragmentation of DNA, cell shrinkage, dilation of endoplasmic reticulum, cell fragmentation, and/or formation of membrane vesicles (called apoptotic bodies).

The term "antibody" herein is used in the broadest sense and specifically covers monoclonal antibodies, polyclonal antibodies, multispecific antibodies (*e.g.* bispecific antibodies) formed from at least two intact antibodies, and antibody fragments so long as they exhibit the desired biological activity.

"Antibody fragments" comprise a portion of an intact antibody, preferably comprising the antigen binding region thereof. Examples of antibody fragments include Fab, Fab', F(ab')₂, and Fv fragments; diabodies; linear antibodies; single-chain antibody molecules; and multispecific antibodies formed from antibody fragments.

For the purposes herein, an "intact antibody" is one comprising heavy and light variable domains as well as an Fc region.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), *e.g.,* IgG1, IgG2, IgG3, IgG4, IgA, and IgA2. The heavy chain constant domains that correspond to the different classes of antibodies are called α, δ, ε, γ, and µ, respectively. The subunit structures and three-dimensional configurations of different classes of immunoglobulins are well known.

"Single-chain Fv" or "scFv" antibody fragments comprise the V_{H} and V_{L} domains of antibody, wherein these domains are present in a single polypeptide chain. In some embodiments, the Fv polypeptide further comprises a polypeptide linker between the V_{H} and V_{L} domains that enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "monoclonal antibody" as used herein refers to an antibody obtained from a population of substantially homogeneous antibodies, *i.e.,* the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies may be made by the hybridoma method first described by Kohler et al., Nature, 256:495 (1975), or may be made by recombinant DNA methods (*see, e.g.,* U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (*e.g.* Old World Monkey, such as baboon, rhesus or cynomolgus monkey) and human constant region sequences (US Pat No. 5,693,780).

"Humanized" forms of non-human (*e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence, except for FR substitution(s) as noted above. The humanized antibody optionally also will comprise at least a portion of an immunoglobulin constant region, typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

The term "hypervariable region" when used herein refers to the amino acid residues of an antibody that are responsible for antigen binding. The hypervariable region comprises amino acid residues from a "complementarity determining region" or "CDR" *(e.g.* residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" *(e.g.* residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). "Framework" or "FR" residues are those variable domain residues other than the hypervariable region residues as herein defined.

A "naked antibody" is an antibody (as herein defined) that is not conjugated to a heterologous molecule, such as a cytotoxic moiety or radiolabel.

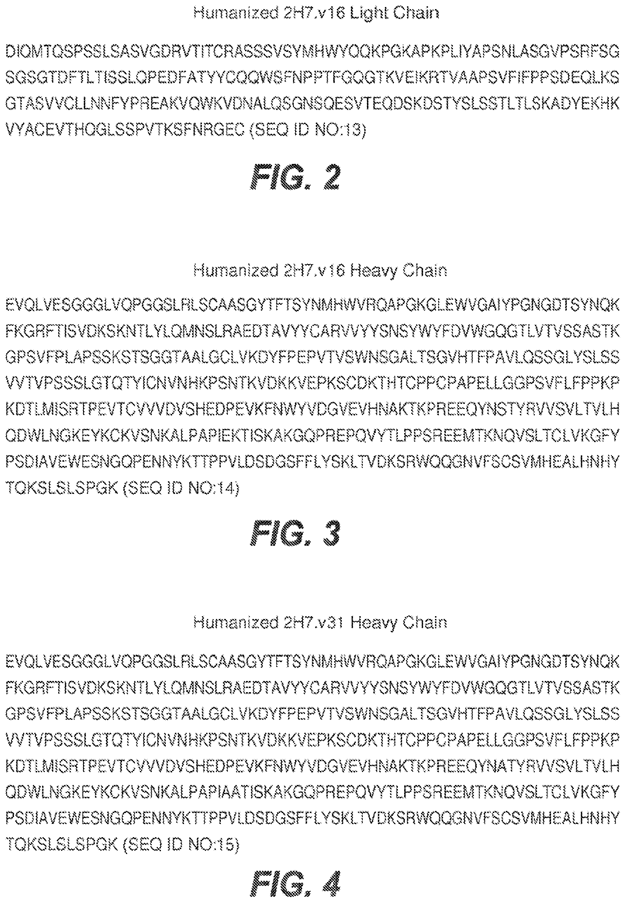

Purely for the purposes herein and unless indicated otherwise, "humanized 2H7" refers to a humanized antibody that binds human CD20, or an antigen-binding fragment thereof, wherein the antibody is effective to deplete primate B cells *in vivo,* the antibody comprising in the H chain variable region (V_{H}) thereof at least a CDR H3 sequence of SEQ ID NO:12 (Fig. 1B) from an anti-human CD20 antibody and substantially the human consensus framework (FR) residues of the human heavy- chain subgroup III (V_{H}III). In some embodiments, this antibody further comprises the H chain CDR H1 sequence of SEQ ID NO:10 and CDR H2 sequence of SEQ ID NO:11, and, in some embodiments, further comprises the L chain CDR L1 sequence of SEQ ID NO:4, CDR L2 sequence of SEQ ID NO:5, CDR L3 sequence of SEQ ID NO:6 and substantially the human consensus framework (FR) residues of the human light chain kappa subgroup I (V_{κ}I), wherein the V_{H} region may be joined to a human IgG chain constant region, wherein the region may be, for example, IgG1 or IgG3. In some embodiments, such antibody comprises the V_{H} sequence of SEQ ID NO:8 (v16, as shown in Fig. 1B), optionally also comprising the V_{L} sequence of SEQ ID NO:2 (v16, as shown in Fig. 1A), which may have the amino acid substitutions of D56A and N100A in the H chain and S92A in the L chain (v96). In some embodiments, the antibody is an intact antibody comprising the light and heavy chain amino acid sequences of SEQ ID NOS: 13 and 14, respectively, as shown in Figs. 2 and 3. In some embodiments, the antibody is 2H7.v31 comprising the light and heavy chain amino acid sequences of SEQ ID NOS: 13 and 15, respectively, as shown in Figs. 2 and 4. The antibody herein may further comprise at least one amino acid substitution in the Fc region that improves ADCC and/or CDC activity, such as one wherein the amino acid substitutions are S298A/E333A/K334A, and in some embodiments, the 2H7.v31 having the heavy chain amino acid sequence of SEQ ID NO: 15 (as shown in Fig. 4). Any of these antibodies may further comprise at least one amino acid substitution in the Fc region that decreases CDC activity, for example, comprising at least the substitution K322A. *See* U.S. Patent No. 6,528,624B1 (Idusogie et al*.).*

The term "ocrelizumab" (CAS Registration No. 637334-45-3) herein refers to the genetically engineered humanized monoclonal antibody directed against the CD20 antigen and comprising (a) a light chain comprising the amino acid sequence of SEQ ID NO: 13 and (b) a heavy chain comprising the amino acid sequence of SEQ ID NO: 14. Ocrelizumab is available from Genentech.

An "isolated" antibody is one that has been identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. In some embodiments, the antibody will be purified (1) to greater than 95% by weight of antibody as determined by the Lowry method, and in some embodiments, more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, in some embodiments, silver stain. Isolated antibody includes the antibody *in situ* within recombinant cells since at least one component of the antibody's natural environment will not be present. Ordinarily, however, isolated antibody will be prepared by at least one purification step.

A "subject" or "patient" herein is a human subject or patient. Generally, the subject or patient is eligible for treatment for multiple sclerosis. For the purposes herein, such eligible subject or patient is one who is experiencing, has experienced, or is likely to experience, one or more signs, symptoms or other indicators of multiple sclerosis; has been diagnosed with multiple sclerosis, whether, for example, newly diagnosed (with "new onset" MS), previously diagnosed with a new relapse or exacerbation, previously diagnosed and in remission, etc; and/or is at risk for developing multiple sclerosis. One suffering from or at risk for suffering from multiple sclerosis may optionally be identified as one who has been screened for elevated levels of CD20-positive B cells in serum, cerebrospinal fluid (CSF) and/or MS lesion(s) and/or is screened for using an assay to detect autoantibodies, assessed qualitatively, and preferably quantitatively. Exemplary such autoantibodies associated with multiple sclerosis include anti-myelin basic protein (MBP), anti-myelin oligodendrocytic glycoprotein (MOG), anti-ganglioside and/or anti-neurofilament antibodies. Such autoantibodies may be detected in the subject's serum, cerebrospinal fluid (CSF) and/or MS lesion. By "elevated" autoantibody or B cell level(s) herein is meant level(s) of such autoantibodies or B cells which significantly exceed the level(s) in an individual without MS.

As used herein, "treatment" or "treating" is an approach for obtaining beneficial or desired results including clinical results. For purposes of this invention, beneficial or desired clinical results include, but are not limited to, one or more of the following: decreasing one or more symptoms resulting from the disease, diminishing the extent of the disease, stabilizing the disease (*e.g.,* preventing or delaying the worsening of the disease), delay or slowing the progression of the disease, ameliorating the disease state, decreasing the dose of one or more other medications required to treat the disease, and/or increasing the quality of life.

As used herein, "delaying" or "slowing" the progression of multiple sclerosis means to prevent, defer, hinder, slow, retard, stabilize, and/or postpone development of the disease. This delay can be of varying lengths of time, depending on the history of the disease and/or individual being treated.

As used herein, "at the time of starting treatment" refers to the time period at or prior to the first exposure to a multiple sclerosis drug, such as an anti-CD20 antibody. In some embodiments, "at the time of starting treatment" is about any of one year, nine months, six months, three months, second months, or one month prior to a multiple sclerosis drug, such as an anti-CD20 antibody. In some embodiments, "at the time of starting treatment" is immediately prior to coincidental with the first exposure to a multiple sclerosis drug, such as an anti-CD20 antibody.

As used herein, "based upon" includes (1) assessing, determining, or measuring the patient characteristics as described herein (and preferably selecting a patient suitable for receiving treatment; and (2) administering the treatment(s) as described herein.

A "symptom" of MS is any morbid phenomenon or departure from the normal in structure, function, or sensation, experienced by the subject and indicative of MS.

"Multiple sclerosis" refers to the chronic and often disabling disease of the central nervous system characterized by the progressive destruction of the myelin. There are four internationally recognized forms of MS, namely, primary progressive multiple sclerosis (PPMS), relapsing-remitting multiple sclerosis (RRMS), secondary progressive multiple sclerosis (SPMS), and progressive relapsing multiple sclerosis (PRMS).

"Progressive multiple sclerosis" as used herein refers to primary progressive multiple sclerosis (PPMS), secondary progressive multiple sclerosis (SPMS), and progressive relapsing multiple sclerosis (PRMS). In some embodiments, progressive multiple sclerosis is characterized by documented, irreversible loss of neurological function persisting for ≥ 6 months that cannot be attributed to clinical relapse.

"Primary progressive multiple sclerosis" or "PPMS" is characterized by a gradual progression of the disease from its onset with rare superimposed relapses and remissions. There may be periods of a leveling off of disease activity and there may be good and bad days or weeks. PPMS differs from RRMS and SPMS in that onset is typically in the late thirties or early forties, men are as likely as women to develop it, and initial disease activity is often in the spinal cord and not in the brain. PPMS disease activity can also be observed (or found) in the brain. PPMS is the sub-type of MS that is least likely to show inflammatory (gadolinium enhancing) lesions on MRI scans. The Primary Progressive form of the disease affects between 10 and 15% of all people with multiple sclerosis. PPMS may be defined according to the criteria in Polman et al. Ann Neurol 69:292-392 (2010). The subject with PPMS treated herein is usually one with probable or definitive diagnosis of PPMS.

"Relapsing-remitting multiple sclerosis" or "RRMS" is characterized by relapses (also known as exacerbations) during which time new symptoms can appear and old ones resurface or worsen. The relapses are followed by periods of remission, during which time the person fully or partially recovers from the deficits acquired during the relapse. Relapses can last for days, weeks or months and recovery can be slow and gradual or almost instantaneous. The vast majority of people presenting with MS are first diagnosed with RRMS. This is typically when they are in their twenties or thirties, though diagnoses much earlier or later are known. Twice as many women as men present with this sub-type of MS. During relapses, myelin, a protective insulating sheath around the nerve fibers (neurons) in the white matter regions of the central nervous system (CNS), may be damaged in an inflammatory response by the body's own immune system. This causes a wide variety of neurological symptoms that vary considerably depending on which areas of the CNS are damaged. Immediately after a relapse, the inflammatory response dies down and a special type of glial cell in the CNS (called an oligodendrocyte) sponsors remyelination - a process whereby the myelin sheath around the axon may be repaired. It is this remyelination that may be responsible for the remission. Approximately 50% of patients with RRMS convert to SPMS within 10 years of disease onset. After 30 years, this figure rises to 90%. At any one time, the relapsing-remitting form of the disease accounts around 55% of all people with MS.

"Secondary progressive multiple sclerosis" or "SPMS" is characterized by a steady progression of clinical neurological damage with or without superimposed relapses and minor remissions and plateaus. People who develop SPMS will have previously experienced a period of RRMS which may have lasted anything from two to forty years or more. Any superimposed relapses and remissions there are, tend to tail off over time. From the onset of the secondary progressive phase of the disease, disability starts advancing much quicker than it did during RRMS though the progress can still be quite slow in some individuals. After 10 years, 50% of people with RRMS will have developed SPMS. By 25 to 30 years, that figure will have risen to 90%. SPMS tends to be associated with lower levels of inflammatory lesion formation than in RRMS but the total burden of disease continues to progress. At any one time, SPMS accounts around 30% of all people with multiple sclerosis.

"Progressive relapsing multiple sclerosis" refers to "PRMS" is characterized by a steady progression of clinical neurological damage with superimposed relapses and remissions. There is significant recovery immediately following a relapse but between relapses there is a gradual worsening of symptoms. PRMS affects around 5% of all people with multiple sclerosis. Some neurologists believe PRMS is a variant of PPMS. The expression "effective amount" refers to an amount of the antibody (or other drug) that is effective for ameliorating or treating the multiple sclerosis. Such an effective amount will generally result in an improvement in the signs, symptoms or other indicators of MS, such as reducing relapse rate, preventing disability, reducing number and/or volume of brain MRI lesions, improving timed 25-foot walk, slow or delay the progression of the disease such as extending the time to disease progression (*e.g.* using Expanded Disability Status Scale, EDSS), etc.

"Antibody exposure" refers to contact with or exposure to the antibody herein in one or more doses administered over a period of time of about 1-20 days. The doses may be given at one time or at fixed or irregular time intervals over this period of exposure. Initial and later (*e.g.* second or third) antibody exposures are separated in time from each other as described in detail herein.

As used herein, an "interval" between antibody exposures refers to time period between an earlier antibody exposure and a later antibody exposure. An antibody exposure of the present disclosure may include one or two doses. In cases where the antibody exposures contain one dose, an interval between two antibody exposures refers to the amount of time elapsed between the dose of one antibody exposure (*e.g.,* Day 1) and the dose of the next antibody exposure. If one antibody exposure includes two doses and the next antibody exposure includes one dose, an interval between the two antibody exposures refers to the amount of time elapsed between the first of the two doses of the first antibody exposure (*e.g.,* Day 1) and the dose of the next antibody exposure. In cases where each of the two antibody exposures contain two doses, an interval between to the antibody exposures refers to the amount of time elapsed between the first of the two doses of the first antibody exposure (*e.g.,* Day 1) and the first dose of the two doses of the second antibody exposure. For example, if the antibody for use in a method of the present disclosure includes a first antibody exposure with two doses and a second antibody exposure with two doses, and the second antibody exposure is not provided until about 24 weeks or 6 months after the first antibody exposure, then the interval between the first dose of the first antibody exposure and the first dose of the second antibody exposure is about 24 weeks or 6 months. In certain embodiments, the second antibody exposure is not provided until about 20-24 weeks or about 5-6 months after the first antibody exposure. In some embodiments, the interval between the first dose of the first antibody exposure and the first dose of the second antibody exposure is about 20-24 weeks or about 5-6 months. In some embodiments, "about 20-24 weeks" refers to a time point between 20 weeks and 24 weeks. In some embodiments, "about 20-24 weeks" refers to a variation of a week or 7 days before or after the 24^{th} week. In some embodiments, "about 5-6 months" refers to a time point between 5 and 6 months.

The term "immunosuppressive agent" as used herein for adjunct therapy refers to substances that act to suppress or mask the immune system of the mammal being treated herein. This would include substances that suppress cytokine production, down-regulate or suppress self-antigen expression, or mask the MHC antigens. Examples of such agents include 2-amino-6-aryl-5-substituted pyrimidines (*see* U.S. Pat. No. 4,665,077); nonsteroidal anti-inflammatory drugs (NSAIDs); ganciclovir, tacrolimus, glucocorticoids such as cortisol or aldosterone, anti-inflammatory agents such as a cyclooxygenase inhibitor, a 5-lipoxygenase inhibitor, or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Pat. No. 4,120,649); anti-idiotypic antibodies for MHC antigens and MHC fragments; cyclosporin A; steroids such as corticosteroids or glucocorticosteroids or glucocorticoid analogs, *e*.*g*., prednisone, methylprednisolone, and dexamethasone; dihydrofolate reductase inhibitors such as methotrexate (oral or subcutaneous); hydroxychloroquine; sulfasalazine; leflunomide; cytokine or cytokine receptor antagonists including anti-interferon-alpha, -beta, or -gamma antibodies, anti-tumor necrosis factor-alpha antibodies (infliximab or adalimumab), anti-TNF-alpha immunoadhesin (etanercept), anti-tumor necrosis factor-beta antibodies, anti-interleukin-2 antibodies and anti-IL-2 receptor antibodies; anti-LFA-1 antibodies, including anti-CD11a and anti-CD18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published 7/26/90); streptokinase; TGF-beta; streptodornase; RNA or DNA from the host; FK506; RS-61443; deoxyspergualin; rapamycin; T-cell receptor (Cohen et al., U.S. Pat. No. 5,114,721); T-cell receptor fragments (Offner et al., Science, 251: 430-432 (1991); WO 90/11294;Janeway, Nature, 341: 482 (1989); and WO 91/01133); and T cell receptor antibodies (EP 340,109) such as T10B9.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, or fragments thereof.

A "chemotherapeutic agent" is a chemical compound useful in the treatment of cancer. Examples of chemotherapeutic agents include alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e. g.,* calicheamicin, especially calicheamicin gammalI and calicheamicin omegaI1 (*see, e.g.,* Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, *e*.*g*., TAXOL^{®} paclitaxel (Bristol- Myers Squibb Oncology, Princeton, N.J.), ABRAXANE^{™} Cremophor-free, albumin-engineered nanoparticle formulation of paclitaxel (American Pharmaceutical Partners, Schaumberg, Illinois), and TAXOTERE^{®} doxetaxel (Rhône- Poulenc Rorer, Antony, France); chloranbucil; GEMZAR^{®} gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeloda; ibandronate; CPT-11; topoisomerase inhibitor RFS 2000; difluoromethylomithine (DMFO); retinoids such as retinoic acid; capecitabine; and pharmaceutically acceptable salts, acids or derivatives of any of the above.

Also included in this definition are anti-hormonal agents that act to regulate or inhibit hormone action on tumors such as anti-estrogens and selective estrogen receptor modulators (SERMs), including, for example, tamoxifen (including NOLVADEX^{®} tamoxifen), raloxifene, droloxifene, 4-hydroxytamoxifen, trioxifene, keoxifene, LY117018, onapristone, and FARESTON toremifene; aromatase inhibitors that inhibit the enzyme aromatase, which regulates estrogen production in the adrenal glands, such as, for example, 4(5)-imidazoles, aminoglutethimide, MEGASE^{®} megestrol acetate, AROMASIN^{®} exemestane, formestanie, fadrozole, RIVISOR^{®} vorozole, FEMARA^{®} letrozole, and ARIMIDEX^{®} anastrozole; and antiandrogens such as flutamide, nilutamide, bicalutamide, leuprolide, and goserelin; as well as troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Ralf and H-Ras; vaccines such as gene therapy vaccines, for example, ALLOVECTIN^{®} vaccine, LEUVECTIN^{®} vaccine, and VAXID^{®} vaccine; PROLEUKIN^{®} rIL-2; LURTOTECAN^{®} topoisomerase 1 inhibitor; ABARELIX^{®} rmRH; and pharmaceutically acceptable salts, acids or derivatives of any of the above.

The term "cytokine" is a generic term for proteins released by one cell population that act on another cell as intercellular mediators. Examples of such cytokines are lymphokines, monokines; interleukins (ILs) such as IL-1, IL-1α, IL-2, IL-3, IL-4, IL-5, IL-6, IL-7, IL-8, IL-9, IL-11, IL-12, IL-15; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines, including synthetically produced small-molecule entities and pharmaceutically acceptable derivatives and salts thereof.

The term "hormone" refers to polypeptide hormones, which are generally secreted by glandular organs with ducts. Included among the hormones are, for example, growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); prolactin, placental lactogen, mouse gonadotropin-associated peptide, inhibin; activin; mullerian-inhibiting substance; and thrombopoietin. As used herein, the term hormone includes proteins from natural sources or from recombinant cell culture and biologically active equivalents of the native sequence hormone, including synthetically produced small-molecule entities and pharmaceutically acceptable derivatives and salts thereof.

The term "growth factor" refers to proteins that promote growth, and include, for example, hepatic growth factor; fibroblast growth factor; vascular endothelial growth factor; nerve growth factors such as NGF-β; platelet-derived growth factor; transforming growth factors (TGFs) such as TGF-α and TGF-β; insulin-like growth factor-I and -II; erythropoietin (EPO); osteoinductive factors; interferons such as interferon-α, -β, and -y; and colony stimulating factors (CSFs) such as macrophage-CSF (M-CSF); granulocyte-macrophage-CSF (GM-CSF); and granulocyte-CSF (G-CSF). As used herein, the term growth factor includes proteins from natural sources or from recombinant cell culture and biologically active equivalents of the native sequence growth factor, including synthetically produced small-molecule entities and pharmaceutically acceptable derivatives and salts thereof.

The term "integrin" refers to a receptor protein that allows cells both to bind to and to respond to the extracellular matrix and is involved in a variety of cellular functions such as wound healing, cell differentiation, homing of tumor cells and apoptosis. They are part of a large family of cell adhesion receptors that are involved in cell-extracellular matrix and cell-cell interactions. Functional integrins consist of two transmembrane glycoprotein subunits, called alpha and beta that are non-covalently bound. The alpha subunits all share some homology to each other, as do the beta subunits. The receptors always contain one alpha chain and one beta chain. Examples include Alpha6beta1, Alpha3beta1, Alpha7beta1, LFA-1, alpha 4 integrin etc. As used herein, the term integrin includes proteins from natural sources or from recombinant cell culture and biologically active equivalents of the native sequence integrin, including synthetically produced small-molecule entities and pharmaceutically acceptable derivatives and salts thereof.

Examples of "integrin antagonists or antibodies" herein include an LFA-1 antibody; an alpha 4 integrin antibody such as natalizumab (TYSABRI^{®}) available from Biogen Idec/Elan Pharmaceuticals, Inc.; diazacyclic phenylalanine derivatives (WO 2003/89410); phenylalanine derivatives (WO 2003/70709, WO 2002/28830, WO 2002/16329 and WO 2003/53926); phenylpropionic acid derivatives (WO 2003/10135); enamine derivatives (WO 2001/79173); propanoic acid derivatives (WO 2000/37444); alkanoic acid derivatives (WO 2000/32575); substituted phenyl derivatives (US Pat. Nos. 6,677,339 and 6,348,463); aromatic amine derivatives (US Pat. No. 6,369,229); and ADAM disintegrin domain polypeptide (US2002/0042368), antibodies to alphavbeta3 integrin (EP 633945); aza-bridged bicyclic amino acid derivatives (WO 2002/02556) etc.

For the purposes herein, "tumor necrosis factor alpha (TNF-alpha)" refers to a human TNF-alpha molecule comprising the amino acid sequence as described in Pennica et al., Nature, 312:721 (1984) or Aggarwal et al., JBC, 260:2345 (1985).

A "TNF-alpha inhibitor" herein is an agent that inhibits, to some extent, a biological function of TNF-alpha, generally through binding to TNF-alpha and neutralizing its activity. Examples of TNF inhibitors specifically contemplated herein are Etanercept (ENBREL^{®}), Infliximab (REMICADE^{®}) and Adalimumab (HUMIRA^{™}).

Examples of "disease-modifying anti-rheumatic drugs" or "DMARDs" include hydroxychloroquine, sulfasalazine, methotrexate, leflunomide, etanercept, infliximab (plus oral and subcutaneous methrotrexate), azathioprine, D-penicillamine, Gold (oral), Gold (intramuscular), minocycline, cyclosporine, Staphylococcal protein A immunoadsorption, including salts and derivatives thereof, etc.

Examples of "nonsteroidal anti-inflammatory drugs" or "NSAIDs" are acetylsalicylic acid, ibuprofen, naproxen, indomethacin, sulindac, tolmetin, including salts and derivatives thereof, etc.

"Corticosteroid" refers to any one of several synthetic or naturally occurring substances with the general chemical structure of steroids that mimic or augment the effects of the naturally occurring corticosteroids. Examples of synthetic corticosteroids include prednisone, prednisolone (including methylprednisolone), dexamethasone, glucocorticoid and betamethasone.

A "package insert" is used to refer to instructions customarily included in commercial packages of therapeutic products, that contain information about the indications, usage, dosage, administration, contraindications, other therapeutic products to be combined with the packaged product, and/or warnings concerning the use of such therapeutic products, etc.

A "label" is used herein to refer to information customarily included with commercial packages of pharmaceutical formulations including containers such as vials and package inserts, as well as other types of packaging.

Reference to "about" a value or parameter herein includes (and describes) variations that are directed to that value or parameter per se. For example, description referring to "about X" includes description of "X."

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

It is to be understood that one, some, or all of the properties of the various embodiments described herein may be combined to form other embodiments of the present invention. These and other aspects of the invention will become apparent to one of skill in the art.

### II. Treatment

As explained above, the present invention provides an anti-CD20 antibody for use in a method of improving functional ability in a human patient having multiple sclerosis comprising administering to the patient an effective amount of the anti-CD20 antibody, wherein the patient has improvement in functional ability after treatment; wherein the anti-CD20 antibody is ocrelizumab; wherein the multiple sclerosis is a primary progressive multiple sclerosis (PPMS) with T1 gadolinium staining lesions; and wherein the improvement in functional ability is measured by the Timed 25-Foot Walk (T-25FW) test or EDSS score. In some embodiments, the method further comprises a step of measuring the patient's functional ability by measuring EDSS score and/or the Timed 25-Foot Walk (T25-FW) after 1, 2, 3, 4, or more than 4 exposures of anti-CD20 antibody.

In certain embodiments, the patient has at least about a 12-week confirmed disability improvement after treatment. In certain embodiments, the patient has at least about a 24-week confirmed disability improvement after treatment. In certain embodiments, the confirmed disability improvement is determined by Expanded Disability Status Scale (EDSS) score. In certain embodiments, the patient's EDSS score decreases by at least about 0.1, about 0.2, about 0.3, about 0.4, about 0.5, about 0.6, about 0.7, about 0.8, about 0.9, about 1.0 or more than about 1.0 points (such as about 1.1, about 1.2, about 1.3, about 1.4, or about 1.5 points).

In certain embodiments, the improvement in functional ability is sustained for at least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about12 weeks, at least about 13 weeks, at least about 14 weeks, at least about 15 weeks, at least about 16 weeks, at least about 17 weeks, at least about 18 weeks, at least about 19 weeks, at least about 20 weeks, at least about 21 weeks, at least about 22 weeks, at least about 23 weeks, including any range in between these values. In certain embodiments, the improvement in functional ability is sustained for at least about 24 weeks, at least about 25 weeks, at least about 26 weeks, at least about 27 weeks, at least about 28 weeks, at least about 29 weeks, at least about 30 weeks, at least about 35 weeks, at least about 40 weeks, at least about 45 weeks, at least about 50 weeks, at least about 55 weeks, at least about 60 weeks, at least about 65 weeks, at least about 70 weeks, at least about 75weeks, or more than about 75 weeks, including any range in between these values.

In certain embodiments, the improvement in functional ability is measured by the Timed 25-Foot Walk (T25-FW) test. In certain embodiments, the time to walk 25 feet following the start of treatment is reduced by about 5 seconds, about 10 seconds, about 30 seconds, about 60 seconds, about 90 seconds, about 2 minutes, about 2.5 minutes, about 3 minutes, about 3.5 minutes, about 4 minutes, about 4.5 minutes, about 5 minutes, about 5.5 minutes, about 6 minutes, about 6.5 minutes, about 7 minutes, about 7.5 minutes, about 8 minutes, about 8.5 minutes, about 9 minutes about 9.5 minutes, or about 10 minutes relative to the time to walk 25 feet immediately prior to starting treatment.

In certain embodiments, improvement in functional activity is demonstrated by no evidence of disease activity (NEDA). In certain embodiments, NEDA is demonstrated by the absence of new or enlarging T2 lesions or T1 gadolinium-enhancing lesions on magnetic resonance imaging. In certain embodiments, NEDA is demonstrated by absence of relapse. In certain embodiments, NEDA is demonstrated by the absence of progression. In certain embodiments, NEDA is demonstrated by lack of worsening of EDSS. In certain embodiments, NEDA is defined as: no protocol-defined relapses, no CDP events, no new or enlarging T2 lesions, and no gadolinium-enhancing T1 lesions. In certain embodiments, NEDA is sustained for about least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about12 weeks, at least about 13 weeks, at least about 14 weeks, at least about 15 weeks, at least about 16 weeks, at least about 17 weeks, at least about 18 weeks, at least about 19 weeks, at least about 20 weeks, at least about 21 weeks, at least about 22 weeks, at least about 23 weeks, including any range in between these values. In certain embodiments, the improvement in functional ability is sustained for at least about 24 weeks, at least about 25 weeks, at least about 26 weeks, at least about 27 weeks, at least about 28 weeks, at least about 29 weeks, at least about 30 weeks, at least about 35 weeks, at least about 40 weeks, at least about 45 weeks, at least about 50 weeks, at least about 55 weeks, at least about 60 weeks, at least about 65 weeks, at least about 70 weeks, at least about 75 weeks, or more than about 75 weeks, including any range in between these values.

As explained before, the invention relates to an anti-CD20 antibody for use in a method of improving functional ability in a human patient having multiple sclerosis, as further defined herein above and in the claims, wherein the patient has T1 gadolinium staining lesions at baseline (i.e., before starting treatment).

In certain embodiments, the patient has not been previously treated with other therapy for multiple sclerosis (i.e., a "naive patient"). In certain embodiments, the naive patient experienced at least 2 relapses in 2 years prior to starting treatment. In certain embodiments, the naive patient experienced at least 1 relapse in the last year prior to starting treatment.

In certain embodiments, the patient is an inadequate responder to other therapy for multiple sclerosis. In certain embodiments, the patient who is an inadequate responder has been previously treated with interferon beta-1a or glatiramer acetate for at least 1 year. In certain embodiments, the patient who is an inadequate responder has experienced at least one relapse or experienced at least 1 baseline gadolinium-enhancing lesion while being treated with another therapy for multiple sclerosis.

As explained above, the patient has primary progressive multiple sclerosis. In certain embodiments, the patient is less than 18 years of age. In certain embodiments, the patient is between 18 and 55 years of age. In certain embodiments, the patient is over 55 years of age. In certain embodiments, the patient has a diagnosis of Primary Progressive Multiple Sclerosis in accordance with the 2005 revised McDonald criteria (Polman et al. (2011) "Diagnostic criteria for multiple sclerosis: 2005 revisions to the `McDonald criteria.'", Ann Neurol 58, 840-846). Additionally or alternatively, in certain embodiments, the patient has an Expanded Disability Status Scale (EDSS) score of 3 to 6.5 points. Additionally or alternatively, in certain embodiments, the patient has a score of at least 2.0 on the pyramidal functions component of the Functional Systems Scale (FSS). Additionally or alternatively, in certain embodiments, the patient has a documented history or presence at screening of elevated IgG index in a cerebrospinal fluid (CSF) specimen and/or one or more IgG oligoclonal bands detected by isoelectric focusing in a cerebrospinal fluid (CSF) specimen. Additionally or alternatively, in certain embodiments, the patient has no history of relapse-remitting multiple sclerosis (RRMS). Additionally or alternatively, in certain embodiments, the patient has no history of secondary progressive multiple sclerosis (SPMS). Additionally or alternatively, in certain embodiments, the patient has history of progressive relapsing multiple sclerosis (PRMS).

In certain embodiments, the patient has had previous treatment with B-cell targeted therapies (e.g. rituximab, ocrelizumab, atacicept, belimumab, or ofatumumab). In certain embodiments, the patient has not had previous treatment with B-cell targeted therapies (e.g. rituximab, ocrelizumab, atacicept, belimumab, or ofatumumab).

In some embodiments, "Confirmed Disability Progression" or "CDP" refers to an increase of at least 1.0 point from the baseline EDSS score in patients with a baseline score of 5.5 or less, or an increase of a 0.5 point in patients with a baseline score over 5.5, during the 96 weeks, wherein increases in the EDSS were confirmed at a regularly scheduled visit at least 12 weeks after the initial neurologic worsening.

In some embodiments, "Confirmed Disability Improvement" or "CDI" refers to a reduction in EDSS score of at least 1.0 compared to baseline in patients with a baseline EDSS score of 5.5 or less, or a reduction of a 0.5 point in patients with a baseline EDSS score above 5.5.

In some embodiments, brain atrophy refers to one or more of the following: axonal loss in the brain, tissue loss within gray matter lesions or white matter lesions, Wallerian degeneration in pathways related to the lesions, or lesion burden. In certain embodiments, brain atrophy refers to a decrease in whole brain volume. In certain embodiments, brain atrophy refers to a decrease in volume of one or more of the structures of the brain (including, but not limited to, the cerebrum, the cerebellum, the thalamus, frontotemporal neocortex, brainstem, hippocampus, parietal lobe, and/or the hypothalamus). In certain embodiments, brain atrophy refers to cortical thinning in precentral gyrus, superior frontal gyrus, thalamus and/or putamen. In certain embodiments, brain atrophy refers to a loss of at least about 0.4%, at least about 0.5%, at least about 0.6%, or at least 0.7% brain volume per year. Further details regarding brain atrophy are detailed in, *e.g.,* Riley et al. (2012) Expert Rev Neurother 12(3), 323-333.

In some embodiments, the patient or subject has highly active multiple sclerosis. In some embodiments, "highly active multiple sclerosis" in treatment naive patients refers to patients who have not been previously treated with other therapy for multiple sclerosis and who have experienced at least 2 relapses in the last year prior to randomization, and either (a) at least 1 baseline gadolinium lesion or (b) increase in T2 lesion count at baseline visit (changing categorically from 0-5 to 6-9 lesions or from 6-9 lesions to > 9 lesions) as compared to a prior MRI. In some embodiments, "highly active multiple sclerosis" in patients who have been previously treated with other therapy for multiple sclerosis and who have had at least one relapse in the previous year and either (a) have at least nine T2-lesions or (b) have at least one gadolinium lesion at baseline. As explained above, in accordance with the present invention, the patient has primary progressive multiple sclerosis (PPMS) with T1 gadolinium staining lesions.

In certain embodiments, a baseline level in a patient refers to the level prior to administration of or treatment with an anti-CD20 antibody to the patient, for example, about 2 months, about 1.5 months, about 1 month, about 30 days, about 25 days, about 21 days, about 14 days, about 7 days, about 6 days, about 5 days, about 4 days, about 3 days, about 2 days, about 1 day prior to administration of or treatment with an anti-CD20 antibody to the patient.

In certain embodiments, the patient maintains the ability to mount a humoral immune response to an antigen during treatment. In certain embodiments, the antigen is a mumps antigen, a rubella antigen, a varicella antigen, an S. pneumonia antigen, a tetanus toxoid antigen, a pneumococcal antigen, or an influenza antigen.

The anti-CD20 antibody for use in a method as described herein may encompass any combination of the embodiments described herein.

### III. Dosages

According to some embodiments of the anti-CD20 antibody for use in a method as described herein, the method comprises administering an effective amount of the anti-CD20 antibody to the multiple sclerosis patient to provide an initial antibody exposure of about 0.3 to about 4 grams (preferably about 0.3 to about 1.5 grams, such as about 0.6 grams or about 1.0 grams) followed by a second antibody exposure of about 0.3 to about 4 grams (preferably about 0.3 to about 1.5 grams, such as about 0.6 grams or about 1.0 grams), the second antibody exposure not being provided until from about 16 to about 60 weeks from the initial antibody exposure. For purposes of this invention, the second antibody exposure is the next time the patient is treated with the anti-CD20 antibody after the initial antibody exposure, there being no intervening anti-CD20 antibody treatment or exposure between the initial and second exposures. In some embodiments, the initial antibody exposure and/or the second antibody exposure is about any of 0.3 grams, 0.4 grams, 0.5 grams, 0.6 grams, 0.7 grams, 0.8 grams, 0.9 grams, or 1.0 grams.

The interval between the initial and second or subsequent antibody exposures can be measured from the first dose of the initial antibody exposure.

In some embodiments, the antibody exposures are approximately 24 weeks or 6 months apart; or approximately 48 weeks or 12 months apart. In some embodiments, the antibody exposures are approximately about 20-24 weeks or about 5-6 months apart. In some embodiments, "about 20-24 weeks" refers to a time point between 20 weeks and 24 weeks. In some embodiments, "about 20-24 weeks" refers to a variation of a week or 7 days before or after the 24^{th} week. In some embodiments, "about 5-6 months" refers to a time point between 5 and 6 months.

In one embodiment, the second antibody exposure is not provided until about 20 to about 30 weeks from the initial exposure, optionally followed by a third antibody exposure of about 0.3 to about 4 grams (preferably about 0.3 to about 1.5 grams), the third exposure not being administered until from about 46 to 60 weeks (preferably from about 46 to 54 weeks) from the initial exposure, and then, in some embodiments, no further antibody exposure is provided until at least about 70-75 weeks from the initial exposure. In some embodiments, the third antibody exposure is about any of 0.3 grams, 0.4 grams, 0.5 grams, 0.6 grams, 0.7 grams, 0.8 grams, 0.9 grams, or 1.0 grams.

In an alternative embodiment, the second antibody exposure is not provided until about 46 to 60 weeks from the initial exposure, and subsequent antibody exposures, if any, are not provided until about 46 to 60 weeks from the previous antibody exposure.

According to some embodiments of the anti-CD20 antibody for use in a method as described herein, the method comprises administering an effective amount of the anti-CD20 antibody to the multiple sclerosis patient to provide an initial antibody exposure of about 0.3 to about 4 grams (preferably about 0.3 to about 1.5 grams, such as about 0.6 grams or about 1.0 grams) followed by a second antibody exposure of about 0.3 to about 4 grams (preferably about 0.3 to about 1.5 grams, such as about 0.6 grams or about 1.0 grams), the second antibody exposure not being provided until from about 20 to about 30 weeks from the initial antibody exposure, followed by a third antibody exposure of about 0.3 to about 4 grams (preferably about 0.3 to about 1.5 grams, such as about 0.6 grams or about 1.0 grams), the third antibody exposure not being provided until from about 46 to about 54 weeks from the initial exposure, followed by a fourth antibody exposure of about 0.3 to about 4 grams (preferably about 0.3 to about 1.5 grams, such as about 0.6 grams or about 1.0 grams), the fourth antibody exposure not being provided until from about 70 to about 75 weeks from the initial exposure.

In certain embodiments the fourth antibody exposure is followed by one or more antibody exposures of about 0.3 to about 4 grams (preferably about 0.3 to about 1.5 grams, such as about 0.6 grams or about 1.0 grams). In certain embodiments each subsequent antibody exposure is about 20 to about 30 weeks from the previous exposure.

For purposes of this invention, the/each subsequent exposure is the next time the patient is treated with the anti-CD20 antibody after the initial antibody exposure, there being no intervening anti-CD20 antibody treatment or exposure between, e.g., the initial and second exposures, the second and third exposures, or the third and fourth exposures, etc. In some embodiments, the initial, second, third, fourth, and/or subsequent antibody exposure is about any of 0.3 grams, 0.4 grams, 0.5 grams, 0.6 grams, 0.7 grams, 0.8 grams, 0.9 grams, or 1.0 grams.

Any one or more of the antibody exposures herein may be provided to the patient as a single dose of antibody, or as two separate doses of the antibody (i.e., constituting a first and second dose). The particular number of doses (whether one or two) employed for each antibody exposure may be dependent, for example, on the type of MS treated, whether and what type of second medicament is employed, and the method and frequency of administration. Where two separate doses are administered, the second dose is preferably administered from about 3 to 17 days, more preferably from about 6 to 16 days, and most preferably from about 13 to 16 days from the time the first dose was administered. In some embodiments, where two separate doses are administered, the second dose is about 14 days (such as 13 days or 15 days). In some embodiments "about 14 days" refers to a variation of 1 day before or after the 14^{th} day. Where two separate doses are administered, the first and second dose of the antibody is preferably about 0.3 to 1.5 grams, more preferably about 0.3 to about 1.0 grams. In some embodiments, where two separate doses are administered, the first and second dose of the antibody is about any of 0.3 grams, 0.4 grams, 0.5 grams, or 0.6 grams. In some embodiments, the initial ocrelizumab exposure comprises a first dose and a second dose of ocrelizumab, wherein the first dose and second dose of ocrelizumab is about 0.3 grams. In some embodiments, the second ocrelizumab exposure comprises a single dose of ocrelizumab, wherein the single dose of ocrelizumab is 0.6 grams.

In one embodiment, the patient is provided at least about three, at least about four, or at least about five exposures of the antibody, for example, from about 3 to 60 exposures, and more particularly about 3 to 40 exposures, most particularly, about 3 to 20 exposures. In some embodiments of the anti-CD20 antibody for use in a method as described herein, the method further comprises providing between about one to about three subsequent ocrelizumab exposures. In some embodiments, such exposures are administered at intervals each of approximately 24 weeks or 6 months, or 48 weeks or 12 months. In certain embodiments, an interval is shortened by approximately 4 weeks, about 3.5 weeks, about 3 weeks, about 2.5 weeks, about 2 weeks, about 1.5 weeks, about 1 week, about 6 days, about 5 days, about 4 days, about 3 days, about 2 days or about 1 day. In certain embodiments, more than 1 interval is shortened by approximately 4 weeks, about 3.5 weeks, about 3 weeks, about 2.5 weeks, about 2 weeks, about 1.5 weeks, about 1 week, about 6 days, about 5 days, about 4 days, about 3 days, about 2 days or about 1 day. In certain embodiments, an interval is lengthened by approximately 4 weeks, about 3.5 weeks, about 3 weeks, about 2.5 weeks, about 2 weeks, about 1.5 weeks, about 1 week, about 6 days, about 5 days, about 4 days, about 3 days, about 2 days or about 1 day. In certain embodiments, more than one interval is lengthened by approximately 4 weeks, about 3.5 weeks, about 3 weeks, about 2.5 weeks, about 2 weeks, about 1.5 weeks, about 1 week, about 6 days, about 5 days, about 4 days, about 3 days, about 2 days or about 1 day. In certain embodiments, more than 1 interval is shortened by approximately 4 weeks, about 3.5 weeks, about 3 weeks, about 2.5 weeks, about 2 weeks, about 1.5 weeks, about 1 week, about 6 days, about 5 days, about 4 days, about 3 days, about 2 days or about 1 day or lengthened by approximately 4 weeks, about 3.5 weeks, about 3 weeks, about 2.5 weeks, about 2 weeks, about 1.5 weeks, about 1 week, about 6 days, about 5 days, about 4 days, about 3 days, about 2 days or about 1 day.

In one embodiment, each antibody exposure is provided as a single dose of the antibody. In an alternative embodiment, each antibody exposure is provided as two separate doses of the antibody. In some embodiments, some exposures are provided as a single dose or as two separate doses.

As explained above, the antibody to be used in accordance with the invention is ocrelizumab. Other anti-CD20 antibodies are disclosed herein, such as Rituximab, humanized 2H7 (*e.g.* comprising the variable domain sequences in SEQ ID NOS. 2 and 8) or humanized 2H7 comprising the variable domain sequences in SEQ ID NOS. 23 and 24, or huMax-CD20 (Genmab). In accordance with the present invention, the antibody is ocrelizumab (comprising (a) a light chain comprising the amino acid sequence of SEQ ID NO: 13 and (b) a heavy chain comprising the amino acid sequence of SEQ ID NO: 14).

In one embodiment, the patient has never been previously treated with drug(s), such as immunosuppressive agent(s), to treat the multiple sclerosis and/or has never been previously treated with an antibody to a B-cell surface marker (*e.g.* never previously treated with a CD20 antibody).

The antibody is administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated (*see, e.g.,* US Patent Appln No. 2002/0009444, Grillo-Lopez, A concerning intrathecal delivery of a CD20 antibody). In addition, the antibody may suitably be administered by pulse infusion, *e.g*., with declining doses of the antibody. In some embodiments, the dosing is given intravenously, subcutaneously or intrathecally. In some embodiments, the dosing is given by intravenous infusion(s).

In certain embodiments, the patient is premedicated prior to infusion with the anti-CD20 antibody. In certain embodiments, the patient is premedicated with methylprednisolone (or an equivalent) approximately 30 minutes prior to each infusion of anti-CD20 antibody. In certain embodiments, the patient is premedicated with 100 mg IV methylprednisolone (or an equivalent) approximately 30 minutes prior to each infusion of anti-CD20 antibody. In certain embodiments, the patient is additionally (or alternatively) premedicated with an antihistaminic drug (e.g. diphenhydramine) approximately 30-60 minutes before each infusion of anti-CD20 antibody. In certain embodiments, the patient is additionally (or alternatively) premedicated with an antipyretic (e.g. acetaminophen/paracetamol).

While the CD20 antibody may be the only drug administered to the patient to treat the multiple sclerosis, one may optionally administer a second medicament, such as a cytotoxic agent, chemotherapeutic agent, immunosuppressive agent, cytokine, cytokine antagonist or antibody, growth factor, hormone, integrin, integrin antagonist or antibody (*e.g.* an LFA-1 antibody, or an alpha 4 integrin antibody such as natalizumab (TYSABRI^{®}) available from Biogen Idec/Elan Pharmaceuticals, Inc) etc, with the antibody that binds a B cell surface marker (*e.g.* with the CD20 antibody).

In some embodiments of combination therapy, the antibody is combined with an interferon class drug such as IFN-beta-1a (REBIF^{®} and AVONEX^{®}) or IFN-beta-1b (BETASERON^{®}); an oligopeptide such a glatiramer acetate (COPAXONE^{®}); a cytotoxic agent such as mitoxantrone (NOVANTRONE^{®}), methotrexate, cyclophosphamide, chlorambucil, azathioprine; intravenous immunoglobulin (gamma globulin); lymphocyte-depleting therapy (*e.g.,* mitoxantrone, cyclophosphamide, alemtuzumab (Campath^{®}, LEMTRADA^{™}), anti-CD4, cladribine, total body irradiation, bone marrow transplantation); corticosteroid (*e.g.* methylprednisolone, prednisone, dexamethasone, or glucorticoid), including systemic corticosteroid therapy; non-lymphocyte-depleting immunosuppressive therapy (*e.g.,* mycophenolate mofetil (MMF) or cyclosporine); cholesterol-lowering drug of the "statin" class, which includes cerivastatin (BAYCOL^{®}), fluvastatin (LESCOL^{®}), atorvastatin (LIPITOR^{®}), lovastatin (MEVACOR^{®}), pravastatin (PRAVACHOL^{®}), Simvastatin (ZOCOR^{®}); estradiol; testosterone (optionally at elevated dosages; Stuve et al. Neurology 8:290-301 (2002)); hormone replacement therapy; treatment for symptoms secondary or related to MS (*e.g.,* spasticity, incontinence, pain, fatigue); a TNF inhibitor; disease-modifying anti-rheumatic drug (DMARD); non-steroidal anti-inflammatory drug (NSAID); plasmapheresis; levothyroxine; cyclosporin A; somatostatin analogue; cytokine or cytokine receptor antagonist; anti-metabolite; immunosuppressive agent; rehabilitative surgery; radioiodine; thyroidectomy; another B-cell surface antagonist/antibody; etc.

The second medicament is administered with the initial exposure and/or later exposures of the CD20 antibody, such combined administration includes co-administration, using separate formulations or a single pharmaceutical formulation, and consecutive administration in either order, wherein preferably there is a time period while both (or all) active agents simultaneously exert their biological activities.

Aside from administration of antibodies to the patient, the present application contemplates administration of antibodies by gene therapy. Such administration of nucleic acid encoding the antibody is encompassed by the expression administering an "effective amount" of an antibody. *See,* for example, WO96/07321 published March 14, 1996 concerning the use of gene therapy to generate intracellular antibodies.

There are two major approaches to getting the nucleic acid (optionally contained in a vector) into the patient's cells; *in vivo* and *ex vivo.* For *in vivo* delivery the nucleic acid is injected directly into the patient, usually at the site where the antibody is required. For *ex vivo* treatment, the patient's cells are removed, the nucleic acid is introduced into these isolated cells and the modified cells are administered to the patient either directly or, for example, encapsulated within porous membranes that are implanted into the patient (*see, e.g.* U.S. Patent Nos. 4,892,538 and 5,283,187). There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. A commonly used vector for *ex vivo* delivery of the gene is a retrovirus.

In some embodiments, the *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, etc. Where liposomes are employed, proteins that bind to a cell surface membrane protein associated with endocytosis may be used for targeting and/or to facilitate uptake, *e*.*g*. capsid proteins or fragments thereof tropic for a particular cell type, antibodies for proteins that undergo internalization in cycling, and proteins that target intracellular localization and enhance intracellular half life. The technique of receptor-mediated endocytosis is described, for example, by Wu et al., J. Biol. Chem. 262:4429-4432 (1987); and Wagner et al., Proc. Natl. Acad. Sci. USA 87:3410-3414 (1990). For review of the currently known gene marking and gene therapy protocols *see* Anderson et al., Science 256:808-813 (1992). *See also* WO 93/25673 and the references cited therein.

### IV. Antibodies and their Production

The anti-CD20 antibody for use in a method of the present invention is an antibody that binds to a B-cell surface marker, especially one that binds to CD20, namely ocrelizumab. Accordingly, methods for generating ocrelizumab and other (reference) anti-CD20 antibodies will be described here. As explained above, the anti-CD20 antibody to be used in accordance with the invention is ocrelizumab. The preparation of other antibodies is disclosed herein as reference.

In some embodiments, the anti-CD20 antibody for use in the methods described here is produced by a method comprising expressing a nucleic acid encoding a humanized antibody comprising the heavy and light chain amino acid sequences of SEQ ID NO: 14 or 13, respectively, in a host cell, and recovering the humanized antibody or an antigen-binding fragment thereof expressed in the host cell. In some embodiments, the host cell is a mammalian cell (e.g., a CHO cell), an insect cell, or a plant cell. In some embodiments the host cell is a bacterial cell. Methods of producing an anti-CD20 are described in further detail in, e.g., U.S. Patent No. 7,799,900.

The B cell surface marker to be used for production of, or screening for, antibodies may be, *e.g.,* a soluble form of the marker or a portion thereof, containing the desired epitope. Alternatively, or additionally, cells expressing the marker at their cell surface can be used to generate, or screen for, antibodies. Other forms of the B cell surface marker useful for generating antibodies will be apparent to those skilled in the art.

A description follows as to exemplary techniques for the production of the antibodies mentioned herein.

### (i) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e*.*g*., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g.,* 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. In some embodiments, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (ii) Monoclonal antibodies

Monoclonal antibodies are obtained from a population of substantially homogeneous antibodies, *i.e.,* the individual antibodies comprising the population are identical and/or bind the same epitope except for possible variants that arise during production of the monoclonal antibody, such variants generally being present in minor amounts. Thus, the modifier "monoclonal" indicates the character of the antibody as not being a mixture of discrete or polyclonal antibodies.

For example, the monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as herein described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

In some embodiments, the myeloma cells are those that fuse efficiently, support stable high-level production of antibody by the selected antibody-producing cells, and are sensitive to a medium such as HAT medium. Among these, in some embodiments, the myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. In some embodiments, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (*e.g.,* by using oligonucleotide probes that are capable of binding specifically to genes encoding the heavy and light chains of murine antibodies). In some embodiments, the hybridoma cells serve as a source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs., 130:151-188 (1992).

Antibodies or antibody fragments can also be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light chain constant domains in place of the homologous murine sequences (U.S. Patent No. 4,816,567; Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iii) Humanized antibodies

Methods for humanizing non-human antibodies have been described in the art. In some embodiments, a humanized antibody has one or more amino acid residues introduced into it from a source that is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence that is closest to that of the rodent is then accepted as the human framework region (FR) for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chain variable regions. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, humanized antibodies may be prepared by a process of analysis of the parental sequences and various conceptual humanized products using three-dimensional models of the parental and humanized sequences. Three-dimensional immunoglobulin models are commonly available and are familiar to those skilled in the art. Computer programs are available that illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

As explained above, the anti-CD20 antibody to be used in accordance with the invention is ocrelizumab. Other anti-CD20 antibodies are also disclosed herein. For example, the anti-CD20 antibody may be a humanized 2H7 antibody. The humanized 2H7 antibody may comprise one, two, three, four, five or six of CDR sequences as shown in FIG. 1A and 1B. In some embodiments, the humanized 2H7 antibody comprises one, two, three, four, five or six of the following CDR sequences:
CDR L1 sequence RASSSVSYXH wherein X is M or L (SEQ ID NO: 18), for example RASSSVSYMH (SEQ ID NO: 4) (Fig. 1A),
CDR L2 sequence APSNLAS (SEQ ID NO: 5) (Fig. 1A),
CDR L3 sequence QQWXFNPPT wherein X is S or A (SEQ ID NO: 19), for example QQWSFNPPT (SEQ ID NO: 6) (Fig. 1A),
CDR H1 sequence GYTFTSYNMH (SEQ ID NO: 10) (Fig. 1B),
CDR H2 sequence of AIYPGNGXTSYNQKFKG wherein X is D or A (SEQ ID NO: 20), for example AIYPGNGDTSYNQKFKG (SEQ ID NO: 11) (Fig. 1B), and
CDR H3 sequence of VVYYSXXYWYFDV wherein the X at position 6 is N, A, Y, W or D, and the X as position 7 is S or R (SEQ ID NO: 21), for example VVYYSNSYWYFDV (SEQ ID NO: 12) (Fig. 1B).

The CDR sequences above are generally present within human variable light and variable heavy framework sequences, such as substantially the human consensus FR residues of human light chain kappa subgroup I (V_{L}6I), and substantially the human consensus FR residues of human heavy chain subgroup III (V_{H}III). *See also* WO 2004/056312 (Lowman et al.).

In some embodiments, the variable heavy region may be joined to a human IgG chain constant region, wherein the region may be, for example, IgG1 or IgG3, including native sequence and variant constant regions.

In some embodiments, such antibody comprises the variable heavy domain sequence of SEQ ID NO:8 (v16, as shown in Fig. 1B), optionally also comprising the variable light domain sequence of SEQ ID NO:2 (v16, as shown in Fig. 1A), which optionally comprises one or more amino acid substitution(s) at positions 56, 100, and/or 100a, *e.g.* D56A, N100A or N100Y, and/or S100aR in the variable heavy domain and one or more amino acid substitution(s) at positions 32 and/or 92, *e.g.* M32L and/or S92A, in the variable light domain. In some embodiments, the antibody is an intact antibody comprising the light chain amino acid sequences of SEQ ID NOs. 13 or 16, and heavy chain amino acid sequences of SEQ ID NO. 14, 15, 17, 22 or 25. In accordance with the invention, the humanized 2H7 antibody is ocrelizumab (Genentech).

As disclosed herein, a humanized 2H7 may be an intact antibody or antibody fragment comprising the variable light chain sequence: 
DIQMTQSPSSLSASVGDRVTITCRASSSVSYMHWYQQKPGKAPKPLIYAPSNLA
SGVPSRFSGSGSGTDFTLTISSLQPEDFATYYCQQWSFNPPTFGQGTKVEIKR
(SEQ ID NO:2);
 and the variable heavy chain sequence:



 (SEQ ID NO:8).

In some embodiments, the humanized 2H7 antibody is an intact antibody, in some embodiments, it comprises the light chain amino acid sequence: 
DIQMTQSPSSLSASVGDRVTITCRASSSVSYMHWYQQKPGKAPKPLIYAPSNLA
SGVPSRFSGSGSGTDFTLTISSLQPEDFATYYCQQWSFNPPTFGQGTKVEIKRTV
AAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
(SEQ ID NO:13);
 and the heavy chain amino acid sequence: 
EVQLVESGGGLVQPGGSLRLSCAASGYTFTSYNMHWVRQAPGKGLEWVGAIY
PGNGDTSYNQKFKGRFTISVDKSKNTLYLQMNSLRAEDTAVYYCARVVYYSN
SYWYFDVWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLT
CLVKGFYPSDIAVEWESNGQPENNYKTTPPVLDSDGSFFLYSKLTVDKSRWQQ
GNVFSCSVMHEALHNHYTQKSLSLSPGK (SEQ ID NO:14)
 or the heavy chain amino acid sequence: 
EVQLVESGGGLVQPGGSLRLSCAASGYTFTSYNMHWVRQAPGKGLEWVGAIY
PGNGDTSYNQKFKGRFTISVDKSKNTLYLQMNSLRAEDTAVYYCARVVYYSN
SYWYFDVWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLT
 
CLVKGFYPSDIAVEWESNGQPENNYKTTPPVLDSDGSFFLYSKLTVDKSRWQQ
GNVFSCSVMHEALHNHYTQKSLSLSPG (SEQ ID NO:26)
 or the heavy chain amino acid sequence: 
EVQLVESGGGLVQPGGSLRLSCAASGYTFTSYNMHWVRQAPGKGLEWVGAIY
PGNGDTSYNQKFKGRFTISVDKSKNTLYLQMNSLRAEDTAVYYCARVVYYSN
SYWYFDVWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNATYRVVSVLTVLHQD
WLNGKEYKCKVSNKALPAPIAATISKAKGQPREPQVYTLPPSREEMTKNQVSL
TCLVKGFYPSDIAVEWESNGQPENNYKTTPPVLDSDGSFFLYSKLTVDKSRWQ
QGNVFSCSVMHEALHNHYTQKSLSLSPGK (SEQ ID NO:15)
 or the heavy chain amino acid sequence:

EVQLVESGGGLVQPGGSLRLSCAASGYTFTSYNMHWVRQAPGKGLEWVGAIY
PGNGDTSYNQKFKGRFTISVDKSKNTLYLQMNSLRAEDTAVYYCARVVYYSN
SYWYFDVWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNATYRVVSVLTVLHQD
WLNGKEYKCKVSNKALPAPIAATISKAKGQPREPQVYTLPPSREEMTKNQVSL
TCLVKGFYPSDIAVEWESNGQPENNYKTTPPVLDSDGSFFLYSKLTVDKSRWQ
QGNVFSCSVMHEALHNHYTQKSLSLSPG (SEQ ID NO:27).

In some embodiments, the amino acid K at C-terminus of the heavy chain is removed.

In some embodiments, the humanized 2H7 antibody comprises 2H7.v511 variable light domain sequence: 
DIQMTQSPSSLSASVGDRVTITCRASSSVSYLHWYQQKPGKAPKPLIYAPSNLAS
GVPSRFSGSGSGTDFTLTISSLQPEDFATYYCQQWAFNPPTFGQGTKVEIKR
(SEQ ID NO:23)
 and 2H7.v511 variable heavy domain sequence:

EVQLVESGGGLVQPGGSLRLSCAASGYTFTSYNMHWVRQAPGKGLEWVGAIY
PGNGATSYNQKFKGRFTISVDKSKNTLYLQMNSLRAEDTAVYYCARVVYYSY
RYWYFDVWGQGTLVTVSS (SEQ ID NO:24).

In some embodiments, the humanized 2H7.v511 antibody is an intact antibody, it may comprise the light chain amino acid sequence: 
DIQMTQSPSSLSASVGDRVTITCRASSSVSYLHWYQQKPGKAPKPLIYAPSNLA
SGVPSRFSGSGSGTDFTLTISSLQPEDFATYYCQQWAFNPPTFGQGTKVEIKRTV
AAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
(SEQ ID NO:16)
 and the heavy chain amino acid sequence of SEQ ID NO: 17 or:

EVQLVESGGGLVQPGGSLRLSCAASGYTFTSYNMHWVRQAPGKGLEWVGAIY
PGNGATSYNQKFKGRFTISVDKSKNTLYLQMNSLRAEDTAVYYCARVVYYSY
RYWYFDVWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNATYRVVSVLTVLHQD
WLNGKEYKCKVSNAALPAPIAATISKAKGQPREPQVYTLPPSREEMTKNQVSL
TCLVKGFYPSDIAVEWESNGQPENNYKTTPPVLDSDGSFFLYSKLTVDKSRWQ
QGNVFSCSVMHEALHNHYTQKSLSLSPG (SEQ ID NO:25).

In some embodiments, the antibody herein may further comprise at least one amino acid substitution in the Fc region that improves ADCC activity, such as one wherein the amino acid substitutions are at positions 298, 333, and 334, preferably S298A, E333A, and K334A, using Eu numbering of heavy chain residues. *See also* US Patent No. 6,737,056B1, Presta. Any of these antibodies may comprise at least one substitution in the Fc region that improves FcRn binding or serum half-life, for example a substitution at heavy chain position 434, such as N434W. *See also* US Patent No. 6,737,056B1, Presta. Any of these antibodies may further comprise at least one amino acid substitution in the Fc region that increases CDC activity, for example, comprising at least a substitution at position 326, preferably K326A or K326W. *See also* US Patent No. 6,528,624B1 (Idusogie et al.).

In some embodiments, the humanized 2H7 variants are those comprising the variable light domain of SEQ ID NO:2 and the variable heavy domain of SEQ ID NO:8, including those with or without substitutions in an Fc region (if present), and those comprising a variable heavy domain with alteration N100A; or D56A and N100A; or D56A, N100Y, and S100aR; in SEQ ID NO:8 and a variable light domain with alteration M32L; or S92A; or M32L and S92A; in SEQ ID NO:2. M34 in the variable heavy domain of 2H7.v 16 has been identified as a potential source of antibody stability and is another potential candidate for substitution.

In some antibodies disclosed herein, the variable region of variants based on 2H7.v16 comprise the amino acid sequences of v16 except at the positions of amino acid substitutions that are indicated in **Table 1** below. Unless otherwise indicated, the 2H7 variants will have the same light chain as that of v!6.

**Table 1: Exemplary Humanized 2H7 Antibody Variants**

| **2H7 Version** | **Heavy chain (V_{H}) changes** | **Light chain (V_{L}) changes** | **Fc changes** |
|---|---|---|---|
| 16 for reference | | | - |
| 31 | - | - | S298A, E333A, K334A |
| 73 | N100A | M32L | |
| 75 | N100A | M32L | S298A, E333A, K334A |
| 96 | D56A, N100A | S92A | |
| 114 | D56A, N100A | M32L, S92A | S298A, E333A, K334A |
| 115 | D56A, N100A | M32L, S92A | S298A, E333A, K334A, E356D, M358L |
| 116 | D56A, N100A | M32L, S92A | S298A, K334A, K322A |
| 138 | D56A, N100A | M32L, S92A | S298A, E333A, K334A, K326A |
| 477 | D56A, N100A | M32L, S92A | S298A, E333A, K334A, K326A, N434W |
| 375 | - | - | K334L |
| 588 | - | - | S298A, E333A, K334A, K326A |
| 511 | D56A, N100Y, S100aR | M32L, S92A | S298A, E333A, K334A, K326A |

### (iv) Human antibodies

As an alternative to humanization, human antibodies can be generated. For example, it is now possible to produce transgenic animals (*e.g.,* mice) that are capable, upon immunization, of producing a full repertoire of human antibodies in the absence of endogenous immunoglobulin production. For example, it has been described that the homozygous deletion of the antibody heavy chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. *See, e.g.,* Jakobovits et al., Proc. Natl. Acad. Sci. USA, 90:2551 (1993); Jakobovits et al., Nature, 362:255-258 (1993); Bruggermann et al., Year in Immuno., 7:33 (1993); and US Patent Nos. 5,591,669, 5,589,369 and 5,545,807.

Alternatively, phage display technology (McCafferty et al., Nature 348:552-553 (1990)) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review *see, e.g.,* Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). *See also,* US Patent Nos. 5,565,332 and 5,573,905.

Human antibodies may also be generated by *in vitro* activated B cells *(see* US Patents 5,567,610 and 5,229,275).

### (v) Antibody fragments

Various techniques have been developed for the production of antibody fragments. Traditionally, these fragments were derived via proteolytic digestion of intact antibodies *(see, e.g.,* Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). *See* WO 93/16185; US Patent No. 5,571,894; and US Patent No. 5,587,458. The antibody fragment may also be a "linear antibody", *e.g.,* as described in US Patent 5,641,870 for example. Such linear antibody fragments may be monospecific or bispecific.

### (vi) Bispecific antibodies

Bispecific antibodies are antibodies that have binding specificities for at least two different epitopes. Exemplary bispecific antibodies may bind to two different epitopes of the B cell surface marker. Other such antibodies may bind the B cell surface marker and further bind a second different B-cell surface marker. Alternatively, an anti-B cell surface marker binding arm may be combined with an arm that binds to a triggering molecule on a leukocyte such as a T-cell receptor molecule (*e.g.* CD2 or CD3), or Fc receptors for IgG (FcyR), such as FcyRI (CD64), FcyRII (CD32) and FcyRIII (CD16) so as to focus cellular defense mechanisms to the B cell. Bispecific antibodies may also be used to localize cytotoxic agents to the B cell. These antibodies possess a B cell surface marker-binding arm and an arm that binds the cytotoxic agent (*e.g.* saporin, anti-interferon-α, vinca alkaloid, ricin A chain, methotrexate or radioactive isotope hapten). Bispecific antibodies can be prepared as full length antibodies or antibody fragments (*e.g.* F(ab')₂ bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. In some embodiments, the fusion is with an immunoglobulin heavy chain constant domain, comprising at least part of the hinge, CH2, and CH3 regions. In some embodiments, the first heavy chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In some embodiments of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in US Patent No. 5,731,168, the interface between a pair of antibody molecules can be engineered to maximize the percentage of heterodimers that are recovered from recombinant cell culture. In some embodiments, the interface comprises at least a part of the C_{H}3 domain of an antibody constant domain. In this method, one or more small amino acid side chains from the interface of the first antibody molecule are replaced with larger side chains *(*e.g. tyrosine or tryptophan). Compensatory "cavities" of identical or similar size to the large side chain(s) are created on the interface of the second antibody molecule by replacing large amino acid side chains with smaller ones *(e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Various techniques for making and isolating bispecific antibody fragments directly from recombinant cell culture have also been described. For example, bispecific antibodies have been produced using leucine zippers. Kostelny et al., J. Immunol., 148(5): 1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy chain variable domain (V_{H}) connected to a light chain variable domain (V_{L}) by a linker that is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. *See* Gruber et al., J. Immunol., 152:5368 (1994).

Antibodies with more than two valencies are contemplated. For example, trispecific antibodies can be prepared. Tutt et al. J. Immunol. 147: 60 (1991).

### V. Conjugates and Other Modifications of the Antibody

As explained above, the anti-CD20 antibody to be used in accordance with the present invention is ocrelizumab. Other (reference) anti-CD20 antibodies are also disclosed herein. The antibody may be optionally conjugated to a cytotoxic agent. For instance, the antibody may be conjugated to a drug as described in WO2004/032828.

Chemotherapeutic agents useful in the generation of such antibody-cytotoxic agent conjugates have been described above.

Conjugates of an antibody and one or more small molecule toxins, such as a calicheamicin, a maytansine (US Patent No. 5,208,020), a trichothene, and CC1065 are also disclosed herein. For example, the antibody may be conjugated to one or more maytansine molecules (*e.g.* about 1 to about 10 maytansine molecules per antibody molecule). Maytansine may, for example, be converted to May-SS-Me, which may be reduced to May-SH3 and reacted with modified antibody (Chari et al. Cancer Research 52: 127-131 (1992)) to generate a maytansinoid-antibody conjugate.

Alternatively, the antibody is conjugated to one or more calicheamicin molecules. The calicheamicin family of antibiotics is capable of producing double-stranded DNA breaks at sub-picomolar concentrations. Structural analogues of calicheamicin that may be used include, but are not limited to, γ₁^{I}, α₂^{I}, α₃^{I}, N-acetyl-γ₁^{I}, PSAG and θ^{I}₁ (Hinman et al. Cancer Research 53: 3336-3342 (1993) and Lode et al. Cancer Research 58: 2925-2928 (1998)).

Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. *See,* for example, WO 93/21232 published October 28, 1993.

Also disclosed herein is an antibody conjugated with a compound with nucleolytic activity (*e.g.* a ribonuclease or a DNA endonuclease such as a deoxyribonuclease; DNase).

A variety of radioactive isotopes are available for the production of radioconjugated antibodies. Examples include At²¹¹, I¹³¹, I¹²⁵, Y⁹⁰, Re¹⁸⁶, Re¹⁸⁸, Sm¹⁵³, Bi²¹², P³² and radioactive isotopes of Lu.

Conjugates of the antibody and cytotoxic agent may be made using a variety of bifunctional protein coupling agents such as N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See* WO94/11026. The linker may be a "cleavable linker" facilitating release of the cytotoxic drug in the cell. For example, an acid-labile linker, peptidase-sensitive linker, dimethyl linker or disulfide-containing linker (Chari et al. Cancer Research 52: 127-131 (1992)) may be used.

Alternatively, a fusion protein comprising the antibody and cytotoxic agent may be made, *e.g.* by recombinant techniques or peptide synthesis.

In yet another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.* avidin) that is conjugated to a cytotoxic agent (*e.g.* a radionucleotide).

The antibodies may also be conjugated with a prodrug-activating enzyme that converts a prodrug (*e.g.* a peptidyl chemotherapeutic agent, *see* WO81/01145) to an active anti-cancer drug. *See,* for example, WO 88/07378 and U.S. Patent No. 4,975,278.

The enzyme component of such conjugates includes any enzyme capable of acting on a prodrug in such a way so as to convert it into its more active, cytotoxic form.

Enzymes that are useful include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs into free active drugs (*see, e.g.,* Massey, Nature 328: 457-458 (1987)). Antibody-abzyme conjugates can be prepared as described herein for delivery of the abzyme to a tumor cell population.

The enzymes can be covalently bound to the antibody by techniques well known in the art such as the use of the heterobifunctional crosslinking reagents discussed above. Alternatively, fusion proteins comprising at least the antigen binding region of an antibody linked to at least a functionally active portion of an enzyme can be constructed using recombinant DNA techniques well known in the art (*see, e.g.,* Neuberger et al., Nature, 312: 604-608 (1984)).

Other modifications of the antibody are contemplated herein. For example, the antibody may be linked to one of a variety of nonproteinaceous polymers, *e.g.,* polyethylene glycol (PEG), polypropylene glycol, polyoxyalkylenes, or copolymers of polyethylene glycol and polypropylene glycol. In some embodiments, the antibody fragments, such as Fab', are linked to one or more PEG molecules.

The antibodies disclosed herein may also be formulated as liposomes. Liposomes containing the antibody are prepared by methods known in the art, such as described in Epstein et al., Proc. Natl. Acad. Sci. USA, 82:3688 (1985); Hwang et al., Proc. Natl. Acad. Sci. USA, 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of an antibody can be conjugated to the liposomes as described in Martin et al. J. Biol. Chem. 257: 286-288 (1982) via a disulfide interchange reaction. A chemotherapeutic agent is optionally contained within the liposome. *See* Gabizon et al. J. National Cancer Inst. 81(19)1484 (1989).

Amino acid sequence modification(s) of the antibody are contemplated. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid changes also may alter post-translational processes of the antibody, such as changing the number or position of glycosylation sites.

A useful method for identification of certain residues or regions of the antibody that are preferred locations for mutagenesis is called "alanine scanning mutagenesis" as described by Cunningham and Wells Science, 244:1081-1085 (1989). Here, a residue or group of target residues are identified (*e.g.,* charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody of an enzyme, or a polypeptide that increases the serum half-life of the antibody.

Another type of variant is an amino acid substitution variant. These variants have at least one amino acid residue in the antibody molecule replaced by different residue. The sites of greatest interest for substitutional mutagenesis of antibody antibodies include the hypervariable regions, but FR alterations are also contemplated. Conservative substitutions are shown in **Table 2** under the heading of "preferred substitutions". If such substitutions result in a change in biological activity, then more substantial changes, denominated "exemplary substitutions" in **Table 2,** may be introduced and the products screened.

**Table 2**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):
(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody. Generally, the resulting variant(s) selected for further development will have improved biological properties relative to the parent antibody from which they are generated. A convenient way for generating such substitutional variants is affinity maturation using phage display. Briefly, several hypervariable region sites (*e.g.* 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g.* binding affinity) as herein disclosed. In order to identify candidate hypervariable region sites for modification, alanine scanning mutagenesis can be performed to identify hypervariable region residues contributing significantly to antigen binding. Alternatively, or in additionally, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. Such altering includes deleting one or more carbohydrate moieties found in the antibody, and/or adding one or more glycosylation sites that are not present in the antibody.

Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Where the antibody comprises an Fc region, the carbohydrate attached thereto may be altered. For example, antibodies with a mature carbohydrate structure that lacks fucose attached to an Fc region of the antibody are described in US Pat Appl No US 2003/0157108 A1 (Presta, L.); *see also* US 2004/0093621 A1 (Kyowa Hakko Kogyo Co., Ltd) concerning a CD20 antibody composition. Antibodies with a bisecting N-acetylglucosamine (GlcNAc) in the carbohydrate attached to an Fc region of the antibody are referenced in WO03/011878, Jean-Mairet et al. and US Patent No. 6,602,684, Umana et al. Antibodies with at least one galactose residue in the oligosaccharide attached to an Fc region of the antibody are reported in WO97/30087 (Patel et al.); *see also* WO98/58964 (Raju, S.) and WO99/22764 (Raju, S.) concerning antibodies with altered carbohydrate attached to the Fc region thereof.

In some embodiments, the glycosylation variant herein comprises an Fc region, wherein a carbohydrate structure attached to the Fc region lacks fucose. Such variants have improved ADCC function. Optionally, the Fc region further comprises one or more amino acid substitutions therein which further improve ADCC, for example, substitutions at positions 298, 333, and/or 334 of the Fc region (Eu numbering of residues). Examples of publications related to "defucosylated" or "fucose-deficient" antibodies include: US Pat. Appl. No. US 2003/0157108 A1, Presta, L; WO 00/61739A1; WO01/29246A1; US2003/0115614A1; US2002/0164328A1; US2004/0093621A1; US2004/0132140A1; US2004/0110704A1; US2004/0110282A1; US2004/0109865A1; WO03/085119A1; WO03/084570A1; WO2005/035778; WO2005/035586 (describing RNA inhibition (RNAi) of fucosylation); Okazaki et al. J. Mol. Biol. 336:1239-1249 (2004); Yamane-Ohnuki et al. Biotech. Bioeng. 87: 614 (2004). Examples of cell lines producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such as alpha-1,6-fucosyltransferase gene, *FUT8*, knockout CHO cells (Yamane-Ohnuki et al. Biotech. Bioeng. 87: 614 (2004)).

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to modify an antibody with respect to effector function, e.g. so as to enhance antigen-dependent cell-mediated cyotoxicity (ADCC) and/or complement dependent cytotoxicity (CDC) of the antibody. This may be achieved by introducing one or more amino acid substitutions in an Fc region of an antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See* Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al. Cancer Research 53:2560-2565 (1993). Alternatively, an antibody can be engineered that has dual Fc regions and may thereby have enhanced complement lysis and ADCC capabilities. *See* Stevenson et al. Anti-Cancer Drug Design 3:219-230 (1989).

WO00/42072 (Presta, L.) describes antibodies with improved ADCC function in the presence of human effector cells, where the antibodies comprise amino acid substitutions in the Fc region thereof. In some embodiments, the antibody with improved ADCC comprises substitutions at positions 298, 333, and/or 334 of the Fc region. In some embodiments, the altered Fc region is a human IgG1 Fc region comprising or consisting of substitutions at one, two or three of these positions.

Antibodies with altered C1q binding and/or complement dependent cytotoxicity (CDC) are described in WO99/51642, US Patent No. 6,194,551B1, US Patent No. 6,242,195B1, US Patent No. 6,528,624B1 and US Patent No. 6,538,124 (Idusogie et al.)*.* The antibodies comprise an amino acid substitution at one or more of amino acid positions 270, 322, 326, 327, 329, 313, 333 and/or 334 of the Fc region thereof.

To increase the serum half-life of the antibody, one may incorporate a salvage receptor binding epitope into the antibody (especially an antibody fragment) as described in US Patent 5,739,277, for example. As used herein, the term "salvage receptor binding epitope" refers to an epitope of the Fc region of an IgG molecule (e.g., IgG₁, IgG₂, IgG₃, or IgG₄) that is responsible for increasing the *in vivo* serum half-life of the IgG molecule. Antibodies with substitutions in an Fc region thereof and increased serum half-lives are also described in WO00/42072 (Presta, L.).

Engineered antibodies with three or more (preferably four) functional antigen binding sites are also contemplated (US Appln No. US2002/0004587 A1, Miller et al.*).*

### VI. Pharmaceutical Formulations

Therapeutic formulations of the antibody used in accordance with the present invention are prepared for storage by mixing the antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as TWEEN^{™}, PLURONICS^{™} or polyethylene glycol (PEG).

Exemplary anti-CD20 antibody formulations are described in WO98/56418. This publication describes a liquid multidose formulation comprising 40 mg/mL Rituximab, 25 mM acetate, 150 mM trehalose, 0.9% benzyl alcohol, 0.02% polysorbate 20 at pH 5.0 that has a minimum shelf life of two years storage at 2-8BC. Another anti-CD20 formulation of interest comprises 10mg/mL Rituximab in 9.0 mg/mL sodium chloride, 7.35 mg/mL sodium citrate dihydrate, 0.7mg/mL polysorbate 80, and Sterile Water for Injection, pH 6.5. As explained above, the anti-CD20 antibody to be used in accordance with the invention is ocrelizumab.

Lyophilized formulations adapted for subcutaneous administration are described in US Pat No. 6,267,958 (Andya et al.*).* Such lyophilized formulations may be reconstituted with a suitable diluent to a high protein concentration and the reconstituted formulation may be administered subcutaneously to the mammal to be treated herein.

Crystalized forms of the antibody or antibody are also contemplated. *See,* for example, US 2002/0136719A1 (Shenoy et al*.).*

The formulation herein may also contain more than one active compound as necessary for the particular indication being treated, in some embodiments, those with complementary activities that do not adversely affect each other. For example, it may be desirable to further provide a cytotoxic agent; chemotherapeutic agent; immunosuppressive agent; cytokine; cytokine antagonist or antibody; growth factor; hormone; integrin; integrin antagonist or antibody (e.g. an LFA-1 antibody, or an alpha 4 integrin antibody such as natalizumab/TYSABRI^{®}) available from Biogen Idec/Elan Pharmaceuticals, Inc.); interferon class drug such as IFN-beta-1a (REBIF^{®} and AVONEX^{®}) or IFN-beta-1b (BETASERON^{®}); an oligopeptide such a glatiramer acetate (COPAXONE^{®}); a cytotoxic agent such as mitoxantrone (NOVANTRONE^{®}), methotrexate, cyclophosphamide, chlorambucil, or azathioprine; intravenous immunoglobulin (gamma globulin); lymphocyte-depleting drug (*e.g.,* mitoxantrone, cyclophosphamide, Campath, anti-CD4, or cladribine); non-lymphocyte-depleting immunosuppressive drug *(e.g.,* mycophenolate mofetil (MMF) or cyclosporine); cholesterol-lowering drug of the "statin" class; estradiol; testosterone; hormone replacement therapy; drug that treats symptoms secondary or related to MS (*e.g.,* spasticity, incontinence, pain, fatigue); a TNF inhibitor; disease-modifying anti-rheumatic drug (DMARD); non-steroidal anti-inflammatory drug (NSAID); corticosteroid (*e.g.* methylprednisolone, prednisone, dexamethasone, or glucorticoid); levothyroxine; cyclosporin A; somatostatin analogue; cytokine antagonist; anti-metabolite; immunosuppressive agent; integrin antagonist or antibody (*e.g.* an LFA-1 antibody, such as efalizumab or an alpha 4 integrin antibody such as natalizumab); or another B-cell surface antagonist/antibody; etc. in the formulation. The type and effective amounts of such other agents depend, for example, on the amount of antibody present in the formulation, the type of multiple sclerosis being treated, and clinical parameters of the patients. These are generally used in the same dosages and with administration routes as used hereinbefore or about from 1 to 99% of the heretofore employed dosages.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

In some embodiments, the formulation comprises one or more of the group consisting of a histidine buffer, trehalose, sucrose, and polysorbate 20. In some embodiments, the histidine buffer is a histidine-acetate buffer, pH 6.0. Examples of formulations suitable for the administration of the anti-CD20 antibody are found in Andya et al., US2006/0088523.

Exemplary anti-CD20 antibody formulations are described in Andya et al., US2006/0088523 and WO98/56418. In some embodiments, formulation is a liquid multidose formulation comprising the anti-CD20 antibody at 40 mg/mL, 25 mM acetate, 150 mM trehalose, 0.9% benzyl alcohol, 0.02% polysorbate 20 at pH 5.0 that has a minimum shelf life of two years storage at 2-8°C. In some embodiments, anti-CD20 formulation of interest comprises 10mg/mL antibody in 9.0 mg/mL sodium chloride, 7.35 mg/mL sodium citrate dihydrate, 0.7mg/mL polysorbate 80, and Sterile Water for Injection, pH 6.5. In some embodiments, the anti-CD20 antibody is in an aqueous pharmaceutical formulation comprising 10-30 mM sodium acetate from about pH 4.8 to about pH 5.5, preferably at pH5.5, polysorbate as a surfactant in a an amount of about 0.01-0.1% v/v, trehalose at an amount of about 2-10% w/v, and benzyl alcohol as a preservative (U.S. 6,171,586). Lyophilized formulations adapted for subcutaneous administration are described in WO97/04801. Such lyophilized formulations may be reconstituted with a suitable diluent to a high protein concentration and the reconstituted formulation may be administered subcutaneously to the mammal to be treated herein.

In some embodiments, the humanized 2H7 variants formulation is antibody at 12-14 mg/mL in 10 mM histidine, 6% sucrose, 0.02% polysorbate 20, pH 5.8. In a specific embodiment, 2H7 variants and in particular 2H7.v16 is formulated at 20mg/mL antibody in 10mM histidine sulfate, 60mg/ml sucrose., 0.2 mg/ml polysorbate 20, and Sterile Water for Injection, at pH5.8. In a specific embodiment, one IV formulation of humanized 2H7 v16 is: 30mg/ml antibody in 20mM sodium acetate, 4% trehalose dihydrate, 0.02% polysorbate 20 (Tween 20^{™}), pH 5.3. In some embodiments, the humanized 2H7.v511 variant formulation is 15-30mg/ml antibody, preferably 20mg/mL antibody, in 10mM histidine sulfate, 60mg/ml sucrose (6%), 0.2 mg/ml polysorbate 20 (0.02%), and Sterile Water for Injection, at pH5.8. In yet another embodiment, the formulation for 2H7 variants and in particular 2H7.v511 is 20 mg/ml 2H7, 20 mM sodium acetate, 4% trehalose dihydrate, 0.02% polysorbate 20, pH 5.5, for intravenous administration. In some embodiments, 2H7.v 114 formulation is antibody at 15-25 mg/ml, preferably 20mg/ml, in 20mM Sodium Acetate, 240mM (8%) trehalose dihydrate, 0.02% Polysorbate 20, pH 5.3. In some embodiments, the anti-CD20 antibody (e.g., 2H7.v16) is in a formulation comprising 30 mg/mL antibody, 20 mM Sodium Acetate, 106 mM Trehalose, 0.02% polysorbate 20, and pH 5.3. The liquid formulation containing the antibody may be in 300 mg/vial, and may be stored at 2-8°C, protected from light. In some embodiments, prior to administration, the antibody formulation is diluted with normal saline (0.9% Sodium Chloride) in an IV bag for administration by infusion.

### VII. Articles of Manufacture and Kits

The invention relates to an anti-CD20 antibody for use in a method of improving functional ability in a human patient having multiple sclerosis, as further defined in the claims and as described herein above. The anti-CD20 antibody for use in accordance with the invention may also be provided in the form of an article of manufacture or kit. Articles of manufacture or kits (such as kits-of parts) containing materials useful for the treatment of progressive multiple sclerosis are described herein. In some embodiments, the article of manufacture comprising, packaged together, a pharmaceutical composition comprising the anti-CD20 antibody and a pharmaceutically acceptable carrier and a label denoting that the anti-CD20 antibody or pharmaceutical composition is indicated for treating patients with multiple sclerosis and provides an improvement in functional ability in patients having multiple sclerosis.

In some embodiments, the article of manufacture or kit comprises, packaged together, a pharmaceutical composition comprising the anti-CD20 antibody and a pharmaceutically acceptable carrier and a label denoting the anti-CD20 antibody or pharmaceutical composition is indicated for treating patients with highly active multiple sclerosis. In some embodiments, the label further denotes that the anti-CD20 antibody or pharmaceutical composition is indicated for treating patients with highly active multiple sclerosis that have not been previously treated with other therapy for multiple sclerosis. In some embodiments, the label further denotes that the anti-CD20 antibody or pharmaceutical composition is indicated for treating patients with highly active multiple sclerosis that have been previously treated with other therapy for multiple sclerosis. In some embodiments, the label further denotes that the anti-CD20 antibody or pharmaceutical composition is indicated for treating patients with highly active multiple sclerosis that are inadequate responders to other therapy for multiple sclerosis.

In some embodiments, the article of manufacture or kit comprises, packaged together, a pharmaceutical composition comprising the anti-CD20 antibody and a pharmaceutically acceptable carrier and a label denoting the anti-CD20 antibody or pharmaceutical composition is indicated for treating patients with multiple sclerosis and that the anti-CD20 antibody or pharmaceutical composition is effective in one or more of the following: (1) reduction in number of lesions in the brain of the patient; (2) reduction in annualized relapse rate; (3) reduction of disability progression; and (4) improvement of function ability.

In certain embodiments, the packet insert has instructions denoting *(i.e.,* indicating) that an amount of ocrelizumab is administered to the patient that is effective to provide an initial ocrelizumab exposure of between about 0.3 to about 0.6 grams followed by a second ocrelizumab exposure of between about 0.3 to about 0.6 grams, the second exposure not being administered until from about 16 to 60 weeks from the initial exposure, and each of the ocrelizumab exposures is provided to the patient as one or two doses of ocrelizumab. In some embodiments, the initial ocrelizumab exposure is about 0.6 grams. In some embodiments, the second ocrelizumab exposure is about 0.6 grams. In some embodiments, the second exposure is administered from about 20-24 weeks from the initial exposure. In some embodiments, "about 20-24 weeks" refers to a time point between 20 weeks and 24 weeks. In some embodiments, "about 20-24 weeks" refers to a variation of a week or 7 days before or after the 24^{th} week. In some embodiments, one or more of the ocrelizumab exposures are provided to the patient as one dose of ocrelizumab. In some embodiments, one or more of the ocrelizumab exposures are provided to the patient as two doses of ocrelizumab. In some embodiments, the two doses of ocrelizumab comprise about 0.3 grams of ocrelizumab.

In certain embodiments, the article of manufacture or kit comprises a container and a label or package insert on or associated with the container. Suitable containers include, for example, bottles, vials, syringes, etc. The containers may be formed from a variety of materials such as glass or plastic. The container holds or contains a composition that is effective for treating the multiple sclerosis and may have a sterile access port (for example the container may be an intravenous solution bag or a vial having a stopper pierceable by a hypodermic injection needle). At least one active agent in the composition is the antibody. In some embodiments, the container comprises between about 0.3 to about 4.0 grams of the anti-CD20 antibody. In some embodiments, the container comprises between about 0.3 to about 1.5 grams of the anti-CD20 antibody.

The label or package insert indicates that the composition is used for treating multiple sclerosis in a patient suffering therefrom with specific guidance regarding dosing amounts and intervals of antibody and any other drug being provided. The article of manufacture may further comprise a second container comprising a pharmaceutically acceptable diluent buffer, such as bacteriostatic water for injection (BWFI), phosphate-buffered saline, Ringer's solution and dextrose solution. The article of manufacture may further include other materials desirable from a commercial and user standpoint, including other buffers, diluents, filters, needles, and syringes.

Optionally, the article of manufacture or kit provided herein further comprises a container comprising an agent other than the antibody for treatment and further comprising instructions on treating the patient with such agent, such agent preferably being a chemotherapeutic agent or immunosuppressive agent, interferon class drug such as IFN-beta-1a (REBIF^{®} and AVONEX^{®}) or IFN-beta-1b (BETASERON^{®}); an oligopeptide such a glatiramer acetate (COPAXONE^{®}); a cytotoxic agent such as mitoxantrone (NOVANTRONE^{®}), methotrexate, cyclophosphamide, chlorambucil, or azathioprine; intravenous immunoglobulin (gamma globulin); lymphocyte-depleting drug (*e.g.,* mitoxantrone, cyclophosphamide, Campath, anti-CD4, or cladribine); non-lymphocyte-depleting immunosuppressive drug (*e.g.,* mycophenolate mofetil (MMF) or cyclosporine); cholesterol-lowering drug of the "statin" class; estradiol; hormone replacement therapy; drug that treats symptoms secondary or related to MS (*e.g.,* spasticity, incontinence, pain, fatigue); a TNF inhibitor; disease-modifying anti-rheumatic drug (DMARD); non-steroidal anti-inflammatory drug (NSAID); corticosteroid (*e.g.* methylprednisolone, prednisone, dexamethasone, or glucorticoid); levothyroxine; cyclosporin A; somatostatin analogue; cytokine or cytokine receptor antagonist; anti-metabolite; immunosuppressive agent; integrin antagonist or antibody (*e.g.* an LFA-1 antibody, such as efalizumab or an alpha 4 integrin antibody such as natalizumab); and another B-cell surface marker antibody; etc.

### EXAMPLES

### Example 1 (Reference): Phase III Studies of Ocrelizumab in Comparison with Interferon Beta-1a (Rebij) in Patients with Relapsing Multiple Sclerosis

Multiple sclerosis is a heterogeneous disease with an unpredictable disease course and no cure (Scalfari et al. (2013) JAMA Neurol.70, 214-22; Tremlett et al. (2006) Neurology. 66, 172-7; Markowitz (2010) Am J Manag Care. 16, S211-8; Hauser et al. (2013) Ann Neurol. 74, 317-27). Despite various treatments for relapsing forms of multiple sclerosis becoming available in recent years, many patients continue to accrue neurologic disability, thus there remains an important unmet need for more efficacious and well-tolerated treatments (Markowitz (2010) Am J Manag Care. 16, S211-8; Rotstein et al. (2015) JAMA Neurol. 72, 152-8; Sorensen (2007) J Neurol Sci. 259,128-32). In addition, the risk profile of higher efficacy treatments has so far prevented their use early in the disease course (Markowitz (2010) Am J Manag Care. 16, S211-8; Hartung et al. (2011) Expert Rev Neurother. 11, 351-62; Hauser SL. (2015) Mult Scler. 21, 8-21).

B cells are a key contributor to the pathogenesis of multiple sclerosis (Monson (2005) J Neuroimmunol. 158, 170-81; Hauser SL. (2015) Mult Scler. 21, 8-21). B cells are rarely observed in the cerebrospinal fluid of healthy controls, but are frequently found at low percentages in the cerebrospinal fluid of patients with multiple sclerosis (Cepok (2005) Brain. 128, 1667-76; Cross et al. (2011) Biochim Biophys Acta. 1812, 231-8) elevated levels in the cerebrospinal fluid correlate with faster disease progression in relapsing-remitting multiple sclerosis and secondary progressive multiple sclerosis (Cepok (2005) Brain. 128, 1667-76). B cells influence the underlying pathogenesis of multiple sclerosis via a number of functions: antigen presentation (Constant (1999) J Immunol. 162, 5695-703; Crawford et al. (2006) J Immunol. 176, 3498-506), autoantibody production (Bar-Or A (2010) Ann Neurol. 67, 452-61; Duddy (2007) J Immunol. 178, 6092-9), cytokine regulation (Genain et al. (1999) NatMed. 5, 170-5; Storch et al. (1998) Ann Neurol. 43, 465-71), and formation of ectopic lymphoid follicle-like aggregates (Magliozzi et al. (2010) Ann Neurol. 68, 477-93; Serafini et al. (2004) Brain Pathol. 14:164-74. Interest in B cells has increased with proof-of-concept and observational studies and interest in their utility in multiple sclerosis has evolved over time (Hauser et al. (2008) N Engl J Med. 358, 676-88; Kappos et al. (2011) Lancet. 378, 1779-87; Lehmann-Horn et al. (2013) Ther Adv Neurol Disord. 6, 161-73).

CD20 is a cell surface antigen found on pre-B cells, mature, and memory B cells, but it is not expressed on lymphoid stem cells and plasma cells (Stashenko et al. (1980) J Immunol. 125, 1678-85; Loken et al. (1987) Blood. 70, 1316-24; Tedder et al. (1994) Immunol Today. 15, 450-4). In the HERMES study, rituximab, an anti-CD20 chimeric monoclonal antibody, significantly reduced inflammatory brain lesions and clinical relapses compared with placebo in patients with relapsing-remitting multiple sclerosis; thus, providing evidence that selective depletion of CD20⁺ B cells is a potentially effective treatment approach in multiple sclerosis (Kappos et al. (2011) Lancet. 378, 1779-87).

Ocrelizumab is a recombinant humanized monoclonal antibody that selectively depletes CD20-expressing B cells (Klein et al. (2013) MAbs. 5, 337-8; Genovese et al. (2008) Arthritis Rheum. 58, 2652-61) while preserving the capacity for B cell reconstitution and pre-existing humoral immunity (Martin et al. (2006) Annu Rev Immunol. 24, 467-96; DiLillo et al. (2008) J Immunol. 180, 361-71). Ocrelizumab binds to the large extracellular loop of CD20 with high affinity, selectively depleting B cells via several mechanisms including antibody-dependent cell-mediated phagocytosis, antibody-dependent cell-mediated cytotoxicity, complement-dependent cytotoxicity, and induction of apoptosis (Klein et al. (2013) MAbs. 5, 22-33).

Two identical Phase 3, multicenter, randomized, double-blind, double-dummy, parallel-group trials were undertaken (STUDY I and STUDY II) to investigate the efficacy and safety of ocrelizumab compared with interferon (IFN) β-1a in patients with relapsing forms of multiple sclerosis. The results from these two studies are reported here.

### Methods

### Eligibility and Exclusion Criteria

Key eligibility criteria included: an age of 18 to 55 years; a diagnosis of multiple sclerosis in accordance with the 2010 revised McDonald criteria (Polman et al. (2011) "Diagnostic criteria for multiple sclerosis: 2010 revisions to the McDonald criteria." Ann Neurol 69, 292-302); an Expanded Disability Status Scale (EDSS, see world-wide-web.neurostatus.org) score of 0 to 5.5 at screening; at least two documented clinical attacks within the previous 2 years or one clinical attack occurring within the year prior to screening (but not within 30 days prior to screening); documented MRI of brain with abnormalities consistent with multiple sclerosis; neurologic stability for at least 30 days prior to both screening and baseline.

Key exclusion criteria included: a diagnosis of primary progressive multiple sclerosis; patients with a disease duration of more than 10 years in combination with an EDSS score of less than or equal to 2.0 at screening; known presence of other neurological disorders which may mimic multiple sclerosis; pregnancy or lactation; previous treatment with any B-cell targeted therapies or other contraindicated medications (i.e., requirement for chronic treatment with systemic corticosteroids or immunosuppressants during the course of the study, history of or currently active primary or secondary immunodeficiency, active infection, or history of or known presence of recurrent or chronic infection (e.g. hepatitis B or C, HIV, syphilis, tuberculosis), history of progressive multifocal leukoencephalopathy, contraindications to or intolerance of oral or intravenous corticosteroids, or contraindications to Rebif or incompatibility with Rebif use).

### Study Design

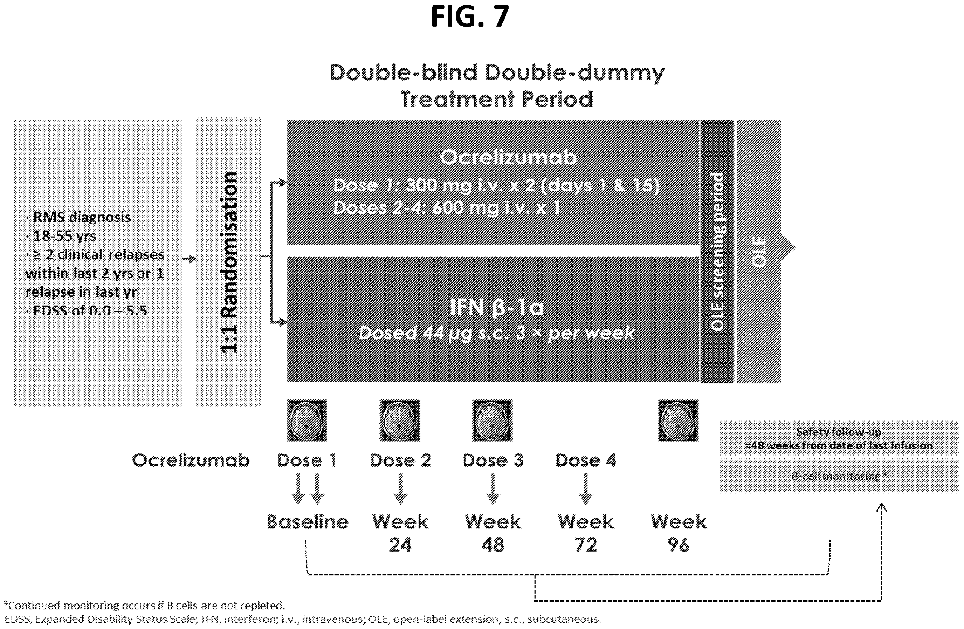

Patients were randomized (1:1) to receive either ocrelizumab 600 mg by intravenous infusion every 24 weeks (administered as two 300-mg infusions on Days 1 and 15 for the first dose, and as a single 600 mg infusion on Day 1 for each 24-week treatment course thereafter), or subcutaneous IFN β-1a three times per week at a dose of 44 µg throughout the 96-week treatment period. *See* **FIG. 7**. Patients in the ocrelizumab group and the IFN β-1a group also received subcutaneous and intravenous placebos, respectively. All patients received intravenous methylprednisolone 100 mg (and optional analgesics/antipyretics and antihistamines) prior to infusion. Randomization was performed centrally by an independent provider. Patients were stratified by region (US/rest of world) and baseline EDSS score (less than 4/greater than or equal to 4).

To maintain concealment of the study-group assignments, each study center had separate treating investigators (neurologists experienced in the care of multiple sclerosis) and examining investigators (neurologists or other healthcare practitioners), all blinded throughout the course of the trial. The treating investigators had access to safety and blinded efficacy data and made treatment decisions based on the patient's clinical response and laboratory findings. The examining investigators conducted the neurologic assessments, including the EDSS scores (Kurtze (1983) "Rating neurologic impairment in multiple sclerosis: an expanded disability status scale (EDSS)." Neurology. 33, 1444-52), the Functional System Scores (Kurtze (1983) "Rating neurologic impairment in multiple sclerosis: an expanded disability status scale (EDSS)." Neurology. 33, 1444-52; Haber and LaRocca, eds. Minimal Record of Disability for multiple sclerosis. New York: National Multiple Sclerosis Society; 1985), the Multiple Sclerosis Functional Composite (MSFC) (Rudick et al. (2002) "The multiple sclerosis functional composite: a new clinical outcome measure for multiple sclerosis trials." Multiple sclerosis (Houndmills, Basingstoke, England) 8, 359-65), Low Contrast Visual Acuity (LCVA) testing (Wieder et al. (2013) "Low contrast visual acuity testing is associated with cognitive performance in multiple sclerosis: a pilot study." BMC Neurology. 13, 167), the Symbol Digit Modalities Test (SDMT) (Smith A. (1982). Symbol digit modalities test: Manual. Los Angeles: Western Psychological Services), and the Karnofsky Performance Status Scale (Mor et al. "The Karnofsky Performance Status Scale. An examination of its reliability and validity in a research setting." Cancer. 53. 2002-2007). MRI assessments were analyzed independently by a central MRI reader blinded to treatment assignment.

Patients who completed the 96-week treatment period were eligible to enter the open-label extension phase of the study. Patients who discontinued prematurely or who did not want to participate in the open-label extension were entered into the 48-week safety follow-up phase, which included B cell monitoring.

### Study Procedures

EDSS scores were determined at screening, baseline, and every 12 weeks; the MSFC, LCVA, and SDMT scores were determined at baseline and every 12 weeks; and Karnofsky Performance Status Scale was determined at baseline and every 24 weeks. MRI was performed at baseline and Weeks 24, 48, and 96. Adverse events were monitored throughout the study.

The primary endpoint was the annualized protocol-defined relapse rate at 96 weeks, wherein relapses were defined as new or worsening neurologic symptoms persisting for over 24 hours that are attributable to multiple sclerosis only. New or worsening neurologic symptoms must have been accompanied by objective neurologic worsening consistent with an increase of at least half a step on the EDSS, of 2 points in one EDSS functional system score, or 1 point in each of two or more EDSS functional system scores. Relapses were reclassified as protocol-defined relapses by an automated algorithm following the rules described above. The algorithm was written before database closure and unblinding of the data.

Key secondary endpoints included: time to onset of 12 week confirmed disability progression (i.e., CDP), defined in the study as an increase of at least 1.0 point from the baseline EDSS score in patients with a baseline score of 5.5 or less, or an increase of a 0.5 point in patients with a baseline score over 5.5, during the 96 weeks, wherein increases in the EDSS were confirmed at a regularly scheduled visit at least 12 weeks after the initial neurologic worsening; total number of T1 gadolinium-enhancing lesions at Weeks 24, 48, and 96; total number of new and/or enlarging T2 hyperintense lesions at Weeks 24, 48, and 96; proportion of patients who have 12-week confirmed disability improvement (i.e., CDI) during the 96 weeks (only analyzed for the subgroup of patients with a baseline EDSS score of at least 2.0); time to onset of 24 week CDP, confirmed at 24 weeks after the initial neurologic worsening, during the 96 weeks; pharmacokinetics, immunogenicity, and pharmacodynamics of ocrelizumab; and safety and tolerability of ocrelizumab. CDI in the study is defined as a reduction in EDSS score of at least 1.0 compared to baseline in patients with a baseline EDSS score of 5.5 or less, or a reduction of a 0.5 point in patients with a baseline EDSS score above 5.5.

Proportion of patients with an EDSS score ≥2.0 who have no evidence of disease activity (NEDA) by Week 96; NEDA analysis in all patients was an exploratory endpoint. Percentage change in brain volume as detected by brain MRI from Week 24 to Week 96; analysis from baseline to Week 96 was also performed as an exploratory analysis.

### Statistical Analysis

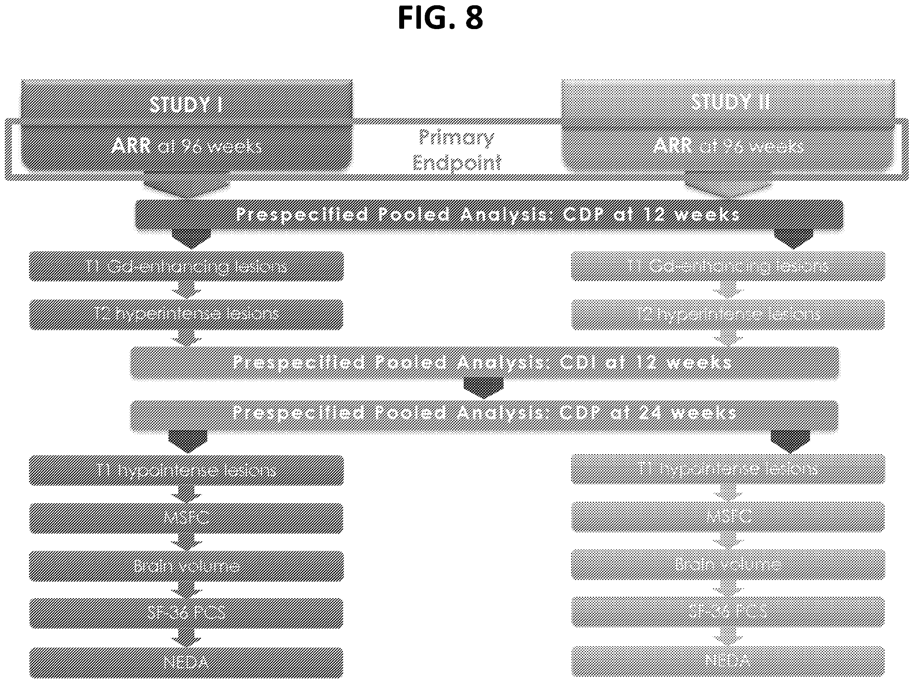

The statistical hierarchy is provided in **FIG. 8**. In order to achieve sufficient statistical power to test the effects of ocrelizumab on CDP and CDI, it was pre-specified that these endpoints would be pooled for both Phase III studies. All other endpoints were analyzed separately for each study. Primary and secondary efficacy analyses were performed on the intention-to-treat population.

All efficacy analyses were performed on the intent-to-treat population. Annualized relapse rate (ARR), the primary efficacy endpoint, was analyzed using a negative binomial model that included, for each patient, the onset between randomization data and date of early treatment-discontinuation/Week 96 in the statistical analysis to take into account the length of exposure, and treatment group, region (USA/rest of world) and baseline EDSS score (less than 4.0/greater than or equal to 4.0) as covariates. A significant result at a two-sided alpha of <0.05 would demonstrate a superior effect of ocrelizumab in reducing ARR compared with IFN β-1a.

The sample size for each of the studies was based on an estimated annualized relapse rate of 0.165 for the ocrelizumab group and 0.33 for the IFN β-1a group. Using a two-sided t-test, it was calculated that a sample of 400 patients per arm would provide 84% statistical power to maintain a type I error rate of 0.05 and detect a relative reduction of 50% on ocrelizumab compared with IFN β-1a (assuming a drop-out rate of approximately 20%). A per-protocol sensitivity analysis evaluated the effect of major protocol violations on the primary endpoint.

Ten secondary efficacy endpoints were tested in hierarchical order of decreasing clinical importance at a two-sided alpha of 0.05 (*see* **FIG. 8**; ARR = annualized relapse rate; CDI = confirmed disability improvement; CDP = confirmed disability progression; Gd = gadolinium; MSFC = Multiple Sclerosis Functional Composite; NEDA = no evidence of disease activity; SF-36 PCS = Short-Form 36, Physical Component Summary). NEDA is defined as: no protocol-defined relapses, no CDP events, no new or enlarging T2 lesions, and no Gd-enhancing T1 lesions. Analyses of secondary efficacy endpoints at the individual study level is as follows:
- For the first secondary efficacy endpoint (the time to onset of confirmed disability progression for at least 12 weeks), the study-level p-value will be interpreted as non-confirmatory, due to inadequate statistical power at the study level to detect relevant treatment differences.
- The second secondary efficacy endpoint (total number of T1 Gd-enhancing lesions at Weeks 24, 48, and 96) will be tested in a confirmatory manner if and only if, in the analysis of both studies combined, the first secondary efficacy endpoint reaches a significance level of 0.05 (i.e., pooled analysis p ≤ 0.05). If, in the analysis of the combined studies, the first secondary efficacy endpoint pooled analysis p > 0.05, then the second and subsequent secondary efficacy endpoint p-values within the hierarchy will be interpreted as non-confirmatory.
- The third secondary efficacy endpoint (total number of new and/or enlarging T2 hyperintense lesions at Weeks 24, 48, and 96) will be tested in a confirmatory manner if and only if the second secondary efficacy endpoint (total number of T1 Gd-enhancing lesions at Weeks 24, 48, and 96) reaches a significance level of 0.05 (i.e., p ≤ 0.05). If the second secondary efficacy endpoint p > 0.05, then the third and subsequent secondary efficacy endpoint p-values within the hierarchy will be interpreted as non-confirmatory.
- For the fourth (proportion of patients who have confirmed disability improvement for at least 12 weeks) and fifth (time to onset of confirmed disability progression for at least 24 weeks) secondary efficacy endpoints, the study-level p-value will be interpreted as non-confirmatory, due to inadequate statistical power at the study level to detect relevant treatment differences.
- The sixth secondary efficacy endpoint (total number of new T1-hypo-intense lesions (chronic black holes) at Weeks 24, 48, and 96) will be tested in a confirmatory manner if and only if, in the analysis of the combined studies, the fifth secondary efficacy endpoint (time to onset of confirmed disability progression for at least 12 weeks) reaches a significance level of 0.05 (i.e., pooled analysis p ≤ 0.05). If, in the analysis of both studies combined, the fifth secondary efficacy endpoint pooled analysis p > 0.05, then the sixth and subsequent secondary efficacy endpoint p-values within the hierarchy will be interpreted as non-confirmatory.
- The seventh (and subsequent) secondary efficacy endpoint will be tested in a confirmatory manner if and only if the sixth (or immediately previous) secondary efficacy endpoint reaches a significance level of 0.05 (i.e., p ≤ 0.05). If the sixth (or immediately previous) secondary efficacy endpoint p > 0.05, then the seventh (or current) and all subsequent secondary efficacy endpoint p-values within the hierarchy will be interpreted as non-confirmatory.

Furthermore, for analyses of secondary efficacy endpoints where data from both studies are combined (so there is sufficient statistical power for all primary and secondary efficacy endpoint comparisons):
- The first secondary efficacy endpoint (the time to onset of confirmed disability progression for at least 12 weeks) will be tested in a confirmatory manner if and only if the primary efficacy endpoint (annualized protocol-defined relapse rate by 2 years), reaches a significance level of 0.05 (i.e., pooled analysis p ≤ 0.05). If the primary efficacy endpoint pooled analysis p > 0.05, then all secondary efficacy endpoint pooled analysis p-values within the hierarchy will be interpreted as non-confirmatory.
- The second (and subsequent) secondary efficacy endpoint will be tested in a confirmatory manner if and only if the first (or immediately previous) secondary efficacy endpoint reaches a significance level of 0.05 (i.e., pooled analysis p ≤ 0.05). If the first (or immediately previous) secondary efficacy endpoint pooled analysis p > 0.05, then the second (or current) and all subsequent secondary efficacy endpoint pooled analysis p-values within the hierarchy will be interpreted as non-confirmatory.

The safety population was used for all analyses of safety data and included all patients who have received any study treatment.

### Results

### Patients

Overall, 1656 patients were enrolled into the first (N=821) and second (N=835) Phase III studies (intention-to-treat population). Baseline demographics and disease characteristics were similar among the populations within each study and between the two studies (*see* **Table 3**).

**Table 3. Baseline demographics and disease characteristics.**

| **CHARACTERISTIC** | | **STUDY I** | | **STUDY II** | |
|---|---|---|---|---|---|
| | | **IFN β-1a 44 µg (n=411)** | **Ocrelizumab 600 mg (n=410)** | **IFN β-1a 44 µg (n=418)** | **Ocrelizumab 600 mg (n=417)** |
| **Age, yr, mean (SD)** | | 36.9 (9.3) | 37.1 (9.3) | 37.4 (9.0) | 37.2 (9.1) |
| **Female sex, n (%)** | | 272 (66.2) | 270 (65.9) | 280 (67.0) | 271 (65.0) |
| **Time since onset, years, mean (SD)** | | 6.3 (6.0) | 6.7 (6.4) | 6.7 (6.1) | 6.7 (6.1) |
| **Time since diagnosis years, mean (SD)** | | 3.7 (3.6) | 3.8 (4.8) | 4.1 (5.1) | 4.2 (5.0) |
| **Relapses in previous 12 months, mean (SD)** | | 1.3 (0.6) | 1.3 (0.7) | 1.3 (0.7) | 1.3 (0.7) |
| **Previously untreated, no. (%)†** | | 292 (71.4) | 301 (73.8) | 314 (75.3) | 304 (72.9) |
| **Mean score on EDSS** | | 2.8 (1.3) | 2.9 (1.2) | 2.8 (1.4) | 2.8 (1.3) |
| **T1 gadolinium-enhancing lesions - no. (%)** | | | | | |
| | **0** | 252 (61.9) | 233 (57.5) | 243 (58.6) | 252 (61.0) |
| | **1** | 52 (12.8) | 64 (15.8) | 62 (14.9) | 58 (14.0) |
| | **2** | 30 (7.4) | 30 (7.4) | 38 (9.2) | 33 (8.0) |
| | **3** | 16 (3.9) | 16 (3.9) | 14 (3.4) | 15 (3.6) |
| | **>4** | 57 (14.0) | 57 (14.0) | 58 (14.0) | 55 (13.3) |
| **T2 lesions - no.** | | 51.06±39.91 | 51.04±39.00 | 51.01±35.69 | 49.26±38.59 |
| **Volume of T2 lesions - cm³** | | 9.74±11.28 | 10.84±13.90 | 10.61±12.30 | 10.73±14.28 |
| **Normalized brain volume - cm³** | | 1499.18±87.68 | 1500.93±84.10 | 1501±90.98 | 1503±92.63 |
| **Patients with no Gd⁺ lesions, n (%)** | | 252 (61.9) | 233 (57.5) | 243 (58.6) | 252 (61.0) |
| **Patients with Gd⁺ lesions, n (%)** | 155 (38.1) | 172 (42.5) | 172 (41.4) | 161 (39.0) | |
| **Number Gd⁺ T1 lesions, mean (SD)** | 1.9 (5.2) | 1.7 (4.2) | 2.0 (4.9) | 1.8 (5.0) | |
| **Number T2 lesions, mean (SD)** | 51.1 (39.9) | 51.0 (39.0) | 51.0 (35.7) | 49.3 (38.6) | |

| | | | | | |
|---|---|---|---|---|---|
| †Patients untreated with disease modifying therapy in the 2 years prior to study entry | | | | | |

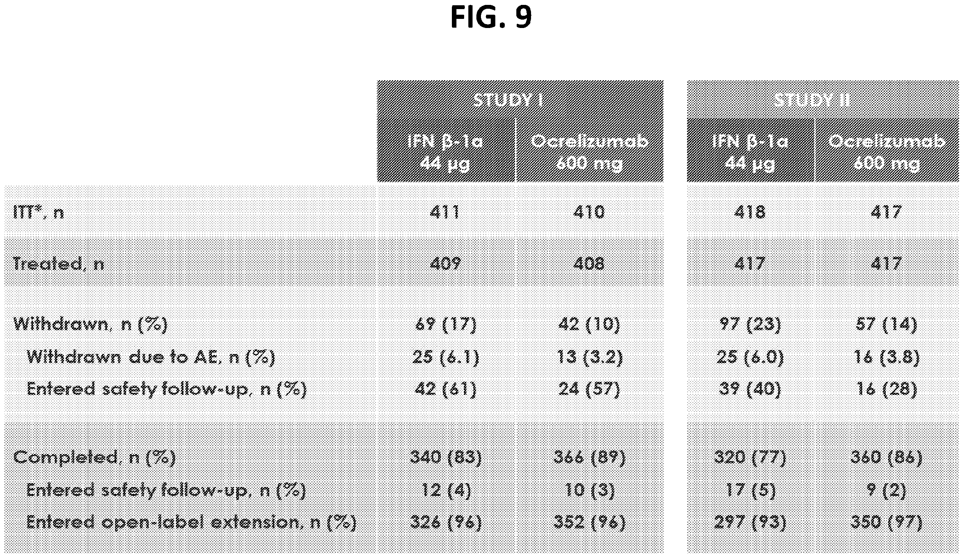

A total of 366 (89%) and 340 (83%) patients in the ocrelizumab and IFN β-1a arms, respectively, completed the first study (*see* **FIG. 9****),** and 360 (86%) and 320 (77%) in the ocrelizumab and IFN β-1a arms in the second study (*see* **FIG. 9****).** Over 85% of the patients in the ocrelizumab arms completed STUDY I and STUDY II. All randomized patients were included in the ITT population. Patients that withdrew prematurely from the studies for any reason and patients for whom assessments were not performed for any reason were still included in the ITT (intent-to-treat) analysis.

### Efficacy

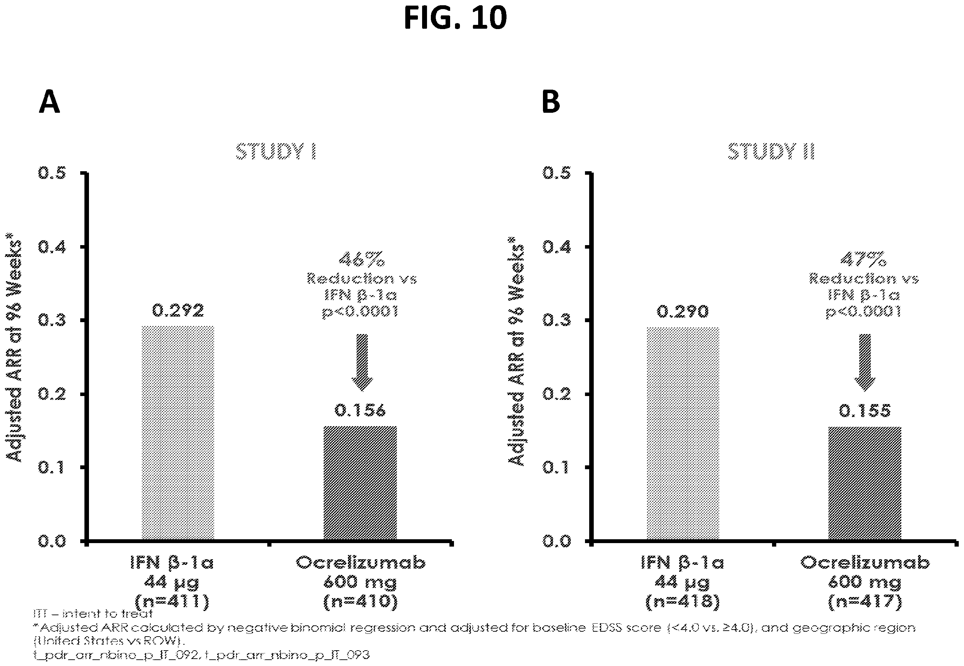

The clinical and MRI outcomes from the first and second studies are summarized in **Table 4.** The frequency of relapses of multiple sclerosis was reduced by ocrelizumab in both studies, with an adjusted annualized relapse (ARR) rate at 96 weeks (primary endpoint) of 0.156 for ocrelizumab (versus 0.292 for IFN β-1a) in STUDY I and 0.155 for ocrelizumab (versus 0.290 for IFN β-1a) in STUDY II *(see* **Table 4** and **FIGs. 10A** and **10B****).** Annualized relapse rates (based on protocol-defined relapses) in patients treated with ocrelizumab were reduced by 46% and 47% versus IFN β-1 in STUDY I and STUDY II, respectively (p<0.0001 for both comparisons). Annualized relapse rates (based on protocol-defined relapses) in patients treated with ocrelizumab were reduced by 46% versus IFN β-1 (STUDY I and STUDY II, pooled data; p=0.0001). Adjusted ARR was calculated by binomial regression and adjusted for baseline EDSS score (<4.0 vs. ≥ 4.0) and geographic location (US vs. rest of world). Annualized relapse rates (based on all clinical relapses) in patients treated with ocrelizumab were reduced by 42% (p=0.001) and 47% (p<0.0001) versus IFN β-1 in STUDY I and STUDY II, respectively.

**Table 4. Clinical and MRI endpoints during the 96-week study (ITTpopulation) ***

| **Endpoint** | | **Pooled STUDY I and II** | | **STUDY I** | | **STUDY II** | | |
|---|---|---|---|---|---|---|---|---|
| | | IFN β-1a 44 µg (n=829) | Ocrelizumab 600 mg (n=827) | IFN β-1a 44 µg (n=411) | Ocrelizumab 600 mg (n=410) | IFN β-1a 44 µg (n=418) | Ocrelizumab 600 mg (n=417) | |
| **Clinical endpoints** | | | | | | | | |
| Annualized relapse rate at 96-weeks | | | | | | | | |
| | Rate (95% CI) | | | 0.292 (0.235-0.361) | 0.156 (0.122-0.200) | 0.290 (0.234-0.361) | 0.155 (0.121-0.198) | |
| value) | Risk reduction vs. IFN β-1a - % (95% CI; p- | | 46 (p<0.0001) | | 46 (p<0.0001) | | 47 (p<0.0001) | |
| **Confirmed disability progression for at least 12 weeks^{†}** | | | | | | | | |
| | **Patients with event - %** | **13.6** | **9.1** | | | | | |
| | **Hazard ratio vs. IFN β-1a (95% CI)** | | **0.60 (0.45-0.81)** | | **0.57 (0.37-0.90)** | | **0.63 (0.42-0.92)** | |
| | **Risk reduction vs. IFN β-1a - % (p-value)** | | **40 (p=0.0006)** | | **43 (p=0.0139)** | | **37 (p<0.0169)** | |
| **Confirmed disability progression for at least 24 weeks^{†}** | | | | | | | | |
| | **Patients with event - %** | **10.5** | **6.9** | | | | | |
| | **Hazard ratio vs. IFN β-1a (95% CI)** | | **0.60 (0.43-0.84)** | | **0.57 (0.34-0.95)** | | **0.63 (0.40-0.98)** | |
| | **Risk reduction vs. IFN β-1a - % (p-value)** | | **40 (p=0.0025)** | | **43 (p=0.0278)** | | **37 (p=0.0370)** | |
| **Confirmed disability improvement for at least 12 weeks^{†,‡}** | | **614** | **628** | | | | | |
| | **No. of patients evaluated** | **15.6** | **20.7** | | | | | |
| | **Patients with improvement sustained for 12 weeks- %** | | **33 (p=0.0194)** | | **61 (p=0.0106)** | | **14 (p=0.4019)** | |
| | **Relative improvement vs. IFN β-1a - % (p-value)** | | | | | | | |
| Multiple Sclerosis Functional Composite score | | | | | | | | |
| | | Mean change in MSFC score from baseline to Week 96 | | | 0.174±0.031 | 0.213±0.031) | 0.169±0.029 | 0.276±0.028 |
| | | Difference in adjusted mean (95% CI; p-value) | | | | 0.039±0.039 (-0.039-0.166; p = 0.3261) | | 0.107±0.037 (0.034-0.180; p=0040) |
| NEDA by Week 96^{‡,§} | | | | | | | | |
| | | No. of patients evaluated | | | 291 | 289 | 270 | 289 |
| | | Patients with NEDA - % | | | 27.1 | 47.4 | 24.1 | 43.9 |
| | | Relative improvement vs. IFN β-1a - % (p-value) | | | | 74 (p<0.0001) | | 81 (p<0.0001) |
| NEDA components by Wk 96^{‡,§} - % | | | | | | | | |
| | Patients relapse free | | | | 66.7 | 80.4 | 64.3 | 78.9 |
| | Patients free from confirmed disability progression | | | | 87.8 | 92.4 | 84.9 | 89.4 |
| | Patients free from T1 gadolinium-enhancing lesions | | | | 69.8 | 91.7 | 63.9 | 90.2 |
| | Patients free from new and/or enlarging T2 | | | | | | | |
| | hyperintense lesions | | | | 38.7 | 61.7 | 38.0 | 60.9 |

| **MRI endpoints** | | | | | | | | |
|---|---|---|---|---|---|---|---|---|
| T1 gadolinium-enhancing lesions at Weeks 24, 48, and 96^{¶} | | | | | | | | |
| | | Patients with any T1 gadolinium-enhancing lesions | | | 30.2 | 8.3 | 36.1 | 9.8 |
| | | Mean no. of lesions (95% CI) | | | 0.286 (0.200-0.409) | 0.016 (0.0009-0.030) | 0.416 (0.309-0.561) | 0.021 (0.012-0.036) |
| | | Risk reduction vs. IFN β-1a - % (p-value) | | | | 94 (p<0.0001) | | 95 (p<0.0001) |
| New and/or enlarging T2 hyperintense lesions at Weeks 24, 48, and 96^{¶} | | | | | | | | |
| | | Patients with any new and/or enlarging T2 | | | 61.3 | 38.3 | 62.0 | 39.1 |
| | | hyperintense lesions - % | | | | | | |
| | | Mean no. of lesions (95% CI) | | | 1.413 (1.123-1.777) | 0.323 (0.256-0.407) | 1.904 (1.536-2.359) | 0.325 (0.259-0.409) |
| | | Rate Ratio (95% CI) | | | | 0.229 (0.174-0.300) | | 0.171 (0.130-0.225) |
| | | Risk reduction vs. IFN β-1a - % (p-value) | | | | 77 (P<0.0001) | | 83 (P<0.0001) |
| T1 hypointense lesions at Weeks 24, 48, and 96* | | | | | | | | |
| | | Mean no. of lesions (95% CI) | | | 0.982 (0.780-1.237) | 0.420 (0.337-0.524) | 1.255 (1.003-1.571) | 0.449 (0.357-0.560) |
| | | Rate Ratio (95% CI) | | | | 0.428 (0.328-0.557) | | 0.357 (0.272-0.470) |
| | | Risk reduction vs. IFN β-1a - % (p-value) | | | | 57 (P<0.0001) | | 64 (P<0.0001) |
| Brain volume from Week 24-96 | | | | | | | | |
| | | Mean change | | | -0.741±0.046 | -0.572±0.044 | -0.750±0.051 | 0.638±0.049 |
| | | p-value | | | | P=0.0042 | | P=0.0900 |
| | | Mean difference in adjusted means (95% CI) | | | | 0.168 (0.053-0.283) | | 0.112 (-0.018-0.241) |
| Brain volume from baseline to Wk 96** | | | | | | | | |
| | | Mean percentage change | | | -1.233±0.055 | -0.943±0.054 | -1.354±0.064 | -1.032±0.062 |
| | | p-value | | | | p<0.0001 | | p<0.0001 |
| | | Mean difference in adjusted means (95% CI) | | | | 0.290 (0.148-0.432) | | 0.322 (0.156-0.488) |

| **Patient-reported outcomes** | | | | | | | | |
|---|---|---|---|---|---|---|---|---|
| Short-form-36 physical component summary from baseline to Week 96 | | | | | | | | |
| | | Mean | | | 45.399±0.529 | 45.065±0.507 | 44.552±0.544 | 44.307±0.541 |
| | | p-value | | | | P=0.2193 | | P=0.0404 |
| | | Difference in adjusted means | | | | 0.693 (-0.414-1.800) | | 1.159 (0.051-2.269) |

| | | | | | | | | |
|---|---|---|---|---|---|---|---|---|
| *Plus-minus values are means ± standard deviation †Bold text indicates pre-specified pooled endpoints †In patients with baseline EDSS score at least 2.0 §NEDA is defined as: no protocol-defined relapses, no CDP events, no new or enlarging T2 lesions, and no gadolinium-enhancing lesions Adjusted by baseline lesion count, EDSS (<4.0 / ≥4.0), and geographical region (US / ROW) | | | | | | | | |

### Disability

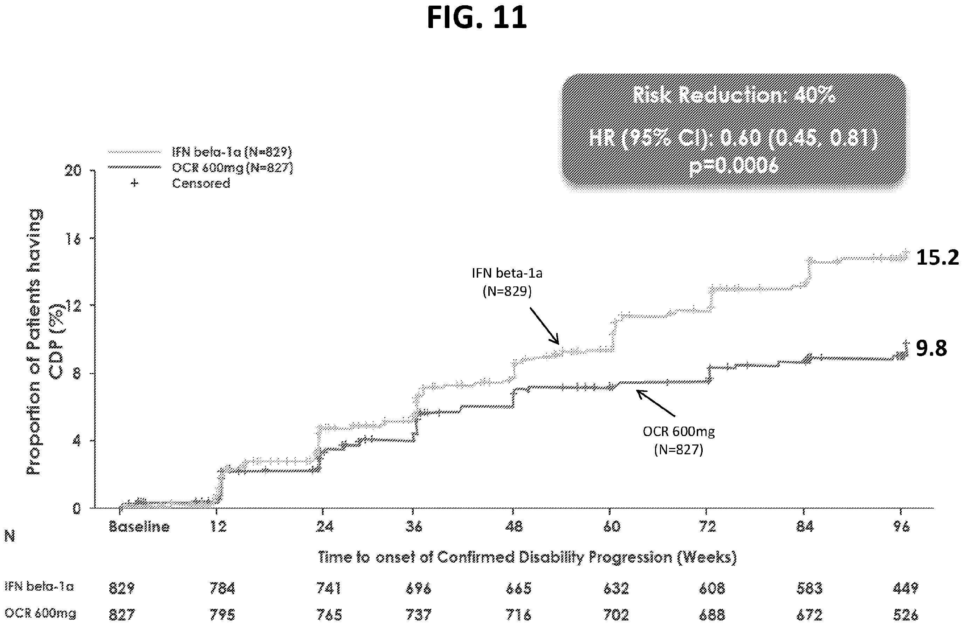

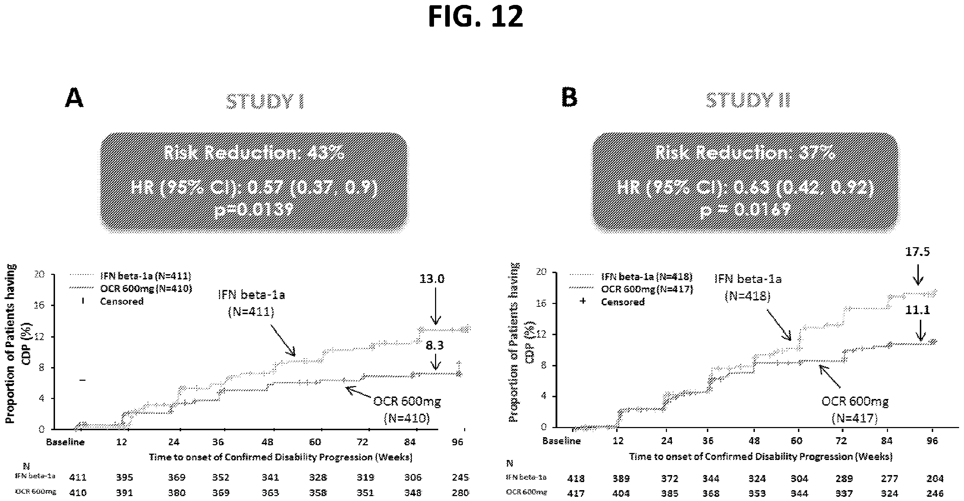

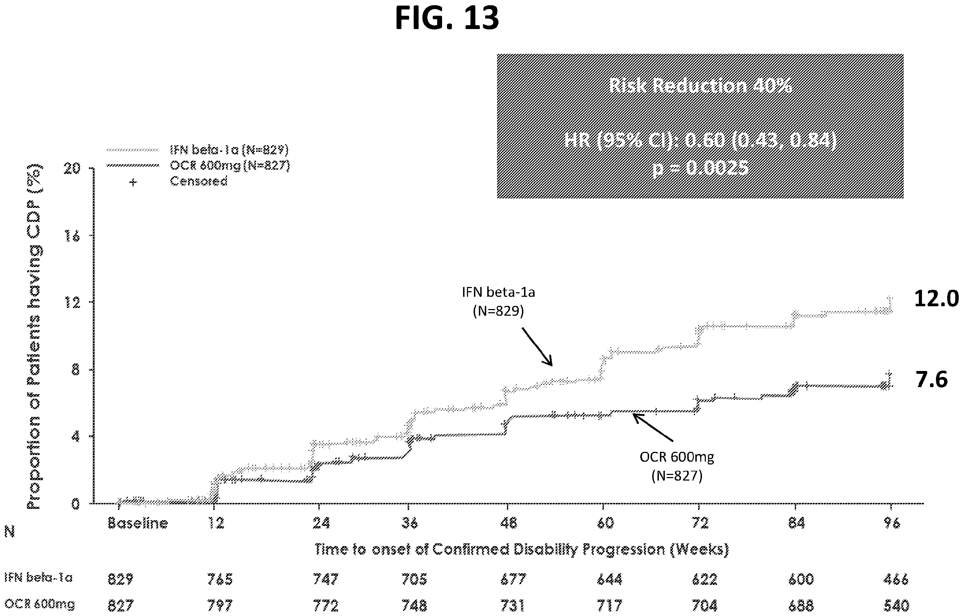

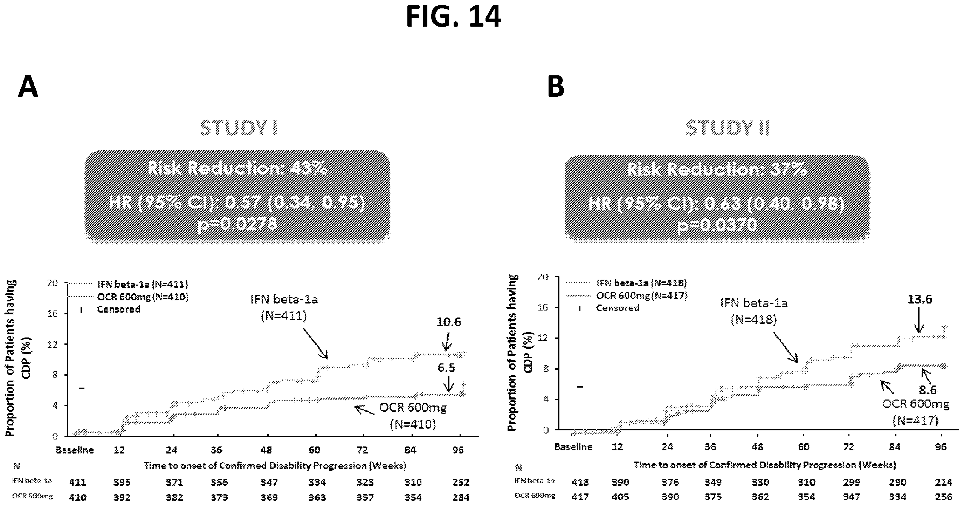

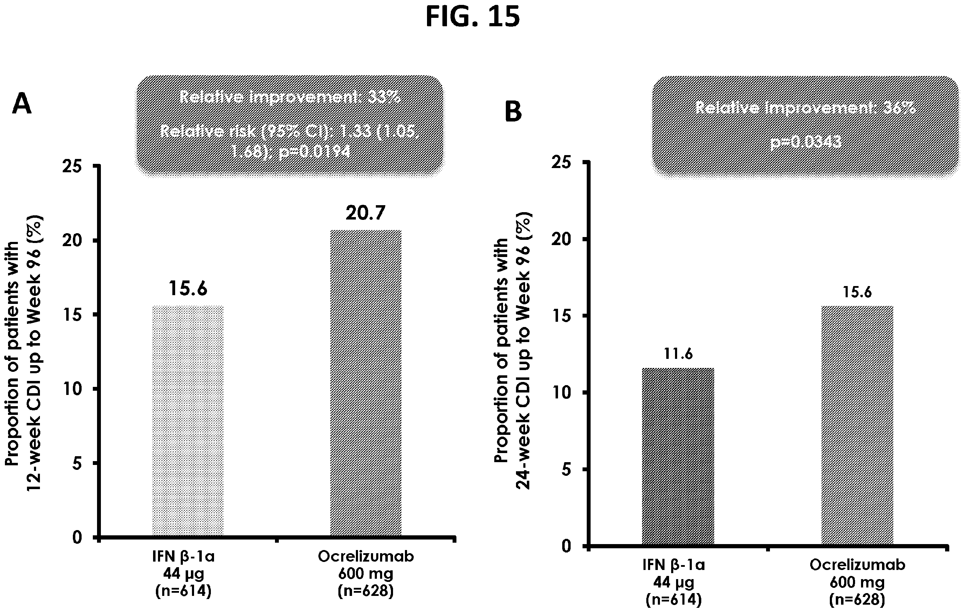

As compared with IFN β-1a, ocrelizumab reduced the risk of confirmed disability progression (i.e., CDP) that was sustained for 12 weeks by 40% over the 96-week study period (STUDY I and STUDY II, pooled data; p=0.0006; **FIG. 11****,** CI = confidence interval; HR = hazard ratio). Unpooled data for STUDY I and STUDY II showing that ocrelizumab reduced the risk of confirmed disability progression (*i.e.,* CDP) that was sustained for 12 weeks as compared to IFN β-1a are shown in **FIG. 12A** (STUDY I) and **FIG. 12B** (STUDY II). Ocrelizumab also reduced the risk of CDP that was sustained for 24 weeks by 40% versus IFN β-1a over the 96-week study period (STUDY I and STUDY II, pooled data; p=0.0025; **FIG. 13****).** Unpooled data for STUDY I and STUDY II showing that ocrelizumab reduced the risk of confirmed disability progression (i.e., CDP) that was sustained for 24 weeks as compared to IFN β-1a is shown in **FIG. 14A** and **FIG. 14B****.** The proportion of patients (with baseline EDSS score of 2.0 or more) with confirmed disability improvement (i.e., CDI) for at least 12 weeks was 20.7% in patients receiving ocrelizumab (n=628) versus 15.6% in patients receiving IFN β-1a (n=614), representing a risk improvement of 33% with ocrelizumab (STUDY I and STUDY II, pooled data; p=0.0194). *See* **FIG. 15A****.** The proportion of patients (with baseline EDSS score of 2.0 or more) with confirmed disability improvement (i.e., CDI) for at least 12 weeks was 15.6% in patients receiving ocrelizumab (n=628) versus 11.6% in patients receiving IFN β-1a (n=614), representing a risk improvement of 36% with ocrelizumab (STUDY I and STUDY II, pooled data; p=0.0343). *See* **FIG. 15B****.** For patients with a baseline EDSS score ≥ 2.0 and ≤ 5.5, disability improvement was defined as a reduction in EDSS score ≥ 1.0 point compared to baseline EDSS score. For patients with a baseline EDSS score > 5.5, disability improvement was defined as a reduction in EDSS score ≥ 0.5 point p value for relative improvement is from the Cochran-Mantel-Haenszel Chi-Squared test, stratified by study, baseline EDSS score (<4.0 vs ≥4.0), and geographic region (US vs rest of world), and includes stratification factors. Patients with missing EDSS or no confirmation after onset of disability improvement are counted as not having CDI.

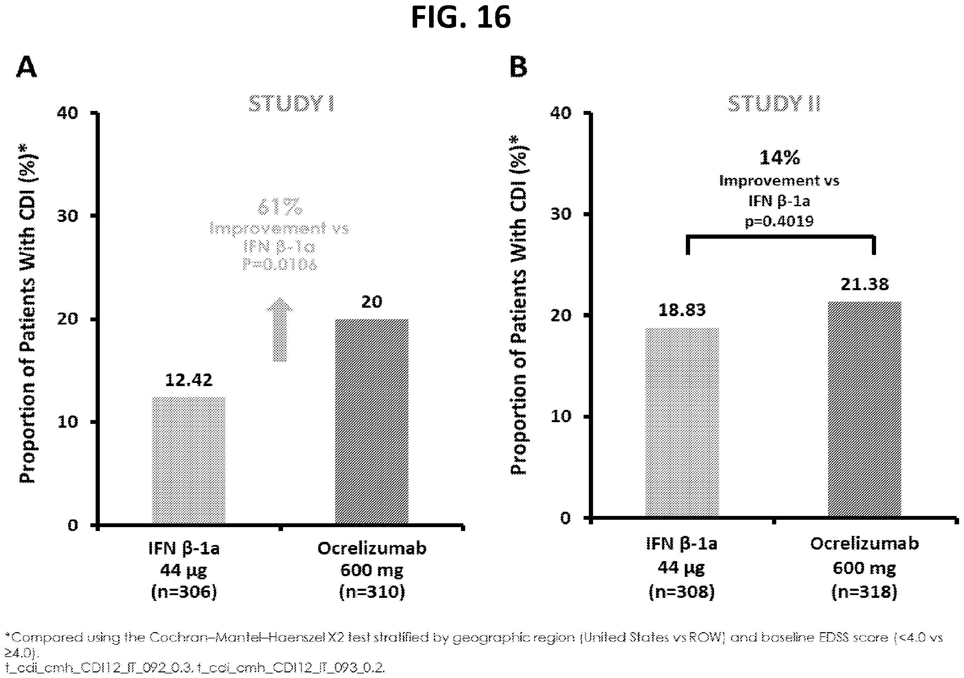

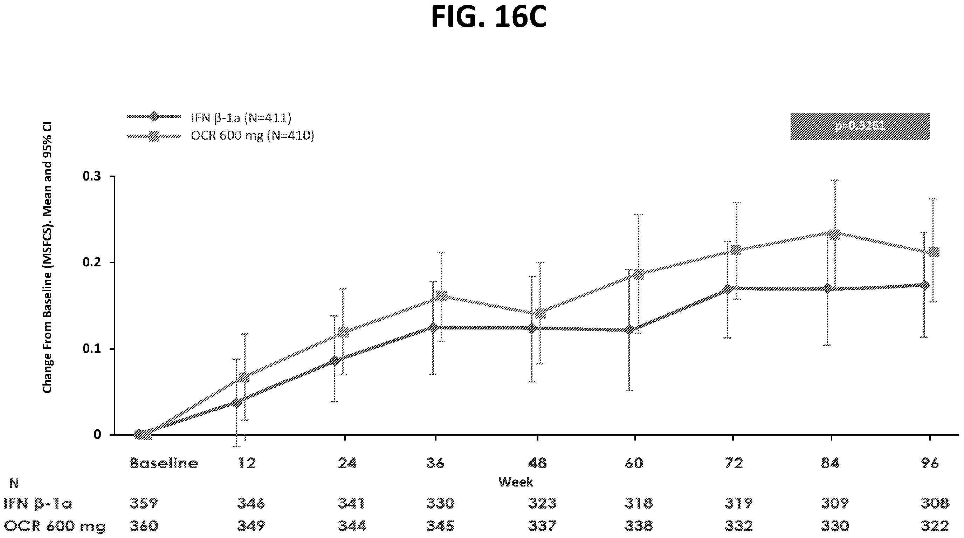

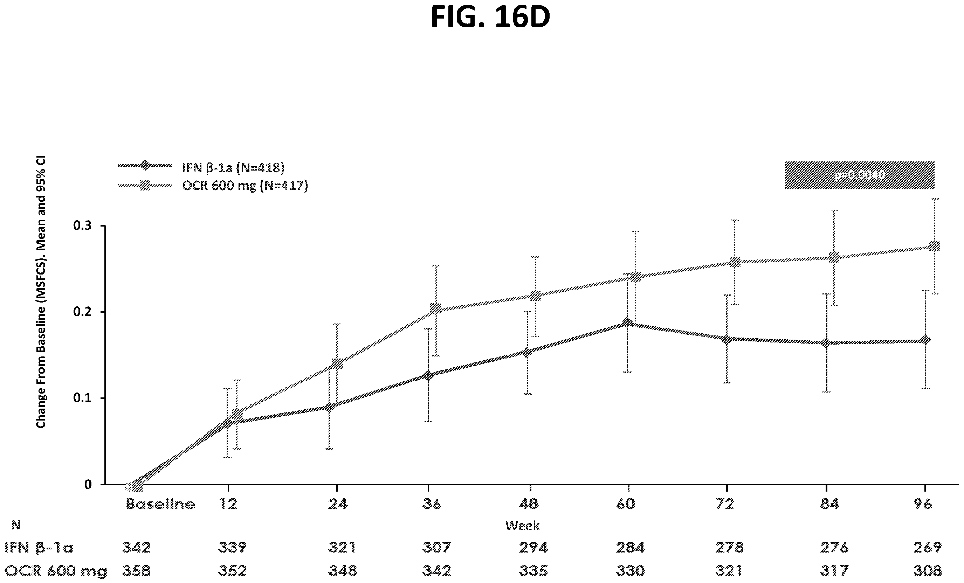

Unpooled data for the proportion of patients (with baseline EDSS score of 2.0 or more) with confirmed disability improvement for at least 12 weeks in STUDY I and STUDY II are provided in **FIG. 16A** and **FIG. 16B****.** Unpooled data for Multiple Sclerosis Functional Composite score outputs are provided in **FIGs. 16C** and **16D****.** **FIGs. 16C** and **16D** include patients with assessment at baseline and at least one post-baseline value. Estimates are from analysis based on mixed-effect model of repeated measures (MMRM) using unstructured covariance matrix: Change = Baseline MSFCS Score + Geographic Region (US vs Rest of World) + Baseline EDSS (<4.0 vs >=4.0) + Week + Treatment + Treatment*Week (repeated values over week) + Baseline MSFCS Score*Week. 15.6% of ocrelizumab-treated patients achieved CDI at 24 weeks (vs. 11.6% for IFNβ-1a-treated patients), representing a 36% relative improvement (relative risk 1.36 [p=0.0343]).

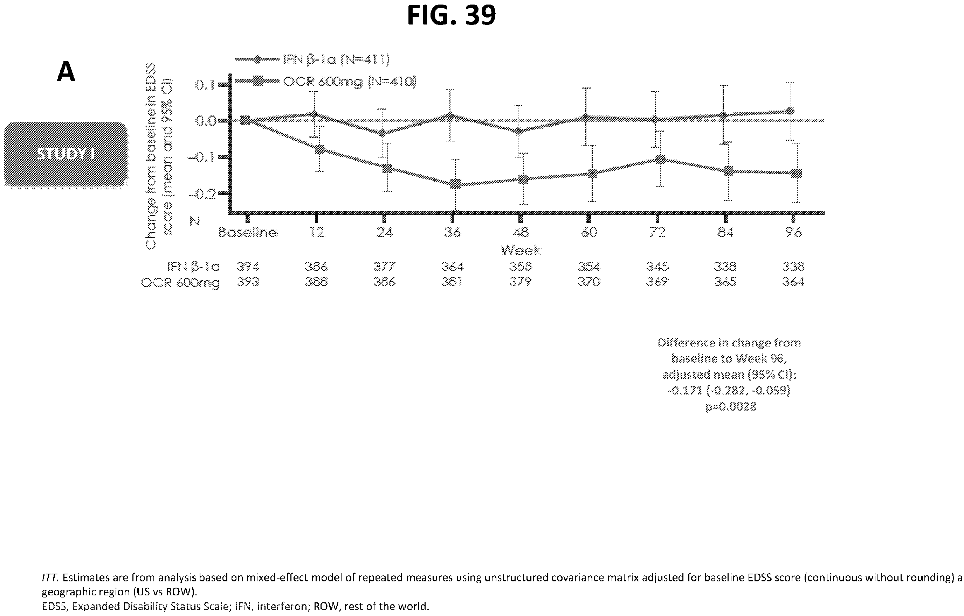

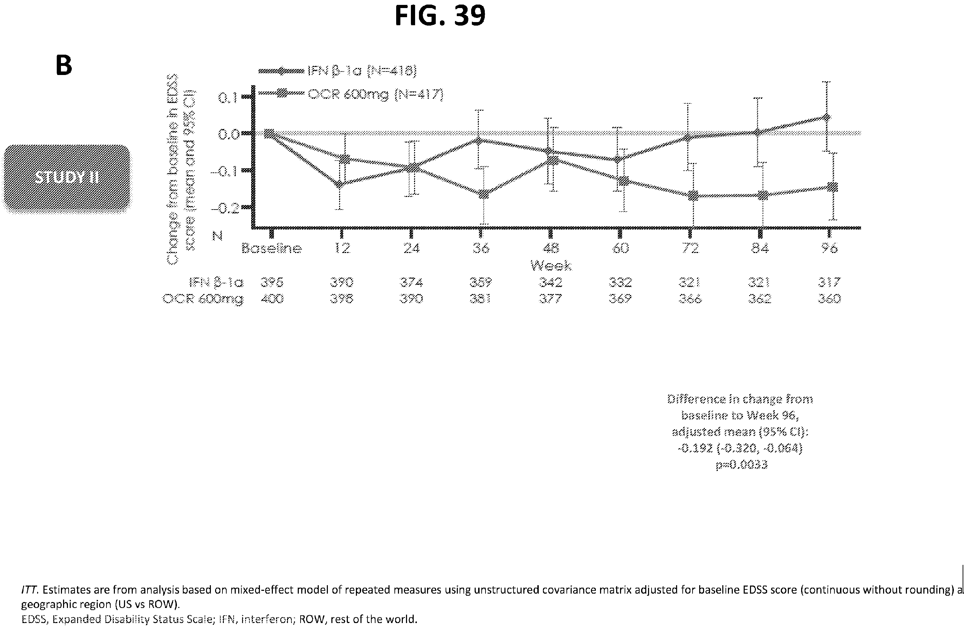

In pooled analyses, a higher proportion of ocrelizumab-treated patients had improved EDSS scores (20.2% [n=146]) as compared to patients treated with IFNβ-1a (15.0% [n=98]) (adjusted odds ratio [aOR] 1.288 [0.964, 1.72]; p=0.0866]). In pooled analyses, significantly fewer patients in the ocrelizumab group had worsened EDSS scores (10.1% [n=73] compared with IFNβ-1a (16.6% [n=109]) (aOR 0.575 [ 0.414-0.797], p=0.0009). See **FIG. 39****.**

Worsened disability (measured as an increase in EDSS score of >0.5 at Week 96 compared with baseline) was reduced by 44% in ocrelizumab-treated patients vs. patients receiving IFN β-1a in STUDY I (p=0.0242) and by 44% in ocrelizumab-treated patients vs. patients receiving IFN β-1a in STUDY II (p=0.121). (p value for relative decrease in worsening is from multinomial logistic regression, adjusted for baseline EDSS score (<4.0 vs ≥4.0), and geographic region (US vs rest of world).) Worsened disability (measured as an increase in EDSS score of >0.5 at Week 96 compared with baseline) was reduced by 43% in ocrelizumab-treated patients vs. patients receiving IFN IFN β-1a (STUDY I and STUDY II, pooled data; p=0.0009).

### MRI-related endpoints

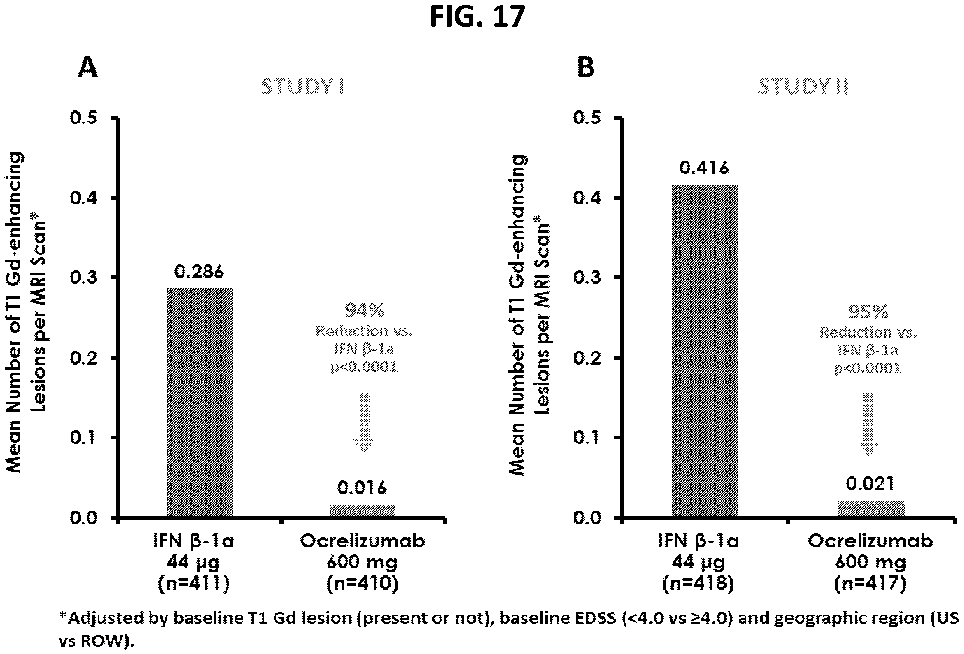

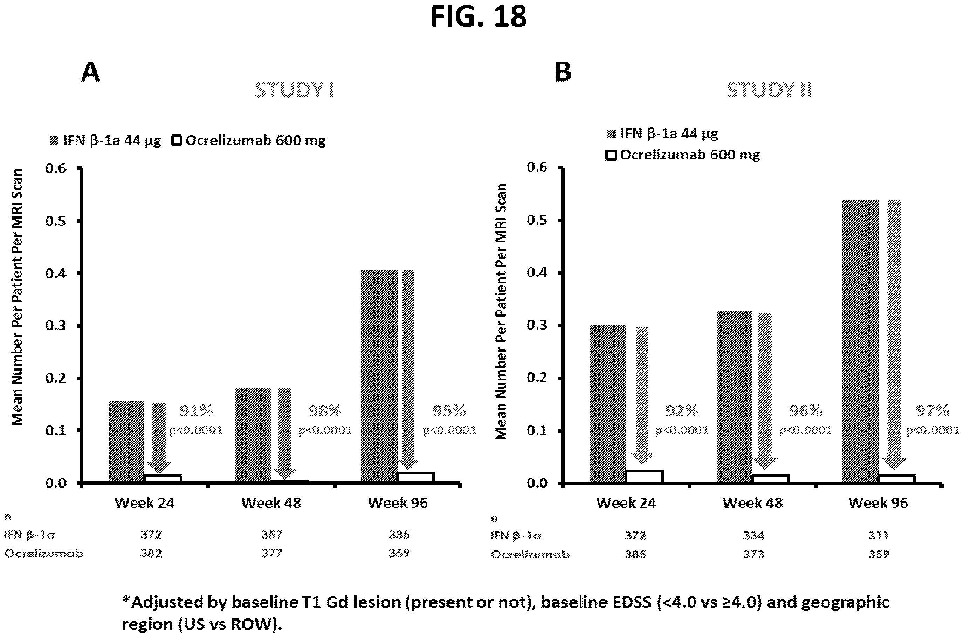

As compared with IFN β-1a, ocrelizumab reduced the total number of T1 gadolinium-enhancing lesions at Weeks 24, 48, and 96 by 94% in STUDY I and 95% in STUDY II (p<0.0001 for both studies; *see* **Table 4** and **FIGs. 17A** and **17B****).** Ocrelizumab reduced mean total number of T1 gadolinium-enhancing lesions by 91% at Week 24, by 98% at Week 48, and by 95% at week 96 compared to IFN β-1a in STUDY I (p<0.0001 for all time points, *see* **FIG. 18A****).** Ocrelizumab reduced mean total number of T1 gadolinium-enhancing lesions by 92% at Week 24, by 96% at Week 48, and by 97% at week 96 compared to IFN β-1a in STUDY II (p<0.0001 for all time points, *see* **FIG. 18B****).** The results in **FIGs. 17A-B** **and** **18A-B** were adjusted by means calculated by negative binomial regression and adjusted for baseline T1 Gd lesion (present or not) baseline EDSS (<4.0 vs. ≥ 4.0), and geographical location (US vs. rest of world). The cumulative number of T1 gadolinium-enhancing lesions was reduced by 94% in patients receiving ocrelizumab vs. patients receiving IFN β-1a (STUDY I and STUDY II, pooled data; p=0.0001).

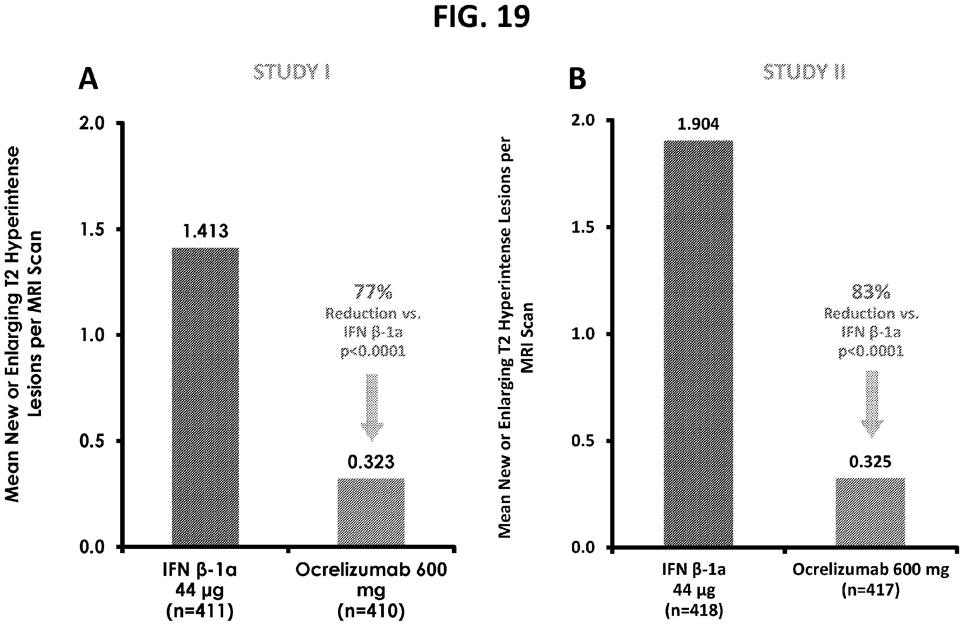

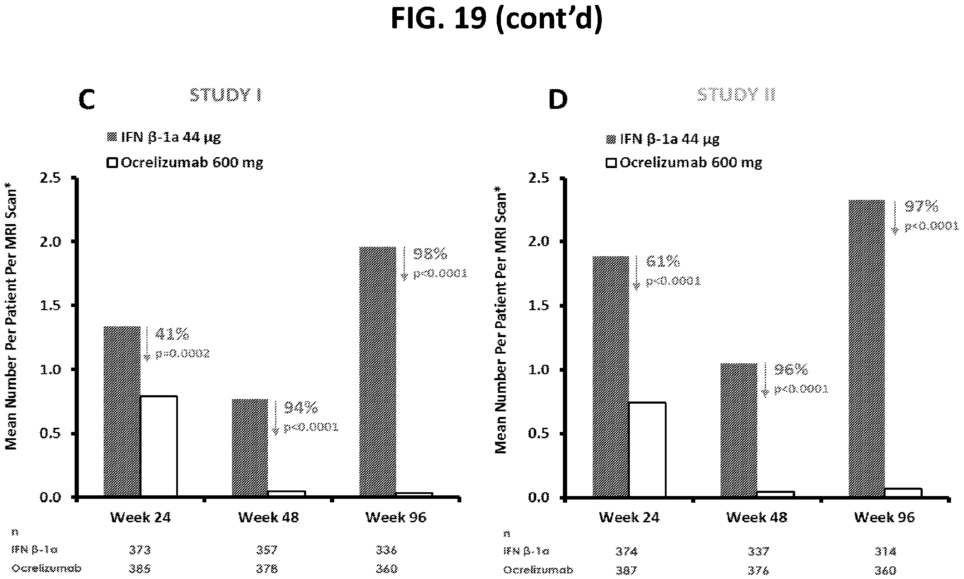

Ocrelizumab also reduced the total number of new/enlarging T2 hyperintense lesions at Weeks 24, 48, and 96 by 77% compared with IFN β-1a in STUDY I and 83% compared with IFN β-1a in STUDY II (p<0.0001 for both comparisons; *see* **Table 4** and **FIGs. 19A** and **19B****).** As shown in **FIG. 19C****,** ocrelizumab reduced the mean number of new/enlarging T2 hyperintense lesions at Week 24 by 41%; at Week 48 by 94%; and by Week 96 by 98% compared with IFN β-1a in STUDY I. As shown in **FIG. 19D****,** ocrelizumab reduced the mean number of new/enlarging T2 hyperintense lesions at Week 24 by 61%; at Week 48 by 96%; and by Week 97 by 97% compared with IFN β-1a in STUDY II. (Adjusted by means calculated by negative binomial regression and adjusted for baseline T2 lesion count, baseline EDSS (<4.0 vs ≥4.0) and geographical region (US vs ROW).) EDSS, Expanded Disability Status Scale; IFN, interferon; MRI, magnetic resonance imaging; ROW, rest of the world. Ocrelizumab reduced the emergence of more chronic or growing areas of MS-related brain injury (T2 hyperintense lesions) at 24, 48, and 96 week by about 80% compared to IFN β-1a (pooled STUDY I and STUDY II). The results in **FIGs. 19A-D** were adjusted by means calculated by negative binomial regression and adjusted for baseline T2 lesion count, baseline EDSS (<4.0 vs. ≥ 4.0), and geographical location (US vs. rest of world). The cumulative number of new and/or enlarging T2 lesions was reduced by 80% in patients receiving ocrelizumab vs. patients receiving IFN β-1a (STUDY I and STUDY II, pooled data; p=0.0001).

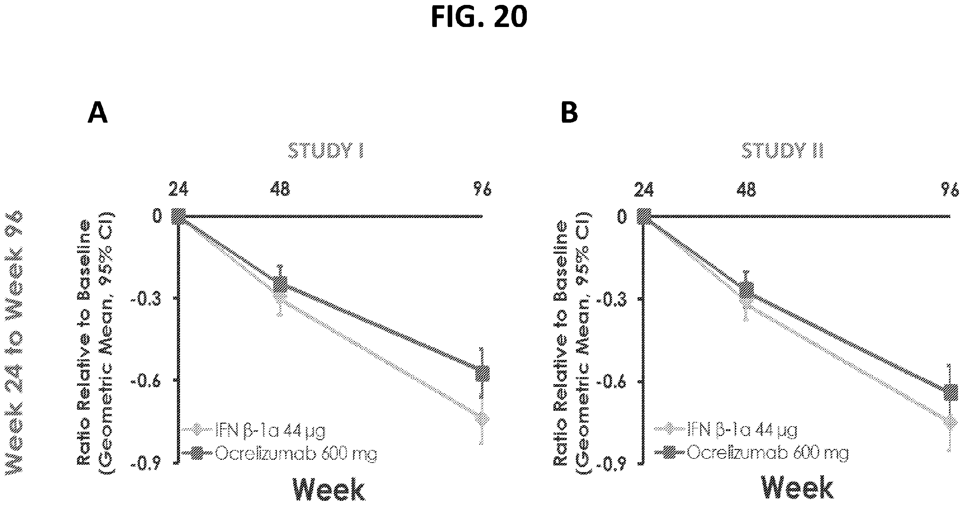

Ocrelizumab slowed brain volume loss from Week 24 to Week 96 as compared with IFN β-1a (STUDY I, p=0.0042; STUDY II, p=0.0900; **FIGs. 20A** and **20B****).** Ocrelizumab reduced the rate of brain volume loss by 18.8% as compared with IFN β-1a (STUDY I and STUDY II, pooled data; p=0.0015; difference in adjusted means (0.140; 95% CI: 0.054, 0.226). Analysis based on ITT population with week 24 and at least one post-week 24 assessment; p-value based on mixed effect model repeat measurement (MMRM) at 96 week visit adjusted by study, for week 24 brain volume, geographic region and age).

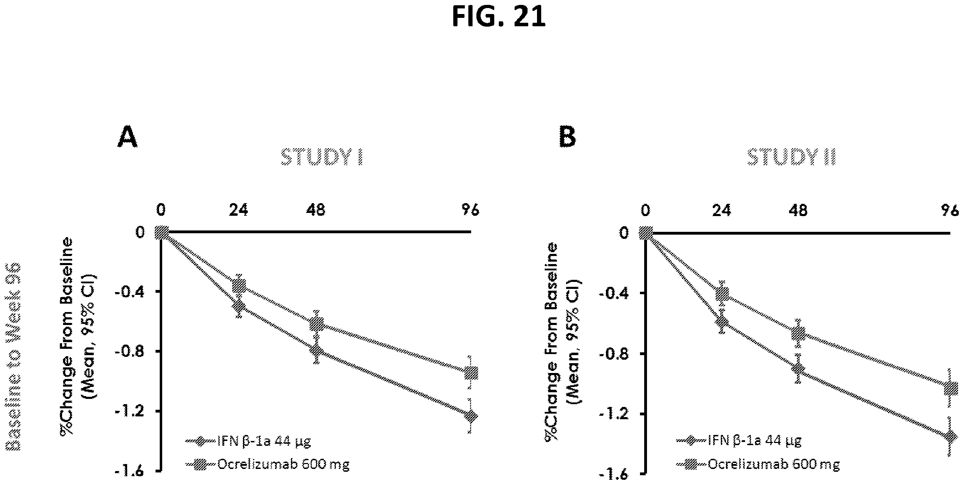

Ocrelizumab slowed also brain volume loss from baseline to Week 96 as compared with IFN β-1a (STUDY I, p=<0.0001; STUDY II, p=0.0001; **FIGs. 21A** and **21B****).** Endpoints in **FIGs. 20A-B** and **21A-B** were compared using the Cochran-Mantel-Haenszel test stratified by geographic region (US vs rest of world) and baseline EDSS score (<4.0 vs. Endpoints in **FIGs. 20A-B** and **21A-B** were compared using the Cochran-Mantel-Haenszel test stratified by geographic region (US vs rest of world) and baseline EDSS score (<4.0 vs. >4.0). Ocrelizumab reduced the rate of brain volume loss from Week 24 to Week 96 by 22.8% in STUDY I (p=0.0042) and 14.9% in STUDY II (p= 0.0900) compared with IFN β-1a. *See* **FIGs. 20A** and **B****.** In STUDY I, brain volume loss (from baseline to week 96) in ocrelizumab-treated patients was reduced by was reduced by 23.5% vs. IFNβ-1a-treated patients (*P*<0.0001). In STUDY II, brain volume loss (from baseline to week 96) in ocrelizumab-treated patients was reduced by was reduced by 23.8% vs. IFNβ-1a-treated patients (*P*=0.0001). *See* **FIGs. 21A** and **B****.**

Ocrelizumab-treated patients showed an 18.8% reduction in brain atrophy as compared to IFN β-1a-treated patients.

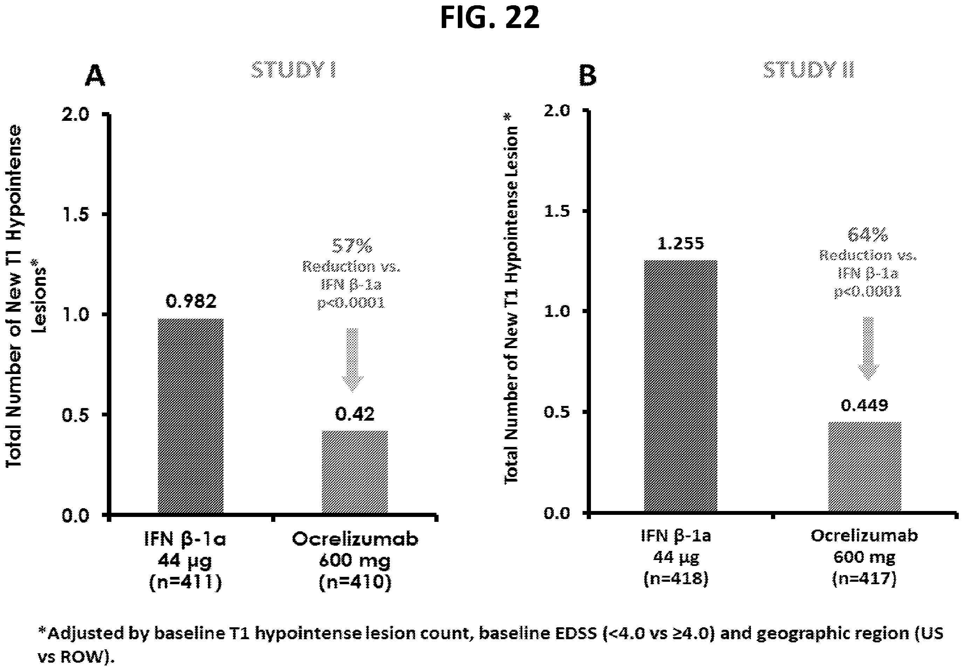

Ocrelizumab reduced the total number of new T1 hypointense lesions by 57% compared to IFN β-1a in STUDY I and by 64% compared to IFN β-1a in STUDY II. *See* **FIGs. 22A** and **22B****.** The cumulative number of new T1 hypointense lesions was reduced by 62% in patients receiving ocrelizumab vs. patients receiving IFN β-1a (STUDY I and STUDY II, pooled data; p=0.0001). Ocrelizumab reduced the number of new T1 hypointense lesions at Week 24 by 27%; at Week 48 by 95%; and by Week 96 by 99% compared with IFN β-1a in STUDY I. Ocrelizumab reduced the number of new T1 hypointense lesions at Week 24 by 33%; at Week 48 by 95%; and by Week 97 by 96% compared with IFN β-1a in STUDY II. (The total number of new T1 hypointense lesions for all patients in the treatment group at each time point (either Week 24, 48 or 96) was divided by the total number of brain MRI scans at that time point. Adjusted by means calculated by negative binomial regression and adjusted for baseline T1 hypointense lesion count, baseline EDSS (<4.0 vs ≥4.0) and geographical region (US vs rest of world).

The efficacy endpoints for STUDY I and STUDY II are summarized in **Table 5.**

**Table 5. Summary of Efficacy for STUD Y I and STUDY II**

| | **STUDY I** | **STUDY II** | **Pooled STUDIES** |
|---|---|---|---|
| **Endpoint** | Risk reduction, | Risk reduction, | Risk reduction, |
| | p value | p value | p value |
| **ARR** | 46%, | 47%, | 46%, |
| | < 0.0001 | < 0.0001 | <0.0001 |
| **Time to CDP** | 43%, | 37%, | 40%, |
| **12 week** | 0.0139 | 0.0169 | 0.0006 |
| **Total T1 Gd lesions (week 24, 48, 96)** | 94%, | 95%, | 94%, |
| | <0.0001 | <0.0001 | <0.0001 |
| **Total new / enlarging T2 lesions (week 24, 48, 96)** | 77%, | 83%, | 80%, |
| | <0.0001 | <0.0001 | <0.0001 |
| **CDI 12 week** | 61% improvement, 0.0106 | 14% improvement, 0.4019 | 33% improvement, 0.0194 |
| **Time to CDP** | 43%, | 37%, | 40%, |
| **24 weeks** | 0.0278 | 0.0370 | 0.0025 |
| **Total T1 hypointense lesions (week 24, 48, 96)** | 57%, | 64%, | 62%, |
| | <0.0001 | <0.0001 | <0.0001 |
| **Difference in mean MSFC (baseline to week 96)** | p=0.3261 | p=0.0040 | |
| **Difference in mean brain volume (week 24 to week 96)** | p=0.0042 | p=0.090 | |
| **Change in SF-36 PCS (baseline to week 96)** | p=0.2193 | p=0.0404 | |
| **NEDA by Week 96 in patients with EDSS ≥ 2.0** | 74% improvement, <0.0001 | 81% improvement, <0.0001 | 75% improvement, <0.0001 |

In STUDY I, 80.4% of ocrelizumab-treated patients were without relapses at 96 weeks, vs. 66.7% of IFNβ-1a-treated patients. In STUDY II, 78.9% of ocrelizumab-treated patients were without relapses at 96 weeks, vs. 64.5% of IFNβ-1a-treated patients.

In STUDY I, 92.4% of ocrelizumab-treated patients were without CDP at 96 weeks, vs. 87.8% of IFNβ-1a-treated patients. In STUDY II, 89.4% of ocrelizumab-treated patients were without CDP at 96 weeks, vs. 84.9% of IFNβ-1a-treated patients.

In STUDY I, 92.3% of ocrelizumab-treated patients had improved/stable disability vs. 86.1% IFNβ-1a-treated patients. In STUDY II, 87.5% of ocrelizumab-treated patients had improved/stable disability vs. 80.4% IFNβ-1a-treated patients.

In STUDY I, 7.7% OCR-treated patients had worsened disability vs 13.9% IFNβ-1a treated patients (adjusted odds ratio 0.559; p=0.0242). In STUDY II, 12.5% OCR-treated patients had worsened disability vs 19.6% IFNβ-1a treated patients (adjusted odds ratio 0.577; p=0.0121).

In STUDY I, 91.7% of ocrelizumab-treated patients were without gadolinium-enhancing T1 lesions at 96 weeks, vs. 69.8% of IFNβ-1a-treated patients. In STUDY II, 90.2% of ocrelizumab - treated patients were without gadolinium-enhancing T1 lesions at 96 weeks, vs. 63.9% of IFNβ-1a-treated patients.

In STUDY I, 61.7% of ocrelizumab-treated patients were without new/enlarging T2 lesions at 96 weeks, vs. 38.7% of IFNβ-1a-treated patients. In STUDY II, 60.9% of ocrelizumab-treated patients were without new/enlarging T2 lesions at 96 weeks, vs. 38.0% of IFNβ-1a-treated patients.

After week 24, ≥96.0% of all OCR-treated patients were without new/enlarging T2 lesions, compared with 60.8-70.9% of IFNβ1-a-treated patients.

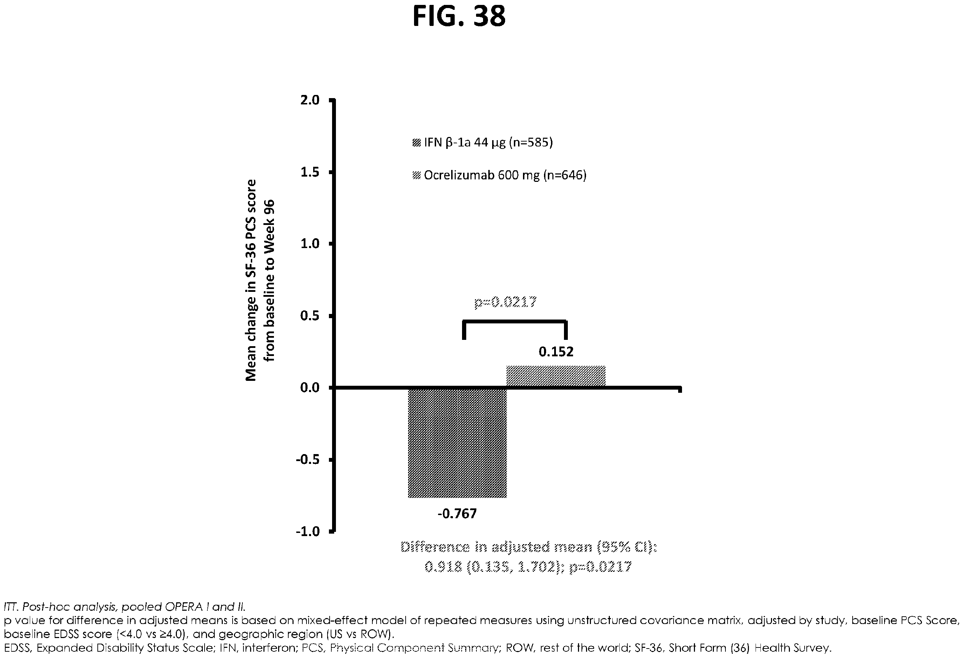

More patients receiving ocrelizumab reported improvement in change in quality of life, as measured by Short Form-36 (SF-36) Physical Component Summary (PCS) than patients receiving IFNβ-1a. See **FIG. 38****.**

### Safety

### Adverse events

The overall incidence of adverse events was 83.3% and 83.3% for patients treated with IFN β-1a and ocrelizumab, respectively, in STUDY I and STUDY II (pooled). (*See* **Table 6A).**

**Table 6A. Adverse events (safety population).**

| **n (%)** | | **POOLED STUDY I AND STUDY II** | |
|---|---|---|---|
| | | **IFN β-1a 44 µg (n=826)** | **Ocrelizumab 600 mg (n=825)** |
| **Total number of patients with ≥ 1 adverse event (AE)** | | **688 (83.3)** | **687 (83.3)** |
| **Total number of patients with ≥ 1 AE occurring at relative frequency > 5%** | | **539 (65.3)** | **544 (65.9)** |
| **Injury, Poisoning, and Procedural Complications** | | **155 (18.8)** | **333 (40.4)** |
| | Infusion-related reaction | 80(9.7) | 283 (34.3) |
| **General Disorders and Administration-Site Conditions** | | **396 (47.9)** | **173 (21.0)** |
| | Influenza-like illness | 177 (21.4) | 38 (4.6) |
| | Injection-site erythema | 127 (15.4) | 1 (0.1) |
| | Fatigue | 64 (7.7) | 64 (7.8) |
| | Injection-site reaction | 45 (5.4) | 2 (0.2) |
| **Infections and Infestations** | | **433 (52.4)** | **482 (58.4)** |
| | Upper respiratory tract infection | 87 (10.5) | 125 (15.2) |
| | Nasopharyngitis | 84 (10.2) | 122 (14.8) |
| | Urinary tract infection | 100 (12.1) | 96 (11.6) |
| | Sinusitis | 45 (5.4) | 46 (5.6) |
| | Bronchitis | 29 (3.5) | 42 (5.1) |
| **Nervous system disorders** | | **252 (30.5)** | **224 (27.2)** |
| | Headache | 124 (15.0) | 93 (11.3) |
| **Psychiatric disorders** | | **144 (17.4)** | **149 (18.1)** |
| | Depression | 54. (6.5) | 64 (7.8) |
| | Insomnia | 38 (4.6) | 46 (5.6) |
| **Musculoskeletal and connective tissue disorders** | | **207 (25.1)** | **204 (24.7)** |
| | Back pain | 37 (4.5) | 53 (6.4) |
| | Arthralgia | 51 (6.2) | 46 (5.6) |

| | | | |
|---|---|---|---|
| Table 6A includes only pooled AEs occurring in ≥5% of patients in at least one treatment group and the corresponding system organ class. | | | |

**Table 6B. Total Serious Adverse Events**

| **n (%)** | **IFN β-1a 44µg (n = 826)** | **Ocrelizumab 600 mg (n = 825)** |
|---|---|---|
| **Overall patients with ≥ SAE** | 72 (8.7) | 57 (6.9) |
| **Infections and infestations** | 24 (2.9) | 11 (1.3) |
| **Nervous system disorders** | 11 (1.3) | 8 (1.0) |
| **Injury, poisoning, and procedural complications** | 10 (1.2) | 6 (0.7) |

Serious adverse events were reported in 8.7% of IFN β-1a-treated patients and 6.9% of ocrelizumab-treated patients in STUDY I and STUDY II (pooled). *(See* **Table 6B).**

### Infections

The incidence of infection was 52.8% with IFN β-1a and 56.6% with ocrelizumab in STUDY I and 52.0% with IFN β-1a and 60.2% with ocrelizumab in STUDY II. The most common infections (reported in at least 10% of patients in at least one treatment arm) were urinary tract infections, upper respiratory tract infections, and nasopharyngitis. The incidence of serious infection in both studies was low (1.2 to 2.9% across all treatment arms in both studies). No serious opportunistic infections were reported in any group during the 96-week study. Overall, herpes-virus infections were reported in 28 patients treated with IFN β-1a and 50 patients treated with ocrelizumab. All cases were mild or moderate with the exception of one case of herpes simplex in a patient treated with ocrelizumab in STUDY I, graded as severe.

### Infusion-related reactions

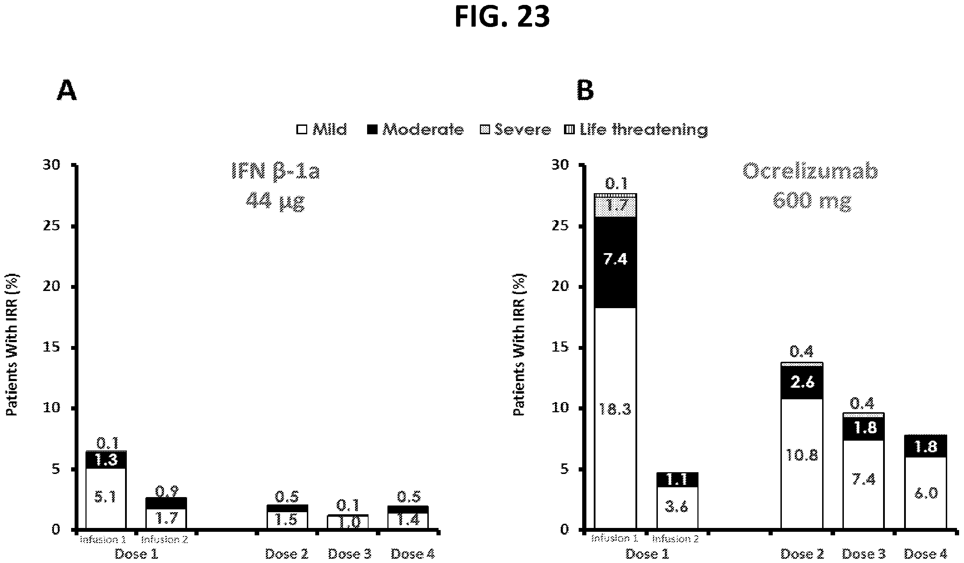

The number of patients with at least one infusion-related reaction (IRR) was higher in patients treated with ocrelizumab versus IFN β-1a (30.9% for ocrelizumab versus 7.3% for IFN β-1a in STUDY I; 37.6% for ocrelizumab versus 12.0% for IFN β-1a in STUDY II; 34.3% for ocrelizumab versus 9.7% for IFN β-1 in both STUDY I and STUDY II (pooled)). One OCR-treated patient had a serious IRR at first infusion, with life-threatening bronchospasm; despite event resolution, subsequent treatment was withdrawn per protocol. Withdrawal from treatment due to IRRs during the first infusion occurred in 11 patients (1.3%) in the ocrelizumab group only. IRR incidence with ocrelizumab-treated patients was highest with the first infusion (27.5%) and markedly decreased with subsequent dosing (13.7% at Dose 2). The majority of infusion-related reactions were Grade 1 or 2 and were reported at the first infusion of dose one (*see* **FIGs. 23A** and **23B****,** pooled data for STUDY I and STUDY II). Numbers in columns represent the proportion of patients experiencing a grade of IRR. Grading per Common Terminology Criteria (CTCAE): **Grade 1** Mild; asymptomatic or mild symptoms; **Grade 2** Moderate; minimal, local or noninvasive intervention indicated; **Grade 3** Severe or medically significant but not immediately life-threatening; **Grade 4** Life-threatening consequences; urgent intervention indicated; **Grade 5** Death related to AE. Note: All received 100-mg i.v. methylprednisolone. Over 96 weeks, 9.7% of IFNβ-1a patients (n=80/826) and 34.3% of ocrelizumab-treated patients (n=283/825) had at least 1 IRR; most were mild to moderate in severity (99% [n=79] and 93% [n=262], respectively). Most IRR symptoms with ocrelizumab-treated included pruritus, rash, throat irritation and flushing. IRRs mostly occurred during infusion (IFNβ-1a, 46.3%; ocrelizumab, 80.6%) and were managed with infusion adjustments and symptomatic treatment (42.5% of patients that had IRRs in IFNβ-1a group and 65.4% in the ocrelizumab group received treatment).

### Malignancies

In total, two malignancies were reported with IFN β-1a (one mantle cell carcinoma and one squamous cell carcinoma), and four malignancies were reported in patients treated with ocrelizumab (two cases of invasive breast ductal breast carcinoma, one renal cell carcinoma, and one malignant melanoma).

### Laboratory assessments

In laboratory assessments, by Week 2, mean CD19 levels had decreased to negligible in patients treated with ocrelizumab. There was no impact on CD3-, CD4, CD8-, CD16-, and CD56-positive cells, supporting that innate immunity and total T-cell numbers are not affected by ocrelizumab. There was no impact of ocrelizumab on existing antibody titers for mumps, rubella, varicella, and pneumococcus.

While only a direct comparison of ocrelizumab to IFN β-1a was measured in STUDY I and STUDY II, these efficacy and safety results suggest the benefit/risk profile of ocrelizumab over a 2 year period is superior to all disease-modifying therapies (DMTs) available for the treatment of RMS patients.

### Effects of ocrelizumab on humoral immunity markers

Prior to study enrollment, physicians were advised to review patient immunization status and follow local guidance for vaccination; immunizations were to be completed ≥6 weeks prior to treatment. Measurements of antibody (Ab) titers against mumps, rubella, varicella, and *Streptococcus pneumoniae* were taken at baseline and at Weeks 12, 24, 48, 72, and 96. The proportion of patients with Ab levels that could be considered protective was assessed for each treatment group over time.

**Mumps:** 94.1% of IFNβ-1a-treated patients and 93.6% of ocrelizumab-treated patients had positive levels of mumps Ab at baseline. (This proportion ranged (min-max) 92.7-94.8% (IFNβ-1a) and 91.8-93.5% (ocrelizumab) over the six measurements taken during the 96-week study treatment period.)

**Rubella:** 87.9% of IFNβ-1a- treated patients and 89.0% of ocrelizumab-treated patients had positive levels of rubella Ab, and ranged (min-max) 89.8-90.8% (IFNβ-1a) and 88.7-89.4% (ocrelizumab) over the six measurements taken during the treatment period.

**Varicella:** 95.5% of both treatment groups had positive levels of varicella Ab at baseline, and ranged (min-max) 96.2-97.5% (IFNβ-1a) and 94.8-95.6% (ocrelizumab) over the six measurements taken during the treatment period.

***S. pneumonia:*** Among the evaluable patients, the mean (standard deviation) level of *S*. *pneumonia* Ab was 53.67 (54.13) mg/mL for IFNβ-1a-treated patients and 55.35 (67.00) mg/mL for ocrelizumab-treated patients at baseline. At week 96, the mean (standard deviation) level of *S. pneumonia* Ab was 51.74 (42.50) mg/mL for IFNβ-1a-treated patients and 54.06 (80.98) mg/mL for ocrelizumab-treated patients. At 96 weeks the mean change from baseline was -1.13 (40.25) mg/mL for IFNβ-1a-treated patients and -1.99 (59.60) mg/mL for ocrelizumab-treated patients.

### Effects of ocrelizumab on immune responses

Infections can lead to MS disease exacerbation and may cause treatment complications with currently employed therapies. Accordingly, vaccinations against infections are a part of the management of patients with MS. This study evaluates whether ocerlizumab-treated patients can mount protective immune responses against clinically relevant vaccines. This study uses the following vaccines to evaluate different immune response pathways:
a) Tetanus toxoid (TT)-containing vaccination to assess the T-cell dependent anamnestic humoral response;
b) 23-valent pneumococcal polysaccharide vaccine (23-PPV) to assess a mostly T-cell independent or pure B-cell humoral response;
c) Keyhole limpet haemocyanin (KLH) to explore the B cell dependent immune response to neo-antigen;
d) Booster 13-valent conjugate pneumococcal vaccine (13-PCV) to assess the clinical efficacy of the 23-PPV vaccine followed by the booster 13-PCV compared to 23-PPV vaccine alone;
e) Influenza vaccine to test the ability to mount a humoral response to a clinically relevant vaccine.

In a Phase III, open-label Study to evaluate the effect of ocrelizumab on immune responses in patients with relapsing Multiple Sclerosis, approximately 100 patients are randomized (2: 1) to receive ocrelizumab 600mg as two 300mg intravenous infusions on Day 1 and Day 15. **Group A** patients receive ocrelizumab prior to immunization. **Group B** patients remain treatment-naive/continue with interferon treatment during immunization.

**Group A** patients are immunized ≥ 85 days/ 12 weeks after the first ocrelizumab administration. Briefly, **Group A** are immunized with tetanus toxoid (TT)-containing adsorbed vaccine at Week 12, with 23-valent pneumococcal polysaccharide vaccine (23-PPV) at Week 16, and with keyhole limpet hemocyanin (KLH) at Weeks 12, 16, and 20. **Group A** patients are further subdivided to either receive:
- booster 13-pneumococcal conjugate vaccine (13-PCV; **Group A1);** or
- influenza vaccine **(Group A2).**

**Group B** patients are immunized with TT on Day 1, with 23-PPV on Day 28, with (KLH) on Days 1, 28, and 56, and with influenza vaccine during Weeks 1-12.

The key inclusion criteria for this study are: RMS diagnosis (2010 revised McDonald criteria, Polman et al. Ann Neurol 2011;69:292-302); age between 18-55 years; receipt of ≥1 previous immunization against TT or tetanus and diphtheria (DT/Td), or tetanus, diphtheria, and acellular pertussis (DTaP/Tdap); EDSS score of 0.0-5.5. The key exclusion criteria are: known hypersensitivity to any component of the TT-containing adsorbed vaccine, pneumococcal polysaccharide/conjugate vaccine, or influenza vaccine; receipt of any PPV <5 years prior to screening or a live vaccine <6 weeks prior to randomization; previous exposure to KLH or immunization with any tetanus-containing vaccine <2 years prior to screening.

All groups undergo post-immunization assessments until the end of the study period **(Group A:** Day 169/Week 24; **Group B:** Day 84/Week 12). Key eligibility criteria include at least 1 previous immunization against TT, tetanus or diphtheria; or tetanus, diphtheria and acellular pertussis. The primary outcome measure compares the proportion of patients in Groups A1 and A2 with a positive TT response (IgG) 8 weeks post-immunization with patients in Group B. A positive response to the booster immunisation is defined as an antibody titre ≥ 0.2 IU/mL (pre-immunisation titres <0.1 IU/mL) or a 4-fold increase in antibody titre (pre-immunisation titres ≥ 0.1 IU/mL).

Key secondary outcome measures are listed below:
*TT response*
   - The proportion of patients treated with ocrelizumab (Groups A1 and A2) vs. the proportion of patients not treated with ocrelizumab (Group B) with a positive response (IgG) to TT vaccine at 4 weeks post-immunization;
   - The proportion of patients treated with ocrelizumab (Groups A1 and A2) vs. the proportion of patients not treated with ocrelizumab (Group B) with a 2-fold increase in tetanus antibody titres (pre-immunisation titres ≥0.1 IU/mL), or with tetanus antibody titres ≥0.2 IU/mL (pre-immunisation titres <0.1 IU/mL) at 4 weeks post-immunisation;
   - Mean levels of anti-tetanus antibody in patients treated with OCR (Groups A1 and A2) and not treated with OCR (Group B) measured immediately prior to and 4 weeks after a booster vaccine
*23 PPV*
   - The proportion of patients treated with ocrelizumab (Groups A1 and A2) vs. the proportion of patients not treated with ocrelizumab (Group B) with a positive response against individual anti-pneumococcal antibody serotype at 4 weeks post-23-PPV. A positive response is defined as developing a 2-fold increase in level or a >1µg/mL rise in level compared with pre-immunisation levels.
*KLH*
   - Mean levels of anti-KLH antibody (IgG) in patients treated with ocrelizumab (Groups A1 and A2) vs. the proportion of patients not treated with ocrelizumab (Group B) measured immediately prior to the first administration and 4 weeks after the last administration of KLH.
*Pneumococcal conjugate booster response in Groups A1 and B*
   - The proportion of patients treated with ocrelizumab (Group A1) in Group A1 vs. the proportion of patients not treated with ocrelizumab (Group B) with positive responses against an individual anti-pneumococcal antibody serotype (23 serotypes) measured 4 weeks after the booster 13-PCV vaccine. A positive response is defined as developing a 2-fold increase in level or a >1µg/mL rise in level compared with pre-immunisation levels.
*Influenza vaccine response*
   - The proportion of patients treated with ocrelizumab (Group A2) who achieve seroprotection (specific haemagglutination inhibition titres >1:40) at 4 weeks post-immunisation compared with patients not treated with ocrelizumab (Group B).
*Immunophenotyping outcome measures*
   - Measures of humoral and cellular immunity including: (a) total B cells (CD19⁺); (b) B-cell subsets (memory; naive; plasma); (c) total T cells (CD3⁺); (d) T helper cells (CD3⁺CD4⁺); (e) cytotoxic T lymphocytes (CD3⁺CD8⁺); and (1) natural killer cells (CD3⁻CD16/56⁺).

### Open-Label Extension Phase

Following completion of the double-blind, controlled treatment phases STUDY I and STUDY II, patients may be eligible to enter open-label extension (OLE) phases to evaluate long-term safety, tolerability, and efficacy of ocrelizumab. The design of the open-label STUDY I and STUDY II extension studies, which evaluates the long-term safety and efficacy of OCR in relapsing MS, is described below.

Patients entering the OLE phase enter the OLE screening phase, lasting up to 4 weeks. During OLE screening, patients receive IFN β-1A or IFN β-1A placebo until first infusion of dose 5. The OLE phase lasts until ocrelizumab is commercially available in the patient's country, as per local regulations or until the sponsor decides to terminate the ocrelizumab MS program. The OLE phase does not exceed 4 years (208 weeks). OLE is not mandatory. Patients unwilling to proceed to the OLE phase are entered into the safety follow-up phase, which is performed in 12-week intervals starting from the date of the patient's latest visit. Continued monitoring occurs if B cells are not repleted.

Key inclusion criteria for the OLE phase include: completion of the blinded treatment period; consent from investigators who determine that the patient may benefit from OCR treatment based on the assessments performed during the treatment period; written informed consent from the patient to enter the OLE phase; and meeting ocrelizumab treatment/retreatment criteria, in which the patient is free from the following conditions and laboratory abnormalities: (a) life-threatening (CTCAE Grade 4) infusion-related event that occurred during a previous OCR infusion; (b) any significant or uncontrolled medical condition or treatment-emergent, clinically significant laboratory abnormality; (c) active infection; (d) absolute neutrophil count <1.5 × 10³/µL; (e) CD4 cell count <250/µL; and (f) hypogammaglobulinemia immunoglobulin G <3.3 g/L.

The schedule of key safety and efficacy assessments are shown in **Table 6C** below.

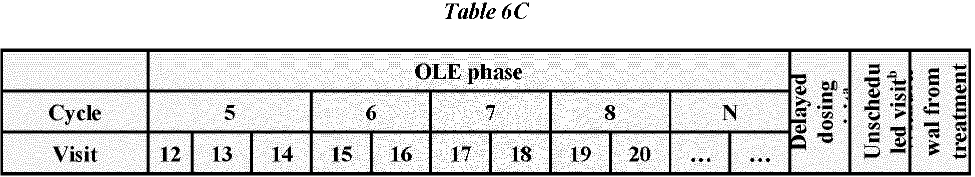

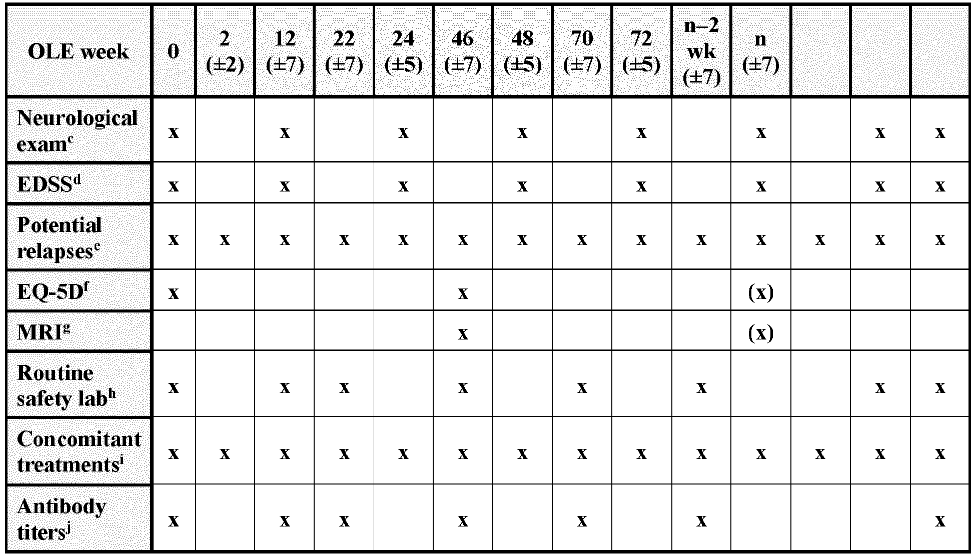

### Summary

STUDY I and STUDY II showed that in patients with relapsing forms of MS, ocrelizumab significantly reduced the annualized relapse rate and the risk of 12- and 24-week confirmed disability progression compared with IFN β-1a. Ocrelizumab is the first treatment for multiple sclerosis to significantly reduce both 12- and 24-week confirmed disability progression in two separate Phase III studies and against an active comparator, demonstrating a consistency of effect. The proportion of patients achieving confirmed disability improvement for at least 12 weeks was also significantly increased in patients receiving ocrelizumab compared with IFN β-1a. These clinical findings are supported by the MRI endpoints whereby ocrelizumab significantly reduced the number of T1 gadolinium-enhancing and new/enlarging T2 lesions in the brain compared with IFN β-1a. The effect on T1 gadolinium-enhancing and new/enlarging T2 lesions is of such magnitude that it suggests focal inflammation and disease activity is largely stopped/suspended.

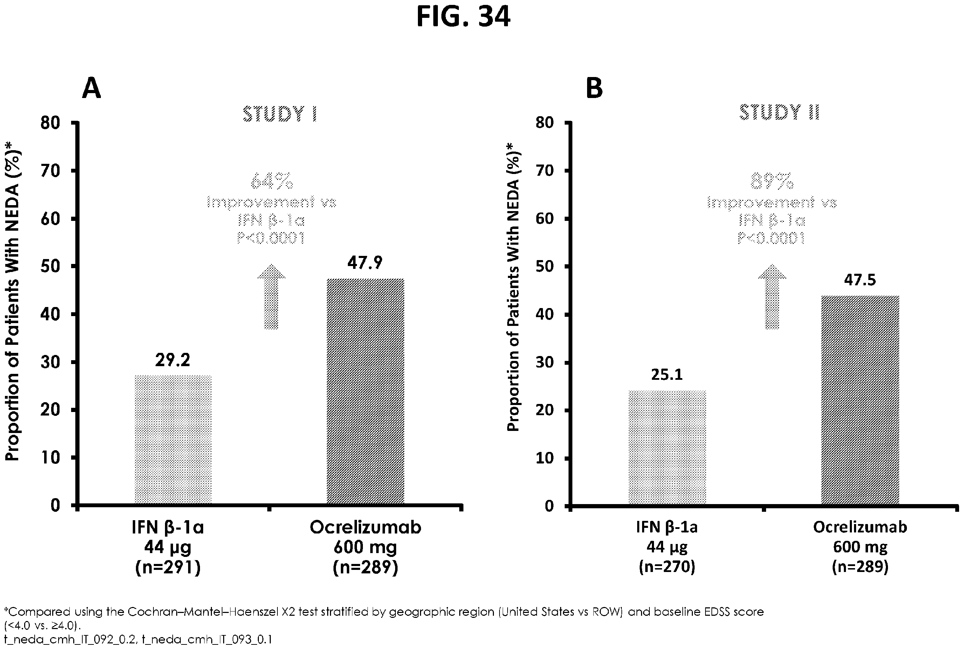

In exploratory analyses, ocrelizumab reduced brain volume loss (see **FIGs. 21A-D)** as compared to IFN β-1a. In further exploratory analyses, ocrelizumab increased the proportion of patients with no evidence of disease activity (NEDA) in the ITT population with an EDSS ≥ 2.0 (see **Tables 4** and **5)** as compared to IFN β-1a. Ocrelizumab increased the proportion of patients with no evidence of disease activity (NEDA) in all patients in the ITT population as compared to IFN β-1a (see **FIGs. 34A** and **34B****).** In **FIGs. 34A** and **34B****,** the proportion of patients with NEDA was compared using the Cochran-Mantel-Haenszel X2 test stratified by geographic region (United States vs rest of world) and baseline EDSS score (<4.0 vs. ≥4.0).

These efficacy results, which demonstrate reduced worsening of disability and in some patients increased functioning, support the hypothesis that ocrelizumab has effects on markers of inflammation and neuronal damage. The efficacy demonstrated by ocrelizumab on both inflammatory as well as degenerative outcomes suggests that treatment with ocrelizumab has potent anti-inflammatory effects and may also have neuroprotective effects.

Benefits with ocrelizumab in STUDY I and STUDY II were observed early and were sustained over the 96-week treatment period; based on earlier experience, the risk of rebound is not expected upon treatment discontinuation (Kappos et al. (2012) "Long-term safety and efficacy of ocrelizumab in patients with relapsing-remitting multiple sclerosis: Week 144 results of a phase II, randomized, multi centre trial." 28th Congress of the European Committee for Treatment and Research in Multiple Sclerosis*).*

Adverse events observed in patients receiving ocrelizumab were consistent with those reported in the Phase II study of ocrelizumab (Kappos et al. (2011) Lancet. 378, 1779-87). There were no cases of serious opportunistic infections reported during the 96-week studies. The results to date suggest ocrelizumab has no apparent impact on immune surveillance. Overall, ocrelizumab had a similar safety profile compared with IFN β-1a over 96 weeks in both STUDY I and STUDY II.

As a class of drugs, CD20⁺ targeted treatments have a low risk of progressive multifocal leukoencephalopathy (<1 in 25,000) (Clifford et al. (2011) Arch Neurol. 68, 1156-64). No cases of progressive multifocal leukoencephalopathy have been reported in the STUDY studies to date.

As expected, infusion-related reactions were more commonly seen with ocrelizumab. Consistent with the Phase II trial with the 600 mg dose of ocrelizumab, these reactions were largely observed with first dose and decreased with subsequent doses (Hauser et al. (2008) N Engl JMed. 358, 676-88). This supports the rationale for splitting the first 600 mg dose into two separate infusions of 300 mg separated by approximately two weeks. Infusion-related reactions were manageable with pre-medication, infusion adjustments and symptomatic treatment.

The clinical and MRI data in aggregate suggest that ocrelizumab is at least as effective as any other tested treatment for multiple sclerosis, including those which are considered to be high-efficacy therapies. While only a direct comparison to IFN β-1a was measured in STUDY I and STUDY II, these efficacy and safety results suggest the benefit / risk profile of ocrelizumab over a 2 year period is superior to all disease modifying therapies available for the treatment of RMS patients. The high efficacy is not associated with an increase in safety risk compared with IFN β-1a. Other benefits include the early start of treatment benefit and no evidence of rebound upon discontinuation of ocrelizumab.

Data obtained from STUDY I and STUDY II show that targeting CD20⁺ B cells may be effective in treatment of relapsing MS.

### Subgroup Analysis

To evaluate the relative benefit/risk with varying degrees of disease activity and prior response to treatment before study entry, four additional subgroups were identified:

**Active treatment naïve;** defined with no prior treatment experiencing at least 2 relapses in the previous 2 year, and at least 1 relapse in the last year prior to randomization.

**Active inadequate responders;** defined as patients previously treated with IFN β-1a or glatiramer acetate for at least 1 year, and either (a) experienced at least one relapse in the previous year or (b) experienced at least 1 baseline gadolinium-enhancing lesion

**Highly active treatment naive patients;** defined as patients naive to treatment who have experienced at least 2 relapses in the last year prior to randomization, and either (a) at least 1 baseline gadolinium lesion or (b) increase in T2 lesion count at baseline visit (changing categorically from 0-5 to 6-9 lesions or from 6-9 lesions to > 9 lesions), as compared to a prior MRI.

**Highly active inadequate responders;** defined as patients previously treated with interferon or glatiramer acetate for at least 1 year, had at least one relapse in the previous year, and either (a) have at least nine T2-lesions or (b) have at least one Gd lesion at baseline.

STUDY I and STUDY II were pooled to increase the power to detect differences across the 4 subgroups. There were at least 100 patients in each subgroup to enable adequate detection of treatment differences.

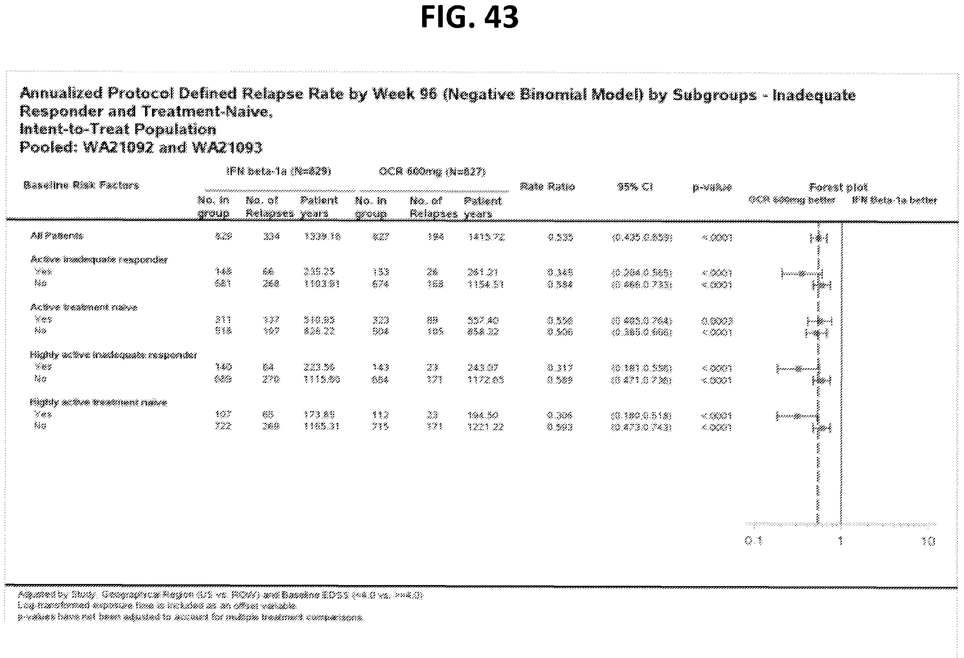

Annualized relapse rate per arm/group was interrogated to determine whether trends in inadequate responders were driven by ocrelizumab IFN β-1a absolute outcomes. Overall, ocrelizumab showed a significant effect in reducing the annualized relapse rate in all subgroups compared with IFN β-1a *(see* **FIG. 43****).** Inadequate responders in the interferon arm had a higher ARR than the ITT group, whereas ocrelizumab patients in the same group had a lower ARR than the ITT group. The active treatment naive group had a higher ARR in both interferon and ocrelizumab groups.

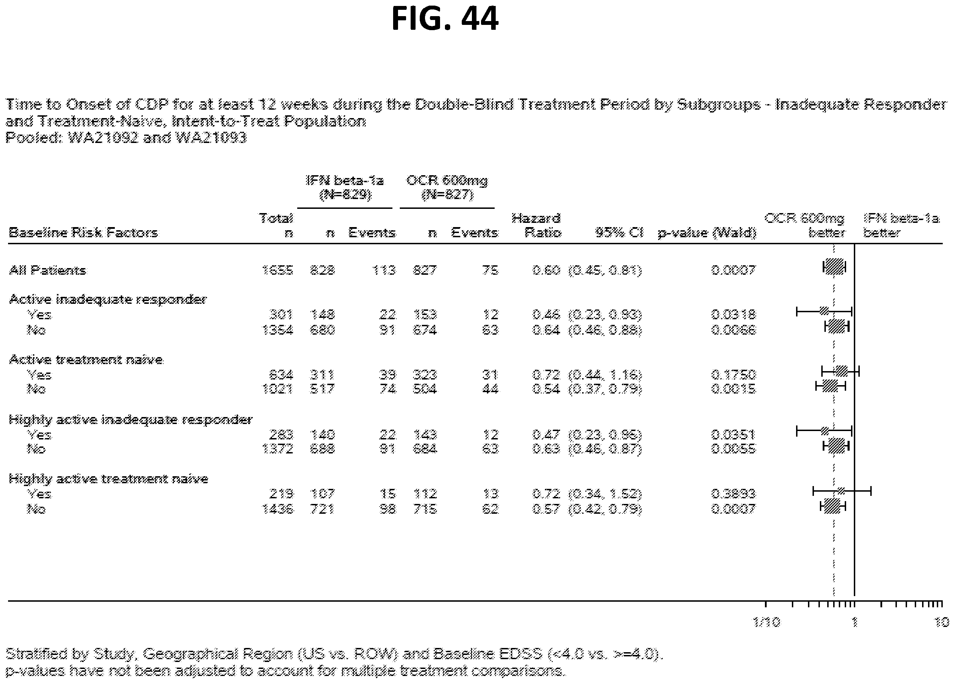

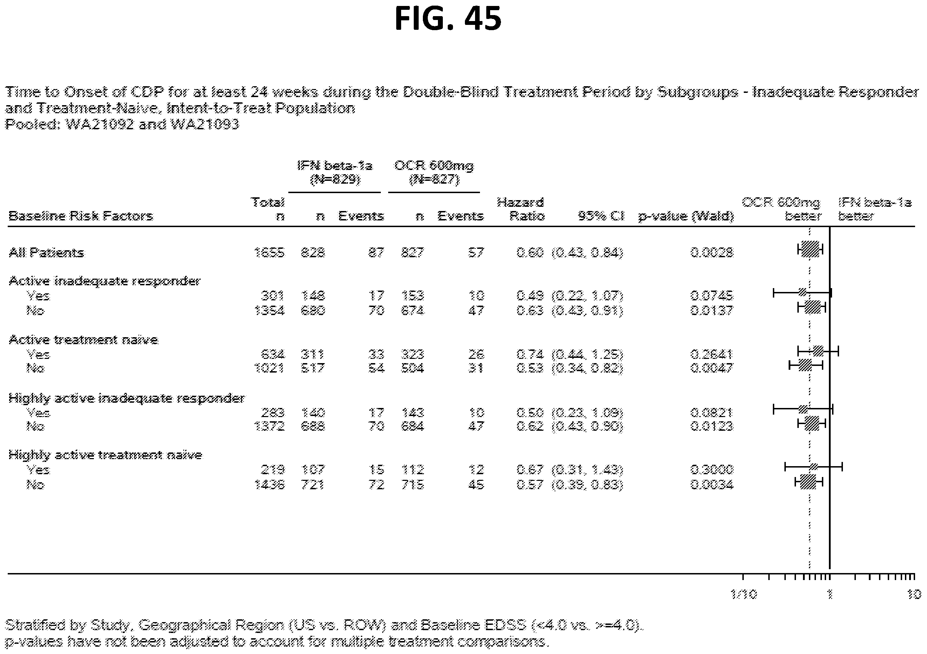

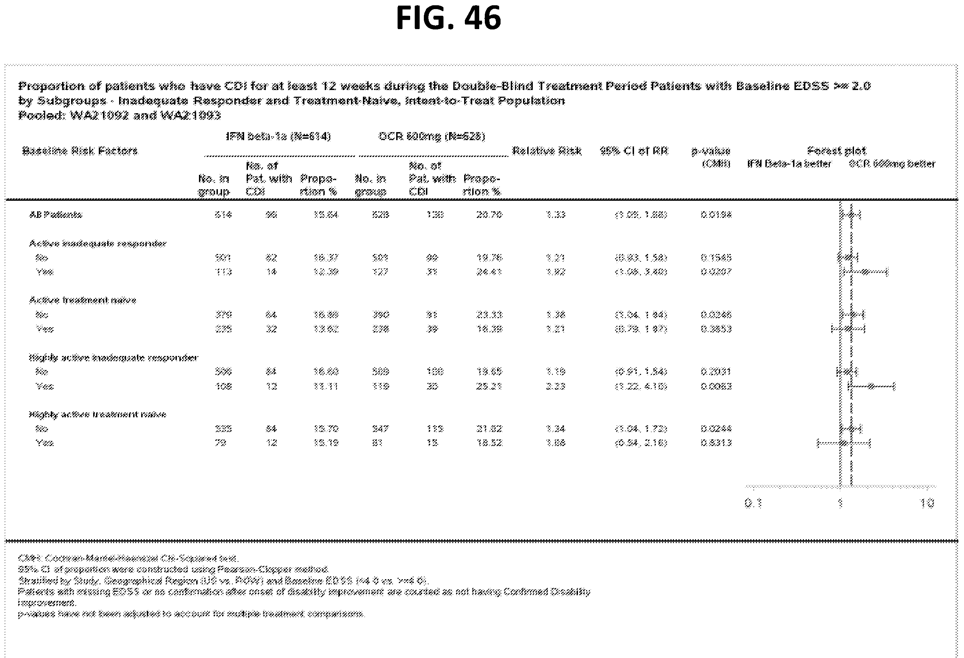

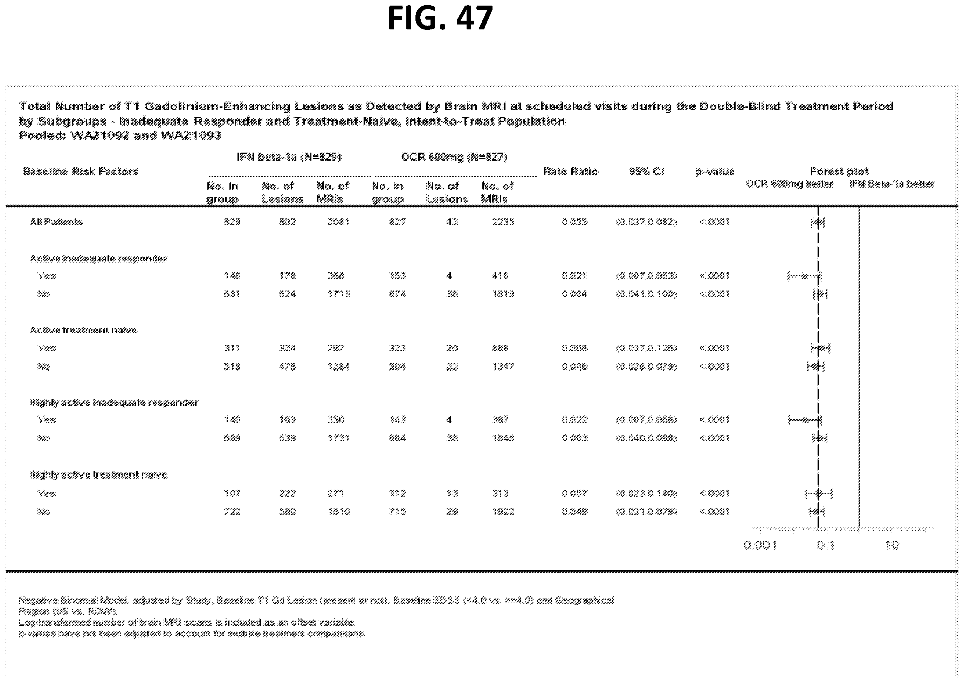

Across all 4 subgroups, ocrelizumab had a greater treatment effect compared with IFN β-1a for ARR, CDP for at least 12 weeks, CDP for at least 24 weeks, CDI, and MRI (see **FIGs. 43-47** above). For ARR and MRI the difference was statistically significant across all 4 subgroups. For CDP for at least 12 weeks, there were statistically significant effects in both active and highly active inadequate responders demonstrating ocrelizumab has consistent treatment effects compared with IFN β-1a. For CDP for at least 24 weeks, these groups demonstrated trends towards significance (p=0.075 and p = 0.082 respectively). When comparing on objective measures like T1 Gd-enhancing lesions (indicative of acute inflammatory effects, consistent with the mechanism of action for OCR), the robust and consistent effects of ocrelizumab compared with IFN β-1a is evident across all subgroups

Similarly, analysis of safety outcomes for the 4 subgroups was undertaken, and compared to the overall safety population. Due to the comparatively small size of the groups, comparison between the groups has been limited in order to not draw inappropriate conclusions based on small numbers of events. Safety outcomes in the 4 subgroups were consistent with each other, and similar with the overall population ("safety population", defined as any patient with any exposure to OCR), with in general a low proportion of patients who experienced any AE, SAEs and AEs leading to withdrawal. Consistent with observations in the safety population, there were more infections in the ocrelizumab group compared with the interferon group, whereas the proportion of patients experiencing serious infections in the ocrelizumab arm was similar to or lower than interferon in all subgroups.

Overall ocrelizumab efficacy and safety outcomes were comparable to the ITT/safety population in every subgroup. Ocrelizumab has thus demonstrated a favorable benefit/risk profile in all subgroups. The consistency of data across subgroups further supports the observed effects in the total population.

### Example 2: A Phase III Study of Ocrelizumab in Patients with Primary Progressive Multiple Sclerosis

A randomized, parallel group, double-blind, placebo controlled study was performed to evaluate the efficacy and safety of ocrelizumab compared with placebo in patients with primary progressive multiple sclerosis.

Clinical trials in progressive forms of MS are typically > 2 years in duration due to the heterogeneity in disease progression rates, which have at times been historically lower than anticipated resulting in inability to show a treatment effect. This study had an event-driven design, meaning that if the projected number of confirmed disability progression events were not reached by week 120 due to slow disease progression, the treatment period was extended until sufficient progression events have occurred to maintain statistical power to detect a treatment difference. As a result, the blinded treatment period was a minimum of 120 weeks from the last patient enrolled; the average treatment period was 3.5 years. Most patients continue to be observed for a 3- to 4-year period in the blinded treatment phase.

### Methods

### Eligibility and Exclusion Criteria

Key eligibility criteria included: an age of 18 to 55 years; a diagnosis of Primary Progressive Multiple Sclerosis in accordance with the 2005 revised McDonald criteria (Polman et al. (2011) "Diagnostic criteria for multiple sclerosis: 2005 revisions to the 'McDonald criteria.'" Ann Neurol 58, 840-846); an Expanded Disability Status Scale (EDSS) score of 3 to 6.5 points at screening; a score of at least 2.0 on the pyramidal functions component of the Functional Systems Scale (FSS) documented history or presence at screening of elevated IgG index in a cerebrospinal fluid (CSF) specimen and/or one or more IgG oligoclonal bands detected by isoelectric focusing in a cerebrospinal fluid (CSF) specimen; no history of relapse-remitting multiple sclerosis (RRMS), secondary progressive multiple sclerosis (SPMS) or progressive relapsing multiple sclerosis (PRMS); disease duration from the onset of MS symptoms of (a) less than 15 years in patients with an EDSS at screening > 5.0 or (b) less than 10 years in patients with an EDSS at screening ≤ 5.0; and no treatment with other multiple sclerosis disease modifying treatments at screening.

Key exclusion criteria included: a history of relapsing remitting multiple sclerosis, secondary progressive, or progressive relapsing multiple sclerosis at screening; contraindications for Magnetic Resonance Imaging (MRI); known presence of other neurologic disorders; known active infection or history of or presence of recurrent or chronic infection; history of cancer, including solid tumors and hematological malignancies (except for basal cell, *in situ* squamous cell carcinomas of the skin and in situ carcinoma of the cervix that have been excised and resolved); previous treatment with B-cell targeted therapies (e.g. rituximab, ocrelizumab, atacicept, belimumab, or ofatumumab); any previous treatment with lymphocyte trafficking blockers (e.g. natalizumab, FTY720), any previous treatment with alemtuzumab (CAMPATH^{®}, LEMTRADA^{™}), anti-CD4, cladribine, cyclophosphamide, mitoxantrone, azathioprine, mycophenolate mofetil, cyclosporine, methotrexate, total body irradiation, or bone marrow transplantation; treatment with β interferons, glatiramer acetate, i.v., immunoglobulin, plasmapheresis, or other immunomodulatory therapies within 12 weeks prior to randomization; systemic corticosteroid therapy within 4 weeks prior to screening; and any concomitant disease that may require chronic treatment with systemic corticosteroids or immunosuppressants during the course of the study. The screening period may be extended (but cannot exceed 8 weeks) for patients who have used systemic corticosteroids for their MS before screening. For a patient to be eligible, systemic corticosteroids should not have been administered between screening and baseline.

### Study Design

Patients were randomized 2:1 to receive either to receive either ocrelizumab (300 mg intravenously, 2 infusions separated by 14 days in each treatment cycle (i.e., exposure) or placebo. The patients were stratified by age (≤ 45 vs. > 45) and region (United States vs. rest of the world).

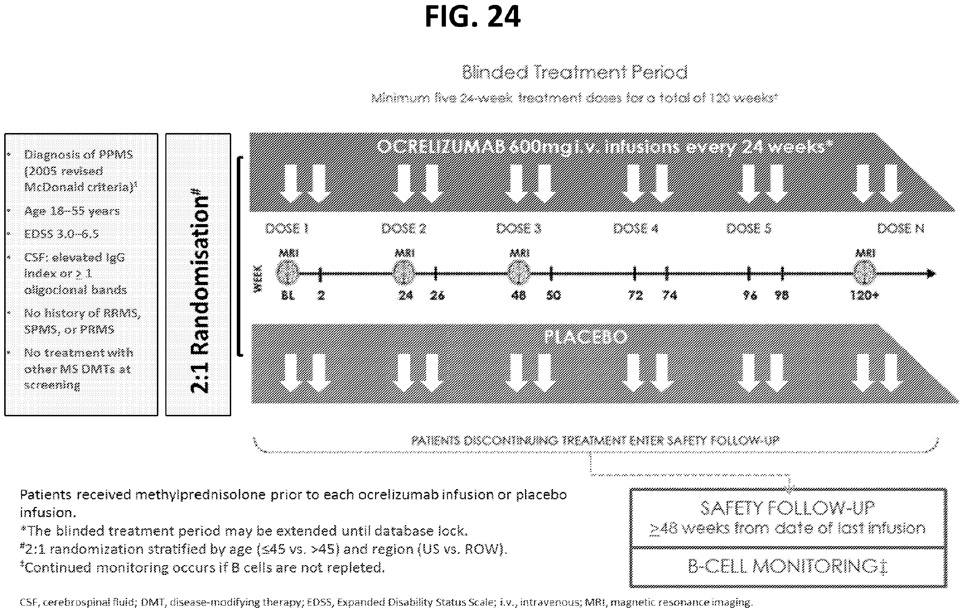

The study consisted of the following periods (summarized in **FIG. 24****):**
Screening for up to 4 weeks.

**Blinded Treatment Period:** All patients underwent at least 120 weeks of study treatment representing five treatment cycles, each cycle 24 weeks in duration. As patients were recruited over a 12-18 month period, this blinded treatment period extended up to 3.5-4 years for the first group of patients enrolled into the study. As shown in **FIG. 24****,** Ocrelizumab doses were administered as dual intravenous infusions of 300 mg x 2 separated by 14 days. Thirty minutes prior to the start of each ocrelizumab or placebo infusion, patients received 100 mg methylprednisolone intravenously. Preinfusion treatment with an oral analgesic/antipyretic (e.g., acetaminophen), and an oral antihistamine (e.g., diphenhydramine) was also recommended. The blinded treatment period was designed to end when approximately 253 events were reached, based on the original sample size assumptions. If the number of events had not been reached by week 120, the study would continue until the target number of events had been reached.

**Open-label Treatment Period:** Following the primary unblinding, patients who can benefit from further treatment receive open-label ocrelizumab. Patients randomized to the ocrelizumab group continue open-label treatment with a dual ocrelizumab infusion (300 mg i.v. infusions administered 14 days apart) every 24 weeks. Patients randomized to placebo receive a dual ocrelizumab infusion for the first open-label treatment cycle, *i.e.,* two i.v. infusions administered 14 days apart, beginning with the next regularly scheduled visit following the interim database lock and primary analysis.

### Study Procedures

The primary efficacy endpoint was time to onset of confirmed disability progression over the treatment period, defined as an increase in EDSS that was sustained for at least 12 weeks, based on regularly scheduled visits. Confirmed disability progression (CDP) refers to an at least 12-week sustained increase of at least 1.0 from baseline EDSS if the baseline score was no greater than 5.5, or an increase of at least 0.5 if the baseline score was greater than 5.5.

Secondary endpoints included: time to onset of confirmed disability progression over the treatment period, defined as an increase in EDSS that was sustained for at least 24 weeks (i.e., 24-week CDP), based on regularly scheduled visits; change in Timed 25-foot walk (T25-FW) from baseline to Week 120; percent change in MRI total T2 lesion volume from baseline to Week 120; percent change in MRI total brain volume from Week 24 to Week 120; and change in Short Form-36 (SF-36) physical component score from baseline to Week 120, and safety and tolerability of ocerlizumab.

Exploratory endpoints included time to onset of 12-week and 24-week confirmed composite disability progression (defined as the first confirmed occurrence of EDSS progression, or at least 20% increase in timed 25-foot walk, and/or at least 20% increase in 9-hole peg test (9-HPT) time), time to sustained progression of at least 20% in timed 25-foot walk, time to sustained progression of at least 20% in 9-hole peg test during the treatment period, and total number of new or enlarging T2 lesions as detected by brain MRI from baseline to Week 120. The 9-HPT is a standardized, quantitative test of upper extremity function and fine manual dexterity. It is the second component of the MSFC (Multiple Sclerosis Functional Composite).

Additional sensitivity analysis was consistent with the primary results and was performed to evaluate an influence of on-study relapses on confirmed disability progression data by excluding patients who experienced physician-reported clinical relapses including protocol-defined relapses **(Table 12A).** Protocol-defined relapses were reported for 11% of patients in the placebo group and 5% in the ocrelizumab group. A subgroup analysis of key clinical and MRI efficacy endpoints in patients with and without T1 gadolinium-enhancing lesions at baseline was consistent with the overall study population **(Tables 12C-12E** below).

**Table 12A: Sensitivity Analysis of Primary Endpoint and First Secondary Endpoint Exploring Influences of Clinical Relapses**

| | | **Excluding patients with clinical relapses** | |
|---|---|---|---|
| **Endpoints** | | **Placebo (n=204)** | **Ocrelizumab 600 mg (n=456)** |
| **Primary endpoint** | | | |
| Confirmed disability progression for at least 12 wks | | 0.74 (0.56-0.98) | |
| | Hazard ratio vs. placebo (95% CI) | P=0.0324 | |
| | P-value | | |

| **Secondary endpoint** | | | |
|---|---|---|---|
| Confirmed disability progression for at least 24 wks | | 0.71 (0.53-0.95) | |
| | Hazard ratio vs. placebo (95% CI) | P=0.0188 | |
| | P-value | | |

### Statistical Analysis

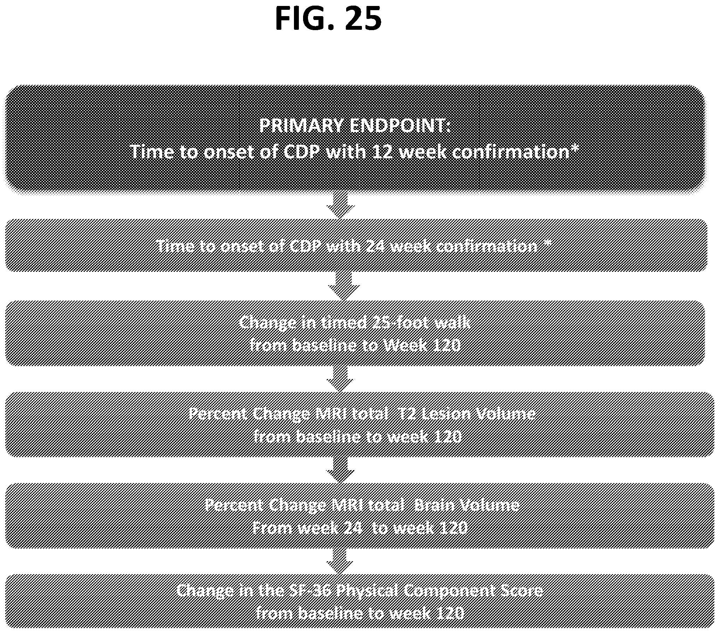

The statistical hierarchy is provided in **FIG. 25****.**

### Results

### Patients

As shown in **Table 12B,** patients' multiple sclerosis disease histories and characteristics were well balanced.

**Table 12B. Patient Demographics and Baseline Disease Characteristics**

| | | | **Placebo n=244** | **Ocrelizumab 600 mg N= 488** |
|---|---|---|---|---|
| **Age, yr, mean (SD)** | | | 44.4 (8.3) | 44.7 (7.9) |
| **Median (range)** | | | 46.0 (18-56) | 46.0 (20-56) |
| **Female, n (%)** | | | 124 (50.8) | 237 (48.6) |
| **Time since symptom onset, yr, mean (SD)** | | | 6.1 (3.6) | 6.7 (4.0) |
| **Median (range)** | | | 5.5 (0.9-23.8) | 6.0 (1.1-32.9) |
| **Time since diagnosis, yr, mean (SD)** | | | 2.8 (3.3) | 2.9 (3.2) |
| **Median (range)** | | | 1.3 (0.1-23.8) | 1.6 (0.1-16.8) |
| **MS disease modifying treatment naive, n (%)** | | | 214 (87.7) | 433 (88.7) |
| **EDSS, mean (SD)** | | | 4.7 (1.2) | 4.7 (1.2) |
| **Median (range)** | | | 4.5 (2.5-6.5) | 4.5 (2.5-7.0) |
| **MRI findings** | | | | |
| | **Patients with 0 Gd⁺ T1 lesions, n (%)** | | 183 (75.3) | 351 (72.5) |
| | **Patients with Gd⁺ T1 lesions, n (%)** | | 60 (24.7%) | 133 (27.5) |
| | | **1 lesion** | 29 (11.9) | 62 (12.8) |
| | | **2 lesions** | 15(6.2) | 22 (4.5) |
| | | **3 lesions** | 5 (2.1) | 17 (3.5) |
| | | **≥ 4 lesions** | 11 (4.5) | 32 (6.6) |
| | **Number of Gd⁺ T1 lesions, mean (SD)** | | 0.6 (1.6) | 1.2 (5.1) |
| | **Number of T2 lesions, mean (SD)** | | 48.15 (39.31) | 48.71 (38.16) |
| | **Median (range)** | | 43 (0-208) | 42.0 (0-249) |
| | **T2 lesion volume, cm³, mean (SD)** | | 10.9 (13.0) | 12.7 (15.1) |
| | **Median (range)** | | 6.17 (0-81.1) | 7.31 (0-90.3) |
| | **Normalised brain volume, cm³, mean (SD)** | | 1469.9 (88.7) | 1462.9 (83.9) |
| | **Median (range)** | | 1464.51 (1216.3-1701.7) | 1462.23 (1214.3-1711.1) |

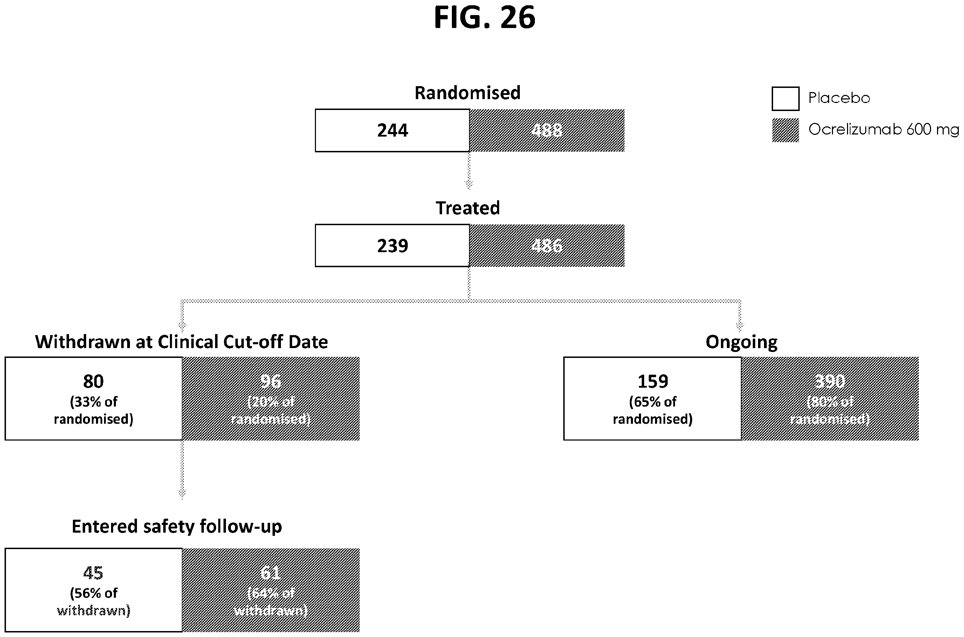

As shown in **FIG. 26****,** the characteristics of the intent-to-treat population were well balanced. At database lock for primary analysis 390 (80%) treated patients in the ocrelizumab group versus 159 (67%) in the placebo group remained on treatment (median study duration was 2.8 years with placebo and 2.9 years with ocrelizumab). Of patients who withdrew from the study before the database lock, 61 (64%) in the ocrelizumab group versus 45 (56%) in the placebo group entered safety follow-up.

### Disability

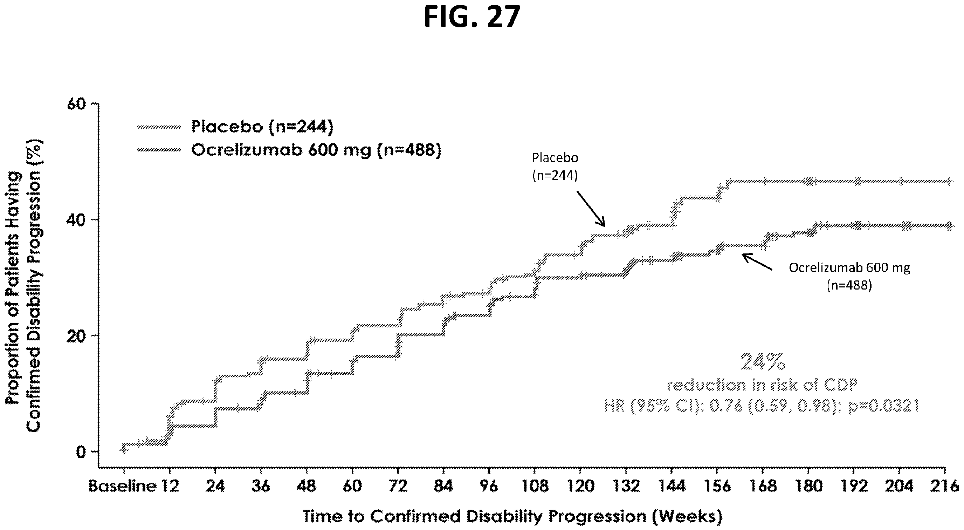

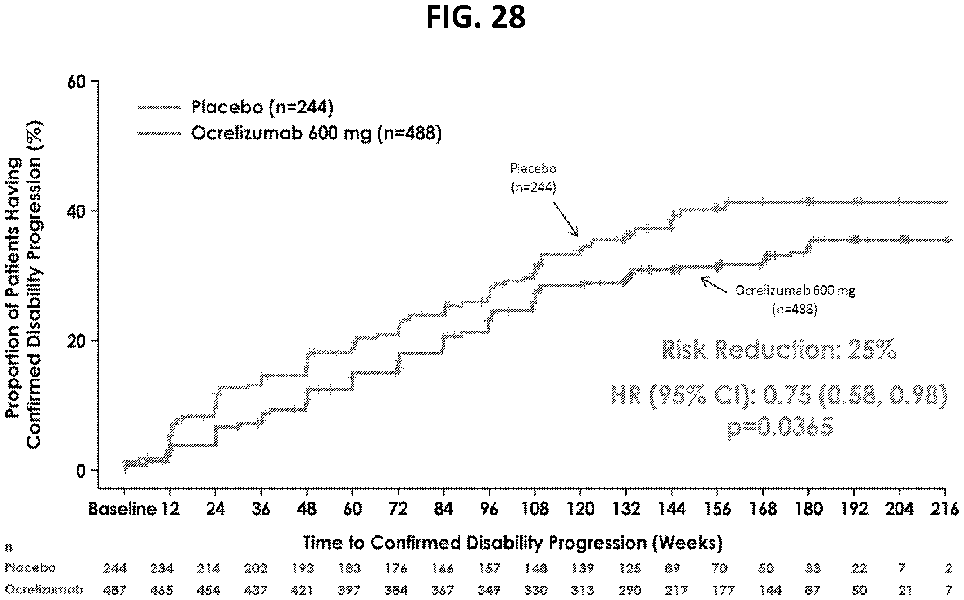

The time to confirmed disability progression that was sustained for at least 12 weeks in patients receiving 600mg ocrelizumab compared to patients receiving placebo is provided in **FIG. 27** (HR - hazard ratio). The time to confirmed disability progression that was sustained for at least 24 weeks in patients receiving 600mg ocrelizumab compared to patients receiving placebo is provided in **FIG. 28****.** ITT patients with initial disability progression who discontinued treatment early with no confirmatory EDSS assessment were considered as having confirmed disability progression, p-value based on log-rank test stratified by geographic region and age. Ocrelizumab reduced the risk of CDP sustained for ≥ 12 weeks by 24% versus placebo and reduced the risk of CDP sustained for ≥ 24 weeks by 25% versus placebo. Analyses in **FIGs. 27** and **28** were based on ITT population; p-value based on log rank test stratified by geographic region and age. Patients with initial disability progression who discontinued treatment early with no confirmatory EDSS assessment were considered as having confirmed disability progression.

Risk of 12-week CDP was reduced by 35% in ocrelizumab-receiving patients with T1 Gd⁺ lesions at baseline (hazard ratio, 0.65; 95% CI, 0.40-1.06; p=0.0826) vs. 16% in ocrelizumab-receiving patients without T1 Gd⁺ lesions at baseline (hazard ratio, 0.84; 95% CI, 0.62-1.13; p=0.2441). *See* **Table 12C** below.

**Table 12C**

| | **Total** | **Placebo (N=244)** | | **Ocrelizumab 600mg (N=488)** | | **Hazard Ratio** | **95% CI** | **p value** |
|---|---|---|---|---|---|---|---|---|
| | **n** | **n** | **Events** | **n** | **Events** | | | |
| **Overall population** | 731 | 244 | 96 | 487 | 160 | 0.76 | (0.59, 0.98) | 731 |
| **T1 Gd⁺-enhancing lesions at baseline** | 193 | 60 | 27 | 133 | 43 | 0.65 | (0.40, 1.06) | 0.0826 |
| **No T1 Gd⁺-enhancing lesions at baseline** | 533 | 183 | 68 | 350 | 115 | 0.84 | (0.62, 1.13) | 0.2441 |

Risk of 24-week CDP was reduced by 33% in ocrelizumab-receiving patients with T1 Gd⁺ lesions at baseline (hazard ratio, 0.67; 95% CI, 0.40-1.14; p=0.1417) vs. 19% in ocrelizumab-receiving patients without T1 Gd⁺ lesions at baseline (hazard ratio, 0.81; 95% CI, 0.59-1.10; p=0.1783). *See* **Table 12D** below.

**Table 12D**

| | **Total** | **Placebo (N=244)** | | **Ocrelizumab 600mg (N=488)** | | **Hazard Ratio** | **95% CI** |
|---|---|---|---|---|---|---|---|
| | **n** | **n** | **Events** | **n** | **Events** | | |
| **Overall population** | 731 | 244 | 87 | 487 | 144 | 0.75 | (0.58, 0.98) |
| **T1 Gd-enhancing lesions at baseline** | 193 | 60 | 23 | 133 | 39 | 0.67 | (0.40, 1.14) |
| **No T1 Gd-enhancing lesions at baseline** | 533 | 183 | 63 | 350 | 103 | 0.81 | (0.59, 1.10) |

Additional subgroup analyses of key clinical and MRI endpoints in patients with or without gadolinium-enhancing lesions on MRI scan at baseline are summarized in **Table 12E** below.

**Table 12E**

| **Endpoints** | | **Placebo (n=244)** | | **Ocrelizumab 600 mg (n=488)** | | | |
|---|---|---|---|---|---|---|---|
| | | **Gd lesions** | **No Gd lesions** | **Gd lesions** | **P value** | **NoGd lesions** | **P value** |
| **Primary endpoint** | | | | | | | |
| Confirmed disability progression for at least 12 wks | | 27/60 | 68/183 | 43/133 0.65 (0.40-1.06) | P=0.082 6 | 115/350 0.84 (0.62-1.13) | P=0.244 1 |
| | Patients with event - n Hazard ratio vs. placebo (95% CI) | | | | | | |

| **Secondary endpoints** | | | | | | | |
|---|---|---|---|---|---|---|---|
| Confirmed disability progression for at least 24 wks | | 23/60 | 63/183 | 39/133 0.67 (0.40-1.14) | P=0.141 7 | 103/350 0.81 (0.59-1.10) | P=0.178 3 |
| | Patients with event - n Hazard ratio vs. placebo (95% CI) | | | | | | |
| T25FW from baseline to wk 120 | | 39 | 134 | 106 0.859 (0.655-1.112) | P=0.246 4 | 288 0.922 (0.801-1.060) | P=0.253 6 |
| | No. in group | | | | | | |
| | Ratio of adjusted geometric means (95% CI) | | | | | | |
| T2 lesion volume from baseline to wk 120 | | 39 | 144 | 107 0.859 (0.818-0.901) | P<0.000 1 | 291 0.913 (0.885-0.943 | P<0.000 1 |
| | No. in group | | | | | | |
| | Ratio of adjusted geometric means (95% CI) | | | | | | |
| Whole brain volume from wk 24-120 | | 31 | 119 | 83 18.1 (-21.1 to 57.2) | P=0.362 5 | 241 20.6 (3.3 to 37.9) | P=0.019 8 |
| | No. in group | | | | | | |
| | Difference in adjusted mean vs. placebo - % (95% CI) | | | | | | |

| **Exploratory endpoints** | | | | | | | |
|---|---|---|---|---|---|---|---|
| 12-week confirmed composite disability progression* | | 43/60 | 127/18 3 | 78/133 0.77 (0.52-1.13) | P=0.182 4 | 206/351 0.75 (0.60-0.94) | P=0.010 9 |
| | Patients with event - n Hazard ratio vs. placebo (95% CI) | | | | | | |
| 12-week confirmed progression in T25FW by at least 20% | | 38/60 | 106/18 3 | 65/133 0.67 (0.45-1.02) | P=0.059 1 | 170/351 0.78 (0.61-0.99) | P=0.043 1 |
| | Patients with event - n (%) | | | | | | |
| | Hazard ratio vs. placebo (95% CI) | | | | | | |
| 12-week confirmed progression in 9HPT by at least 20% | | 22/60 | 43/183 | 24/133 0.42 (0.23-0.76) | P=0.004 4 | 58/351 0.64 (0.43-0.95) | P=0.025 4 |
| | Patients with event - n (%) | | | | | | |
| | Hazard ratio vs. placebo (95% CI) | | | | | | |

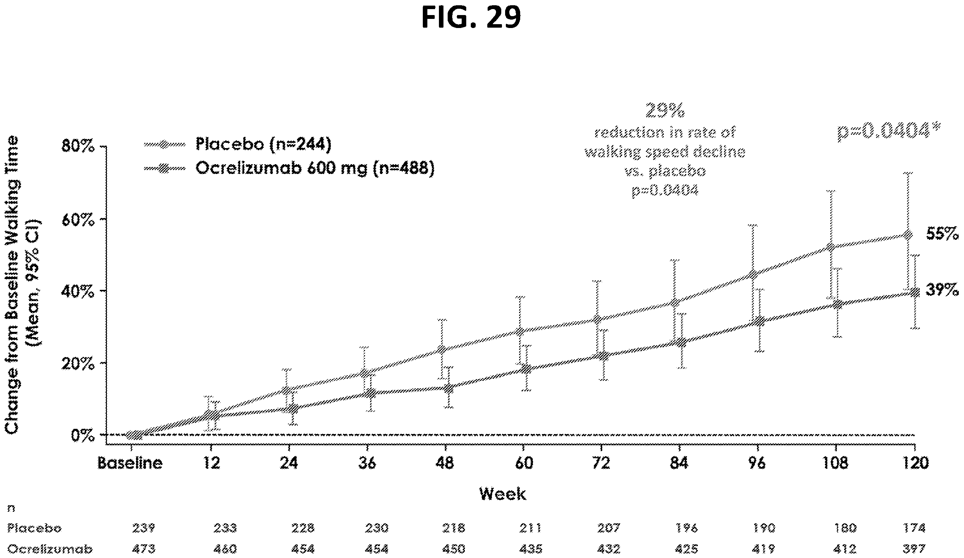

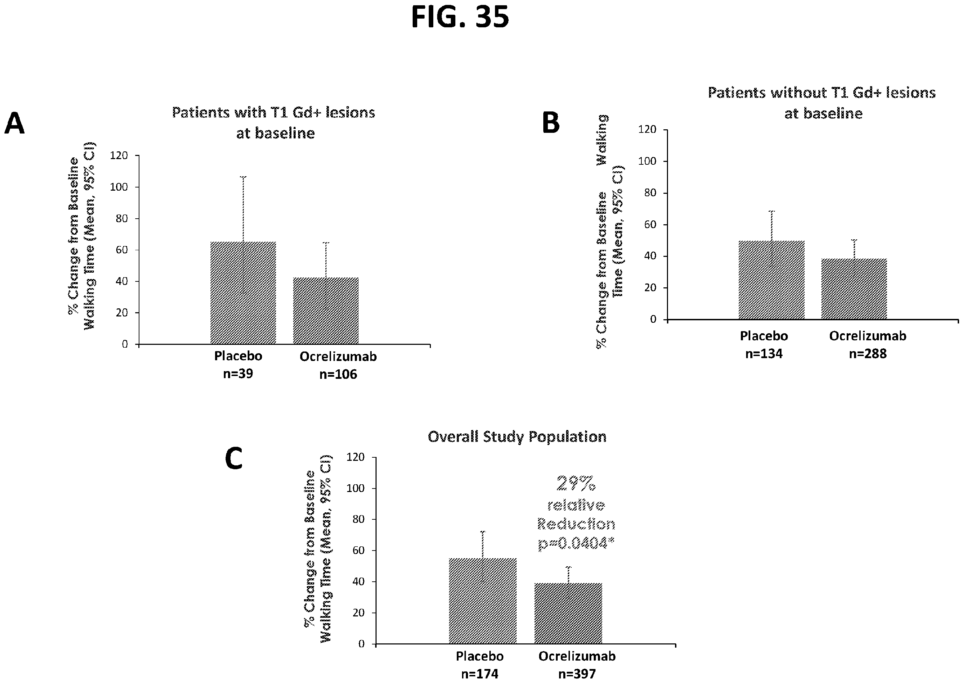

**FIG. 29** shows the rate of decline in walking speed (i.e., reduction in the progression rate of walking time) as measured by the Timed 25-Foot Walk, in patients receiving 600mg ocrelizumab compared to patients receiving placebo from baseline to Week 120. Ocrelizumab reduced the rate of walking speed decline by 29% versus placebo. Analysis was based on the ITT population; p-value was based on ranked ANCOVA at 120 week visit adjusted for baseline 25-foot timed walk, geographic region and age with missing values imputed by last observation carried forward *(i.e.,* LOCF). Points estimates and 95% CIs were based on log-transformed data. Analysis in **FIG. 19** is based on ITT (intent-to-treat population); p-value base on ranked ANCOVA at Week 120 visit adjusted for baseline Timed 25-Foot Walk, geographic regions, and age , with missing values imputed by LOCF (last observation carried forward). Point estimates and 95% CI (confidence interval) was based on MMRM analysis and log-transformed data adjusted for baseline timed 25-foot walk, geographic region, and age. The reduction in the percent change from baseline walking time to Week 120 with ocrelizumab vs placebo in patients with Gd⁺ lesions at baseline *(see* **FIG. 35A****)** and without T1 Gd⁺ lesions at baseline *(see* **FIG. 35B****)** was consistent with the overall study population *(see* **FIG. 35C****).** The analysis in **FIGs. 35A-C** were based on intent-to-treat population; p-value based on ranked ANCOVA at 120-week visit adjusted for baseline timed 25-foot walk, geographic region and age with missing values imputed by LOCF (*i.e.,* last observation carried forward). Point estimates and 95% confidence intervals based on MMRM analysis on log-transformed data adjusted for baseline timed 25-foot walk, geographic region and age.

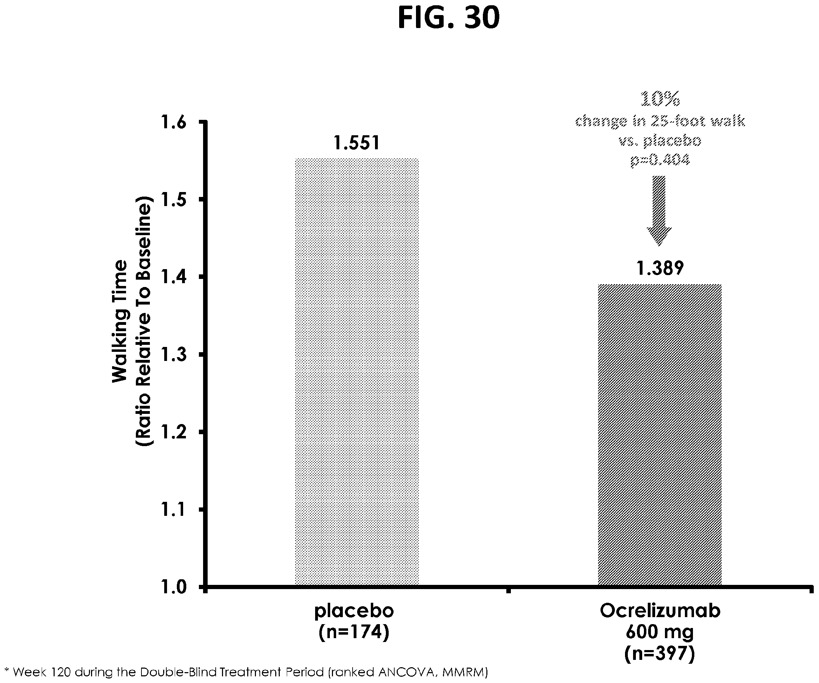

**FIG. 30** shows the rate of decline in walking speed at Week 120 relative to baseline in patients receiving 600mg ocrelizumab compared to patients receiving placebo. Ocrelizumab reduced the rate of decline in walking speed by 10% versus placebo (p=0.404).

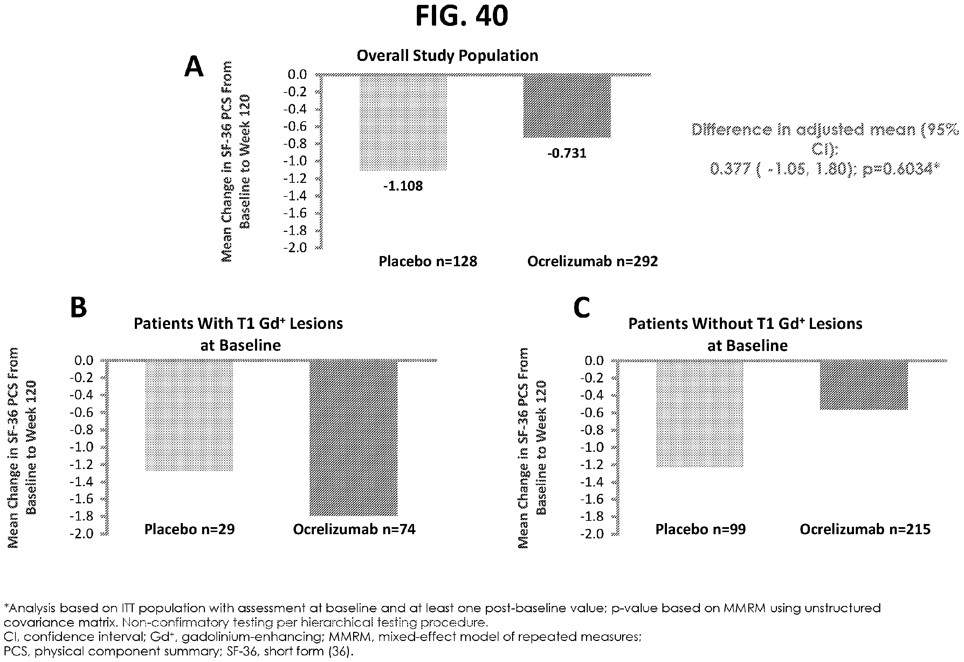

**FIG. 40A** shows the change in SF-36 Physical Component Summary score from base line to Week 120 in patients in the overall study population receiving 600mg ocrelizumab compared to patients in the overall study population receiving placebo. **FIG. 40B** shows the change in SF-36 Physical Component Summary score from base line to Week 120 in patients with T1 gadolinium-enhancing lesions at baseline receiving 600mg ocrelizumab compared to patients with T1 gadolinium-enhancing lesions at baseline receiving placebo. **FIG. 40C** shows the change in SF-36 Physical Component Summary score from base line to Week 120 in patients without T1 gadolinium-enhancing lesions at baseline receiving 600mg ocrelizumab compared to patients without T1 gadolinium-enhancing lesions at baseline receiving placebo.

### MRI-related endpoints

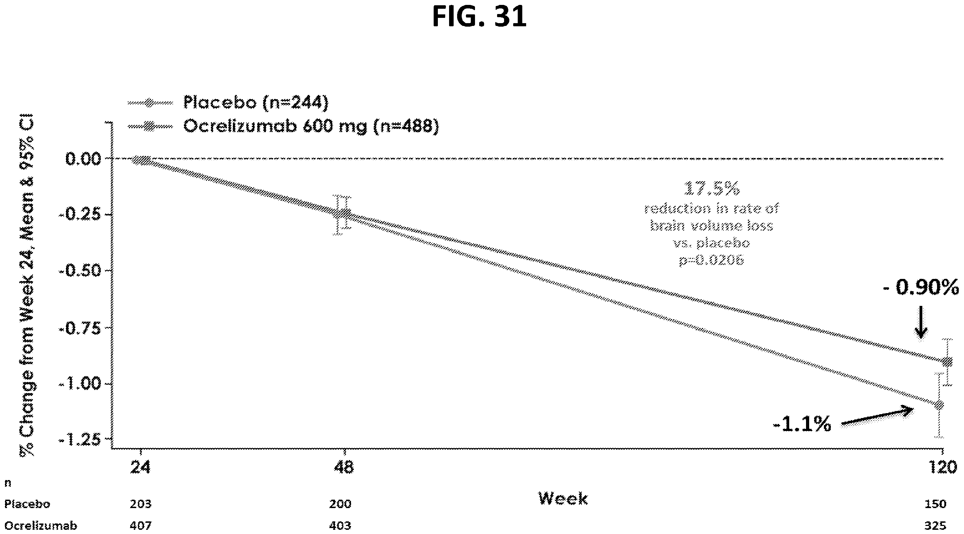

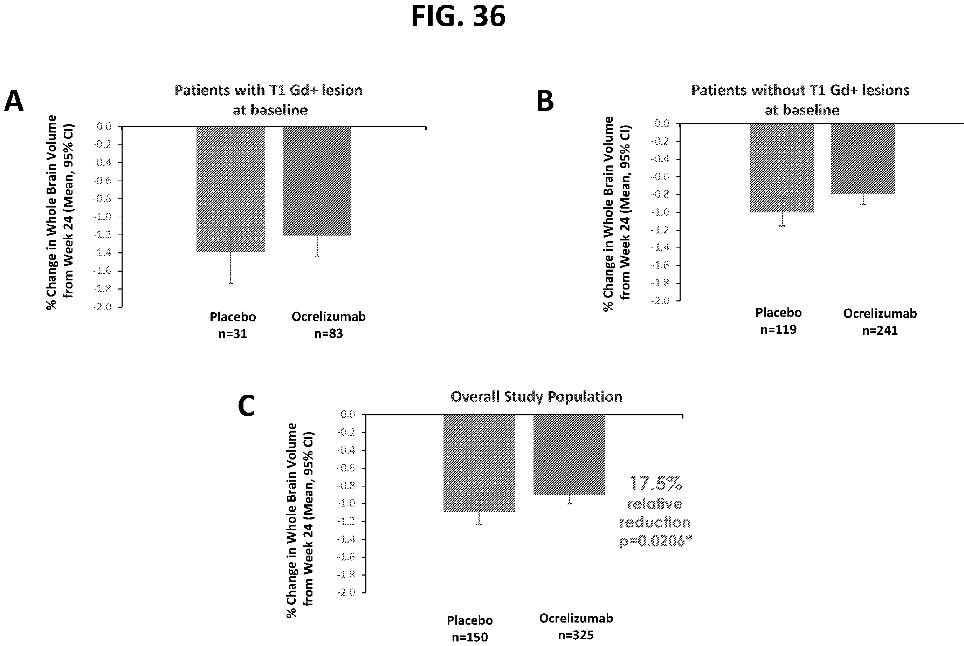

**FIG. 31** shows the percent change of whole brain volume from Week 24 to Week 96 in patients receiving 600mg ocrelizumab compared to patients receiving placebo. Ocrelizumab reduced the rate of whole brain volume loss from Week 24 to Week 96 by 17.5% as compared to placebo (p=0.0206). Analysis was based on ITT population with Week 24 and at least one post-Week 24 assessment; p-value was based on MMRM at Week 120 visit adjusted for Week 24 brain volume, geographic region and age. The rate of whole brain volume loss from Week 24 to Week 120 in OCR-treated patients with T1 Gd⁺ lesions at baseline *(see* **FIG. 36A****)** and without T1 Gd⁺ lesions at baseline *(see* **FIG. 36B****)** was consistent with the overall population data *(see* **FIG. 36C****).** Analyses in **FIGs. 36A-C** were based on intent-to-treat population with week 24 and at least one post-week 24 assessment; p-value based on MMRM at 120 week visit adjusted for week 24 brain volume, geographic region and age.

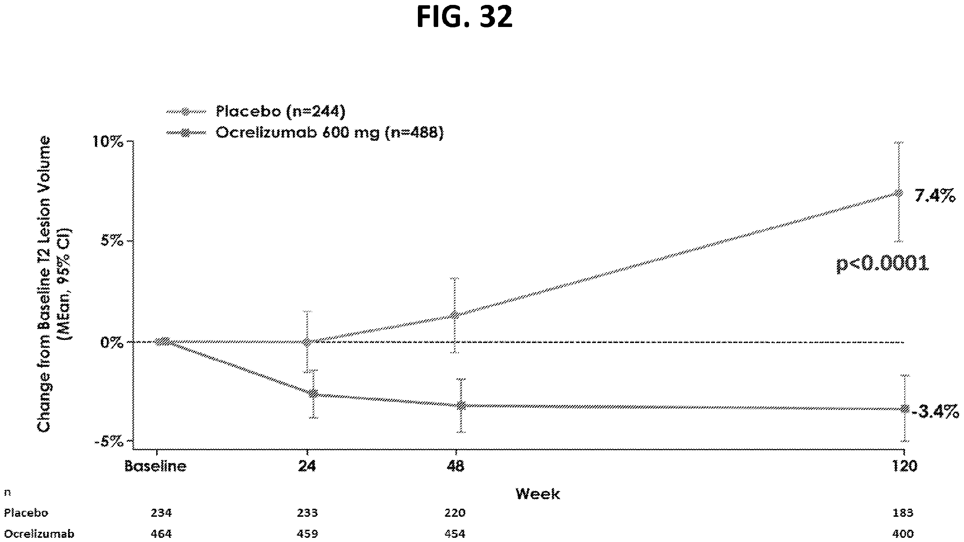

Patients receiving 600mg ocrelizumab showed substantial reduction in T2 lesion volumes from baseline to Week 120 compared to patients receiving placebo (p<0.0001). As shown in **FIG. 32****,** T2 lesion volume increased by 7.4% in patients in the placebo arm, whereas T2 lesion volume decreased by 3.4% in patients receiving ocrelizumab. Analysis was based on intent-to-treat population; p-value was based on ranked ANCOVA at 120 week visit adjusted for baseline T2 lesion volume, geographic region and age with missing values imputed by LOCF (last observation carried forward). Point estimates and 95% CIs (confidence intervals) based on MMRM analysis on log-transformed data adjusted for baseline T2 lesion volume, geographic region, and age.

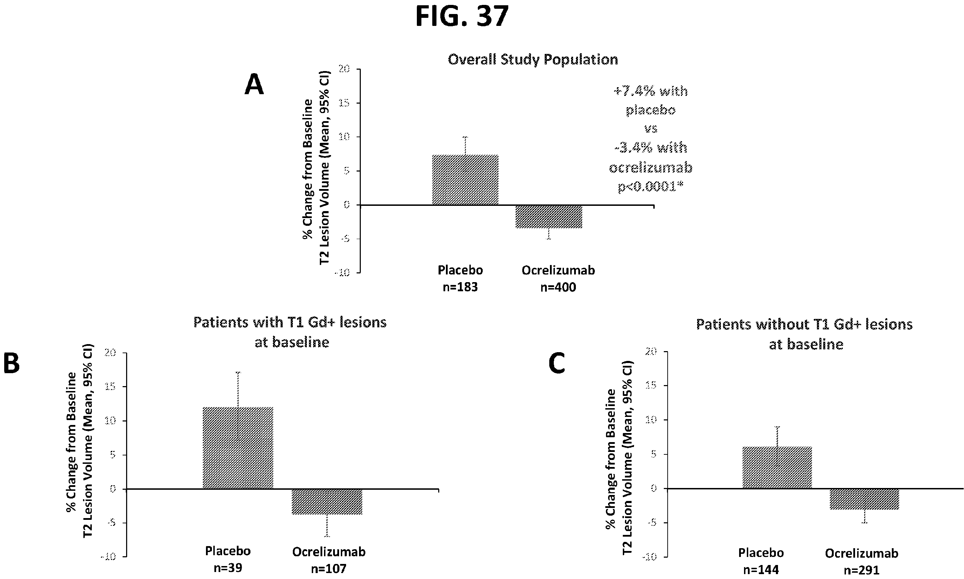

Ocrelizumab treatment reduced the total volume of brain T2 hyperintense lesions from baseline to Week 120 in patients with and without T1 Gd⁺ lesions at baseline, whereas the total lesion volume increased in patients with and without T1 Gd⁺ lesions at baseline in the placebo group. *See* **FIG. 37A****.**

In patients having T1 Gd⁺ lesions at baseline, total T2 lesion volume was reduced by 3.8% in ocrelizumab-treated patients (95% CI, -7.0 to -0.5). By contrast, total T2 lesion volume increased by 12.0% in patients receiving placebo (95% CI, 7.2-17.1; p<0.001). *See* **FIG. 37B****.** In patients without T1 Gd⁺ lesions at baseline, total T2 lesion volume was reduced by 3.1% in ocrelizumab-treated patients (95% CI, -5.0 to -1.1). By contrast, total T2 lesion volume increased by 6.1% in patients receiving placebo (95% CI, 3.3-9.0; p<0.001). *See* **FIG. 37C****.** Analyses in **FIGs. 37A-C** were based on ITT population; p-value based on ranked ANCOVA at 120-week visit adjusted for baseline T2 lesion volume, geographic region and age with missing values imputed by LOCF (last observation carried forward). Point estimates and 95% CIs based on MMRM analysis on log-transformed data adjusted for baseline T2 lesion volume, geographic region and age.

Patients receiving 600mg ocrelizumab showed a 97.6% reduction of T1 gadolinium enhancing lesions compared to patients receiving placebo (p<0.0001). Patients receiving 600mg ocrelizumab also showed a 91.9% reduction of new and/or enlarging T2 lesions compared to patients receiving placebo (p<0.0001). The total number of T1 Gd⁺ lesions and total number of new and/or enlarging T2 lesions was divided by the sum of the individual number of lesions at Weeks 24, 48 and 120) for all patients in the treatment group by the total number of brain MRI scans. Adjusted by means calculated by negative binomial regression and adjusted for baseline *T1 Gd⁺ lesion (present or not) or ^{†}T2 lesion count, baseline age (≤45 vs >45 years) and geographical region (US vs rest of world).

### Efficacy

The efficacy endpoints for this study are summarized in **Table 13A.** Clinical and MRI endpoints (ITT population) are summarized in **Table** 13B.

**Table 13A: Summary of Efficacy**

| **Endpoint** | **Risk Reduction** | **p value** |
|---|---|---|
| **Time to CDP 12 week** | 24% | 0.0321 |
| **Time to CDP 24 week** | 25% | 0.0365 |
| **Change in Timed 25-Foot Walk (baseline to Week 120)** | 29% | 0.0404 |
| **Percent change in MRI total T2 lesion volume (baseline to Week 120)** | placebo: 7.4% increase | <0.0001 |
| | ocrelizumab: 3.4% decrease | |
| **Percent change in MRI total brain volume (Week 24 to Week 120)** | 17.5% | 0.0206 |
| **Change in SF-36 PCS (baseline to Week 120)** | | |

**Table 13B: Clinical and MRI endpoints (ITT population)**

| **Endpoints** | | **Placebo (n=244)** | | **Ocrelizumab 600 mg (n=488)** | |
|---|---|---|---|---|---|
| **Clinical endpoints** | | | | | |
| ***Primary* endpoint** | | | | | |
| Confirmed disability progression for at least 12 wks | | 96 (39.3) | | 160 (32.9) 0.76 (0.59-0.98) P=0.0321 | |
| | Patients with event: n (%) | | | | |
| | Hazard ratio vs. placebo (95% CI) | | | | |
| | P-value | | | | |

| ***Secondary* endpoints** | | | | | |
|---|---|---|---|---|---|
| Confirmed disability progression for at least 24 weeks | | 87 (35.7) | | 144 (29.6) 0.75 (0.58-0.98) P=0.0365 | |
| | Patients with event - n (%) | | | | |
| | Hazard ratio vs. placebo (95% CI) | | | | |
| | P-value | | | | |
| T25FW from baseline to Week 120 | | 55.10 | | 39.93 29.337 (-1.618 to 51.456) P=0.0404 | |
| | Mean percentage change | | | | |
| | Relative reduction vs. placebo - % (95% CI) | | | | |
| | P-value | | | | |

| ***Exploratory endpoints*** | | **12-week CDP** | **24-week CDP** | **12-week confirmed** | **24-week confirmed** |
|---|---|---|---|---|---|
| Composite disability progression* | | 171 (70.1) | 155 (63.5) | 287 (58.8) 0.74 (0.61-0.89) P=0.0014 | 251 (51.4) 0.71 (0.58-0.87) P=0.0008 |
| | Patients with event - n (%) | | | | |
| | Hazard ratio vs. placebo (95% CI) | | | | |
| | P-value | | | | |
| Progression in T25FW by at least 20% | | | | | |
| | Patients with event - n (%) | 145 (59.4) | 127 (52.0) | 238 (48.8) | 202 (41.4) |
| | Hazard ratio vs. placebo (95% CI) | | | 0.75 (0.61-0.92) | 0.73 (0.59-0.91) |
| | P-value | | | P=0.0053 | P=0.0055 |
| Progression in 9HPT by at least 20% | | 66 (27.0) | 57 (23.4) | | |
| | Patients with event - n (%) | | | 83 (17.0) | 69(14.1) |
| | Hazard ratio vs. placebo (95% CI) | | | 0.56 (0.41-0.78) | 0.55 (0.38-0.77) |
| | P-value | | | P=0.0004 | P=0.0006 |

| **MRI endpoints** | | | | | |
|---|---|---|---|---|---|
| ***Secondary* endpoints** | | | | | |
| T2 lesion volume from baseline to Wk 120 | | 7.426 (4.967-9.942) | | -3.366 (-4.987 to -1.718) | |
| | Adjusted geometric mean percentage change (95% CI) Ratio of adjusted geometric mean vs. placebo (95% CI) | | | 0.900 (0.876-0.924) | |
| | P-value | | | P<0.0001 | |
| Total brain volume from Wk 24-120 | | -1.093±0.072 | | | |
| | Mean percentage change | | | -0.902±0.052 | |
| | Relative reduction vs. placebo - % (95% CI) | | | 17.475 (3.206-29.251) | |

| | P-value | | | P=0.0206 | |
|---|---|---|---|---|---|
| ***Exploratory* endpoint** | | | | | |
| | | | | | |
| New and/or enlarging T2 lesions | | 3.880 (2.841-5.299) | | 0.313 (0.246-0.397) | |
| | Mean no. of lesions per MRI scan (95% CI)^{‡} Percent change (95% CI)^{‡} | | | 0.081 (0.058-0.111) -91.9 (-88.9 to -94.2) | |

| | P-value | | | P<0.0001 | |
|---|---|---|---|---|---|
| **Patient-reported outcomes** | | | | | |
| ***Secondary* endpoint** | | | | | |
| SF-36 physical component summary score from baseline to Week 120 | | -1.108 (-2.394 to 0.177) | | -0.731 (-1.655 to 0.193) | |
| | Adjusted mean change (95% CI) | | | 0.377 (-1.048 to 1.802) | |
| | Difference in adjusted means (95% CI) | | | P=0.6034 | |
| P-value | | | | | |

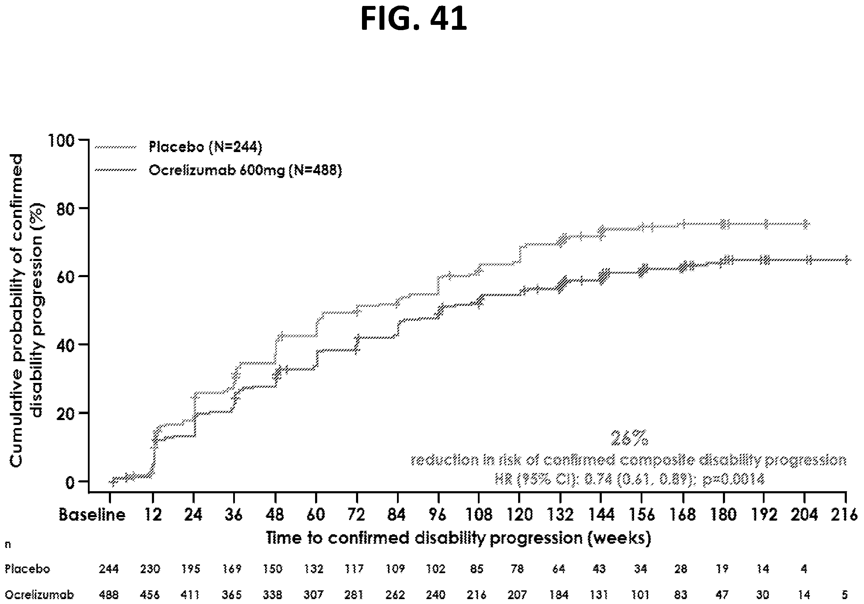

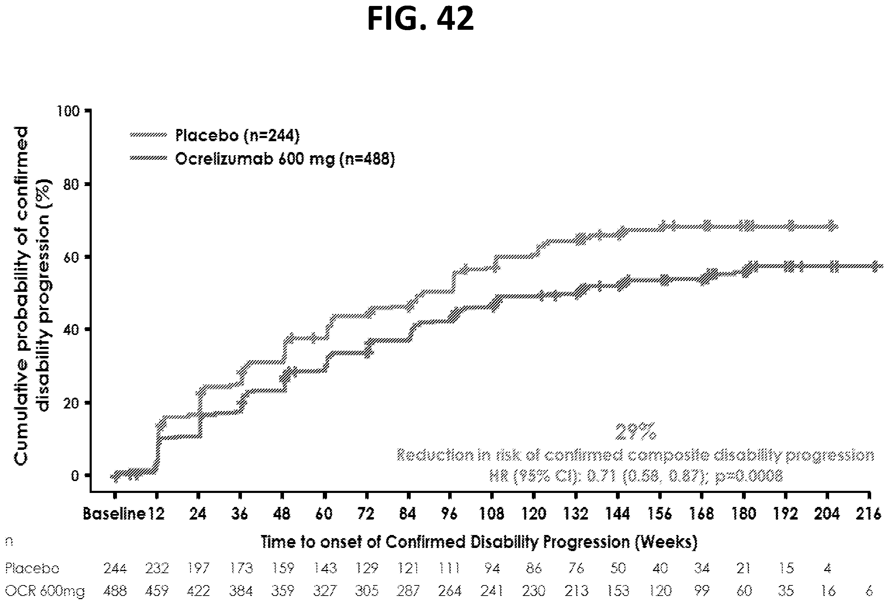

At the time of the primary analysis, compared with placebo, ocrelizumab significantly reduced the risk of 12-week and 24-week confirmed disability progression by 24% (HR = 0.76, 95% CI [0.59, 0.98]; P=0.0321; **Table 13B;** **FIG. 27****)** and by 25% (0.75, 95% CI [0.58, 0.98]; P=0.0365; **Table 13B;** **FIG. 28****),** respectively. At Week 120, ocrelizumab reduced the progression rate of timed 25-foot walk by 29% (P=0.0404) compared with placebo **(Table 13B).** Compared with placebo, ocrelizumab also significantly reduced the risk of 12-week and 24-week confirmed composite disability progression (*i.e.* EDSS progression, or at least 20% progression in timed 25-foot walk test, and/or at least 20% progression in 9-hole peg test) by 26% (HR=0.74 [0.61-0.89], P=0.0014; **Table 13B;** **FIG. 41****),** and by 29% (HR=0.71 [0.58-0.87], P=0.0008; **Table 13B;** **FIG. 42****),** respectively, by the end of the double-blind treatment period. Regarding individual components of composite disability progression assessment, ocrelizumab consistently and significantly decreased the risk of 12-week and 24-week confirmed 20% progression on timed 25-foot walk **(Table 13B)** and on 9-hole peg test compared with placebo **(Table 13B).**

Additional sensitivity analysis was consistent with the primary results and was performed to evaluate an influence of on-study relapses on confirmed disability progression data by excluding patients who experienced physician-reported clinical relapses including protocol-defined relapses **(Table S1).** Protocol-defined relapses were reported for 11% of patients in the placebo group and 5% in the ocrelizumab group. A subgroup analysis of key clinical and MRI efficacy endpoints in patients with and without T1 gadolinium-enhancing lesions at baseline was consistent with the overall study population **(Table S2).** However, the ORATORIO study was not powered to demonstrate efficacy differences between these subgroups.

### Safety

### Infusion-Related Reactions

725 patients were included in the safety analysis (i.e., 239 in the placebo group and 486 in the ocrelizumab group). Throughout a mean treatment duration of approximately 3 years, the proportion of patients with at least one infusion-related reaction (IRR) was 25.5% (n=61) for patients given placebo and 39.9% (n=194) for ocrelizumab-treated patients; most were mild-to-moderate in severity (93.4% [n=57] and 96.9% [n=188], respectively). The most common IRR symptoms included skin and subcutaneous tissue disorders (45.9% [n=89] for ocrelizumab-treated patients and 13.1% [n=8] for patients given placebo). There were no life-threatening or fatal IRRs. The incidence of IRRs in ocrelizumab-treated patients was highest with the first infusion (27.4%) and decreased markedly with subsequent dosing (11.6% at first infusion of dose 2). Overall, IRRs mostly occurred during infusion in ocrelizumab-treated patients (61.3% compared with 37.7% in patients given placebo group). One patient (0.2%) withdrew ocrelizumab treatment due to an IRR during the first infusion.

### Adverse Events

Adverse events reported by > 10% of the patients in each treatment arm until clinical cut off date are provided in Table **14A.**

**Table 14A.**

| **n (%)** | **Placebo (n=239)** | **Ocrelizumab 600 mg (n=486)** |
|---|---|---|
| **Overall patients with ≥1 AE** | 215 (90.0) | 462 (95.1) |
| **Infections and Infestations*** | 162 (67.8) | 339 (69.8) |
| Nasopharyngitis | 65 (27.2) | 110 (22.6) |
| Urinary tract infection | 54 (22.6) | 96 (19.8) |
| Influenza | 21 (8.8) | 56 (11.5) |
| Upper respiratory tract infection | 14 (5.9) | 53 (10.9) |
| Bronchitis | 12 (5.0) | 30 (6.2) |
| Gastroenteritis | 12 (5.0) | 20 (4.1) |
| **Injury, Poisoning and Procedural Complications** | 104 (43.5) | 263 (54.1) |
| **Musculoskeletal and Connective Tissue Disorders** | 98 (41.0) | 181 (37.2) |
| **Nervous System Disorders** | 79 (33.1) | 174 (35.8) |
| **General Disorders and Administration-site Conditions** | 60 (25.1) | 130 (26.7) |
| **Gastrointestinal Disorders** | 60 (25.1) | 126 (25.9) |
| **Psychiatric Disorders** | 59 (24.7) | 89 (18.3) |
| **Skin and Subcutaneous Tissue Disorders** | 44 (18.4) | 99 (20.4) |
| **Respiratory, Thoracic and Mediastinal Disorders** | 35 (14.6) | 87(17.9) |
| **Metabolism and Nutrition disorders** | 28 (11.7) | 56 (11.5) |
| **Renal and Urinary Disorders** | 30 (12.6) | 51 (10.5) |
| **Vascular Disorders** | 26 (10.9) | 54 (11.1) |
| **Investigations** | 20 (8.4) | 58 (11.9) |

| | | |
|---|---|---|
| *For infections and infestations SOC only: events reported by at least 5% of patients in one treatment arm are presented. | | |

The percent of overall patients experiencing more than 1 serious adverse event was 22.2% in the placebo arm as compared to 20.4% in the treatment arm. *See* **Table 14B.** Total serious adverse events were low and similar in both arms.

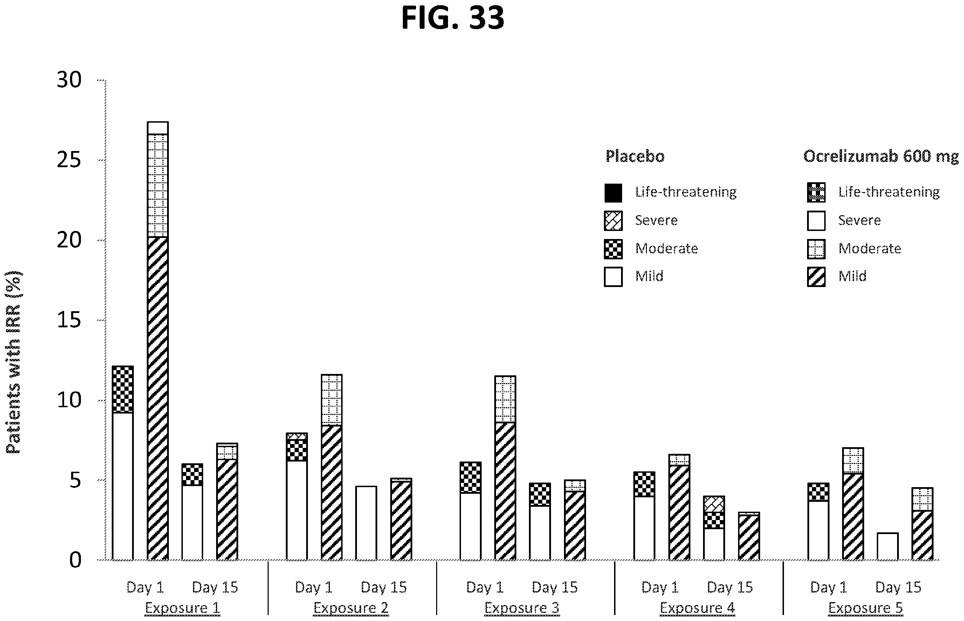

The most common adverse event associated with ocrelizumab was infusion-related reactions (39.9% vs. 25.5% for placebo). One patient (0.2%) withdrew from ocrelizumab treatment due to an IRR at the first infusion. As shown in **FIG. 33****,** infusion related reactions (IRR) decreased in severity with each successive course and with each dose within the same course. Ocrelizumab has a similar safety profile to placebo.

**Table 14B. Total Serious Adverse Events**

| **n (%)** | **Placebo n = 239** | **Ocrelizumab n = 486** |
|---|---|---|
| **Overall patients with ≥1 SAE** | 53 (22.2) | 99 (20.4) |
| **Infections and Infestations** | 14 (5.9) | 30 (6.2) |
| **Injury, Poisoning, and Procedural Complications** | 11 (4.6) | 19 (3.9) |
| **Nervous System Disorders** | 9 (3.8) | 18 (3.7) |
| **Neoplasms: Benign, Malignant, and Unspecified (including cysts and polyps)** | 7 (2.9) | 8 (1.6) |
| **Gastrointestinal Disorders** | 3 (1.3) | 10 (2.1) |
| **Musculoskeletal and Connective Tissue Disorders** | 6 (2.5) | 6 (1.2) |
| **General Disorders and Administration Site Conditions** | 3 (1.3) | 6 (1.2) |
| **Renal and Urinary Disorders** | 3 (1.3) | 5 (1.0) |

Data obtained from this study show that B cells may play a role in PPMS pathophysiology. Ocrelizumab is the first investigational treatment showing a consistent, statistically significant clinical effect in PPMS in a large Phase III study compared to placebo. Ocrelizumab significantly reduced clinical disability progression sustained for 12 weeks (primary endpoint), clinical disability progression sustained for 24 weeks, change in Timed 25-foot walk, change in T2 lesion volume, and brain volume loss. Patients receiving ocrelizumab consistently met key secondary endpoints. Throughout the mean treatment duration of approximately 3 years, ocrelizumab showed a favorable safety profile. Overall incidence of adverse events associated with ocrelizumab was similar to placebo. The most common adverse events were mild-to-moderate infusion-related reactions. Incidence of serious adverse events associated with ocrelizumab, including serious infections, was also similar to placebo.

### Open-Label Extension Phase

Following the randomized, parallel group, double-blind, placebo controlled study, patients may be eligible to enter open-label extension (OLE) phases to evaluate long-term safety, tolerability, and efficacy of ocrelizumab. The design of the open-label extension study, which will evaluate the long-term safety and efficacy of OCR in relapsing MS, is described below.

Following the primary database lock at the completion of the blinded treatment period, patients who can benefit from further treatment receive open-label ocrelizumab. Patients randomized to the ocrelizumab group continue open-label treatment with a dual ocrelizumab infusion (300 mg i.v. infusions administered 14 days apart) every 24 weeks. Patients randomized to placebo receive a dual ocrelizumab infusion for the first open-label treatment cycle, *i.e.,* two i.v. infusions administered 14 days apart, beginning with the next regularly scheduled visit following the interim database lock and primary analysis.

The OLE phase lasts until ocrelizumab is commercially available in the patient's country, as per local regulations or until the sponsor decides to terminate the ocrelizumab MS program. The OLE phase does not exceed 4 years (208 weeks). OLE is not mandatory. Patients unwilling to proceed to the OLE phase are entered into the safety follow-up phase, which is performed in 12-week intervals starting from the date of the patient's latest visit. Continued monitoring occurs if B cells are not repleted.

Key inclusion criteria for the OLE phase include: completion of the blinded treatment period; consent from investigators who determine that the patient may benefit from OCR treatment based on the assessments performed during the treatment period; written informed consent from the patient to enter the OLE phase; and meeting ocrelizumab treatment/retreatment criteria, in which the patient is free from the following conditions and laboratory abnormalities: (a) life-threatening (CTCAE Grade 4) infusion-related event that occurred during a previous OCR infusion; (b) any significant or uncontrolled medical condition or treatment-emergent, clinically significant laboratory abnormality; (c) active infection; (d) absolute neutrophil count <1.5 × 10³/µL; (e) CD4 cell count <250/µL; and (f) hypogammaglobulinemia immunoglobulin G <3.3 g/L.

The schedule of key safety and efficacy assessments are shown in **Table 15** below:

**Table 15**

| **Assessment** | **OLE phase cycles^{a}** | | | **Delayed dosing visit^{b}** | **Unscheduled visit^{c}** | **Withdrawal from treatment visit** |
|---|---|---|---|---|---|---|
| | **Day 1 OLE cycle (±5 days)** | **Day 15 OLE cycle (±5 days)** | **12 weeks post-Day 1 infusion visit Day 1 OLE cycle (±7 days)** | | | |
| **Neurological exam^{d}** | **x** | **x** | **x** | | **x** | **x** |
| **EDSS^{e}** | **x** | | **x** | | **x** | **x** |
| **Potential relapses^{f}** | **x** | **x** | **x** | **x** | **x** | **x** |
| **EQ-5D^{g}** | **x** | | | | | **x** |
| **MRI^{h}** | **x** | | | | | **x** |
| **Routine safety labi** | **x** | | **x** | | **x** | **x** |
| **Concomitant treatments^{j}** | **x** | **x** | **x** | **x** | **x** | **x** |
| **Antibody titres^{k}** | | | | | | **x** |

| | | | | | | |
|---|---|---|---|---|---|---|
| ^{a}Patients continue with blinded 24-week treatment cycles until the last patient receives his/her final course of treatment scheduled at Week 98. At that point, all patients continue to the end of their current (24-week) cycle. If the projected total number of confirmed disability progression events has not been reached at Week 120, then all patients continue with additional blinded 24-week treatment cycles until the projected number of confirmed disability events has been reached. ^{b}A delayed dosing visit is performed when dosing cannot be administered at the scheduled dosing visit. At this time, other tests/assessments may be performed as appropriate. ^{c}During an unscheduled visit (non-dosing) assessments are performed according to the clinical needs of the patient. At this time, other tests/assessments may be performed as appropriate. ^{d}Neurological examination is performed at every planned and unscheduled visit. All patients with new neurological symptoms suggestive of MS worsening or of a relapse should have EDSS performed by examining investigator. 'Expanded Disability Status Scale score is performed in all patients by the examining investigator every 12 weeks. Additional EDSS assessments may be performed between visits (i.e. during an MS relapse, neurological worsening, etc). Disability progression is defined as a ≥1.0 point (where baseline EDSS is ≤5.5) or ≥0.5 point (where baseline EDSS is >5.5) increase from the baseline EDSS score that is not attributable to another aetiology. ^{f}Patients are evaluated for relapses by the treating investigator at each scheduled visit and, if necessary, at unscheduled visits to confirm relapses occurring between the visits. If a relapse is suspected, the EDSS should also be performed in addition to completing the MS relapse electronic case report form. ^{g}The EQ-5D (EuroQol five dimensions questionnaire) assessments will be performed annually. ^{h}Annual brain MRI scans are performed at the start of the OLE phase (if none were taken in the previous 12 weeks), within (±) 4 weeks of scheduled visits and at OLE withdrawal visits (if none were taken in the previous 4 weeks). ⁱRoutine safety laboratory assessments include haematology, chemistry and urinalysis. ^{j}Concomitant medications and procedures are reported at each scheduled and unscheduled visit. ^{k}Antibody titres against common antigens (mumps, rubella, varicella, and *Streptococcus pneumoniae)* are performed. | | | | | | |

The examples, which are intended to be purely exemplary of the invention and should therefore not be considered to limit the invention in any way, also describe and detail aspects and embodiments of the invention discussed above. The foregoing examples and detailed description are offered by way of illustration and not by way of limitation.

## Claims

1. An anti-CD20 antibody for use in a method of improving functional ability in a human patient having multiple sclerosis comprising administering to the patient an effective amount of the anti-CD20 antibody, wherein the patient has improvement in functional ability after treatment; wherein the anti-CD20 antibody is ocrelizumab; wherein the multiple sclerosis is a primary progressive multiple sclerosis (PPMS) with T1 gadolinium staining lesions; and wherein the improvement in functional ability is measured by the Timed 25-Foot Walk (T-25FW) test or EDSS score.

2. The anti-CD20 antibody for use according to claim 1, wherein the improvement in functional ability is measured by the Timed 25-Foot Walk (T-25FW) test and EDSS score.

3. The anti-CD20 antibody for use according to claim 1 or 2, wherein the anti-CD20 antibody is administered to the patient to provide an initial anti-CD20 antibody exposure followed by a second anti-CD20 antibody exposure, wherein the initial and second exposures are each 600 mg of the antibody, and wherein the interval between the initial exposure and the second exposure is 20-24 weeks or 5-6 months.

4. The anti-CD20 antibody for use according to claim 3, wherein the anti-CD20 antibody is administered to the patient to provide a third anti-CD20 antibody exposure, wherein the third exposure is 600 mg of the antibody, and wherein the interval between the second exposure and the third exposure is 20-24 weeks or 5-6 months.

5. The anti-CD20 antibody for use according to claim 4, wherein the anti-CD20 antibody is administered to the patient to provide a fourth anti-CD20 antibody exposure, wherein the fourth exposure is 600 mg of the antibody, and wherein the interval between the third exposure and the fourth exposure is 20-24 weeks or 5-6 months.

6. The anti-CD20 antibody for use according to any one of claims 3-5, wherein the initial exposure, which is 600 mg of the anti-CD20 antibody, comprises a first dose and a second dose of the anti-CD20 antibody, wherein each dose is 300 mg and the first dose and the second dose are separated by two weeks or 14 days.

7. The anti-CD20 antibody for use according to claim 6, wherein the second, third, and/or fourth exposures comprise a single dose of 600 mg.

8. The anti-CD20 antibody for use according to any one of claims 3-5, wherein the initial exposure and second, third, and/or fourth additional exposures comprise a first dose and a second dose of the anti-CD20 antibody, wherein each dose is 300 mg and the first dose and the second dose are separated by two weeks or 14 days.

9. The anti-CD20 antibody for use according to any one of claims 1-8, wherein a second medicament is administered with the initial exposure or later exposures, wherein the anti-CD20 antibody is a first medicament.

10. The anti-CD20 antibody for use according to any one of the preceding claims, wherein the anti-CD20 antibody is in a pharmaceutically acceptable composition.

11. The anti-CD20 antibody for use according to any one of the preceding claims, wherein the anti-CD20 antibody is administered intravenously.

12. The anti-CD20 antibody for use according to claim 11, wherein the anti-CD20 antibody is administered intravenously for each antibody exposure.

13. The anti-CD20 antibody for use according to any one of claims 1-10, wherein the antibody is administered subcutaneously.

14. The anti-CD20 antibody for use according to claim 13, wherein the anti-CD20 antibody is administered subcutaneously for each antibody exposure.

## Patentansprüche

1. Anti-CD20-Antikörper zur Verwendung in einem Verfahren zum Verbessern der Funktionsfähigkeit bei einem menschlichen Patienten mit multipler Sklerose, umfassend das Verabreichen einer wirksamen Menge des Anti-CD20-Antikörpers an den Patienten, wobei nach der Behandlung die Funktionsfähigkeit des Patienten verbessert ist; wobei der Anti-CD20-Antikörper Ocrelizumab ist; wobei die multiple Sklerose eine primäre progressive multiple Sklerose (PPMS) mit T1-Gadolinium-Färbungsläsionen ist und wobei die Verbesserung der Funktionsfähigkeit mit dem Timed 25-Foot-Walk-(T-25FW)-Test oder EDSS-Score gemessen wird.

2. Anti-CD20-Antikörper zur Verwendung nach Anspruch 1, wobei die Verbesserung der Funktionsfähigkeit mit dem Timed 25-Foot-Walk-(T-25FW)-Test und EDSS-Score gemessen wird.

3. Anti-CD20-Antikörper zur Verwendung nach Anspruch 1 oder 2, wobei der Anti-CD20-Antikörper dem Patienten verabreicht wird, um eine initiale Anti-CD20-Antikörperexposition, gefolgt von einer zweiten Anti-CD20-Antikörperexposition, bereitzustellen, wobei die initiale und zweite Exposition jeweils 600 mg des Antikörpers betragen und wobei das Intervall zwischen der initialen Exposition und der zweiten Exposition 20-24 Wochen oder 5-6 Monate beträgt.

4. Anti-CD20-Antikörper zur Verwendung nach Anspruch 3, wobei der Anti-CD20-Antikörper dem Patienten verabreicht wird, um eine dritte Anti-CD20-Antikörperexposition bereitzustellen, wobei die dritte Exposition 600 mg des Antikörpers beträgt und wobei das Intervall zwischen der zweiten Exposition und der dritten Exposition 20-24 Wochen oder 5-6 Monate beträgt.

5. Anti-CD20-Antikörper zur Verwendung nach Anspruch 4, wobei der Anti-CD20-Antikörper dem Patienten verabreicht wird, um eine vierte Anti-CD20-Antikörperexposition bereitzustellen, wobei die vierte Exposition 600 mg des Antikörpers beträgt und wobei das Intervall zwischen der dritten Exposition und der vierten Exposition 20-24 Wochen oder 5-6 Monate beträgt.

6. Anti-CD20-Antikörper zur Verwendung nach einem der Ansprüche 3-5, wobei die initiale Exposition, die 600 mg des Anti-CD20-Antikörpers beträgt, eine erste Dosis und eine zweite Dosis des Anti-CD20-Antikörpers umfasst, wobei jede Dosis 300 mg beträgt und die erste Dosis und die zweite Dosis zwei Wochen oder 14 Tage beabstandet sind.

7. Anti-CD20-Antikörper zur Verwendung nach Anspruch 6, wobei die zweite, dritte und/oder vierte Exposition eine Einzeldosis von 600 mg umfassen/umfasst.

8. Anti-CD20-Antikörper zur Verwendung nach einem der Ansprüche 3-5, wobei die initiale Exposition und die zweite, dritte und/oder vierte zusätzliche Exposition eine erste Dosis und eine zweite Dosis des Anti-CD20-Antikörpers umfassen, wobei jede Dosis 300 mg beträgt und die erste Dosis und die zweite Dosis zwei Wochen oder 14 Tage beabstandet sind.

9. Anti-CD20-Antikörper zur Verwendung nach einem der Ansprüche 1-8, wobei mit der initialen Exposition oder späteren Expositionen ein zweites Medikament verabreicht wird, wobei der Anti-CD20-Antikörper ein erstes Medikament ist.

10. Anti-CD20-Antikörper zur Verwendung nach einem der vorstehenden Ansprüche, wobei der Anti-CD20-Antikörper eine pharmazeutisch unbedenkliche Zusammensetzung ist.

11. Anti-CD20-Antikörper zur Verwendung nach einem der vorstehenden Ansprüche, wobei der Anti-CD20-Antikörper intravenös verabreicht wird.

12. Anti-CD20-Antikörper zur Verwendung nach Anspruch 11, wobei der Anti-CD20-Antikörper intravenös für jede Antikörperexposition verabreicht wird.

13. Anti-CD20-Antikörper zur Verwendung nach einem der Ansprüche 1-10, wobei der Antikörper subkutan verabreicht wird.

14. Anti-CD20-Antikörper zur Verwendung nach Anspruch 13, wobei der Anti-CD20-Antikörper subkutan für jede Antikörperexposition verabreicht wird.

## Revendications

1. Anticorps anti-CD20 pour une utilisation dans une méthode d'amélioration de la capacité fonctionnelle chez un patient humain atteint de sclérose en plaques comprenant l'administration au patient d'une quantité efficace de l'anticorps anti-CD20, dans lequel le patient présente une amélioration de la capacité fonctionnelle après le traitement ; dans lequel l'anticorps anti-CD20 est l'ocrelizumab ; dans lequel la sclérose en plaques est une sclérose en plaques primaire progressive (SEPPP) avec des lésions par coloration au gadolinium T1 ; et dans lequel l'amélioration de la capacité fonctionnelle est mesurée par le test de marche chronométrée de 25 pieds (T-25FW) ou le score EDSS.

2. Anticorps anti-CD20 pour une utilisation selon la revendication 1, dans lequel l'amélioration de la capacité fonctionnelle est mesurée par le test de marche chronométrée de 25 pieds (T-25FW) et le score EDSS.

3. Anticorps anti-CD20 pour une utilisation selon la revendication 1 ou 2, dans lequel l'anticorps anti-CD20 est administré au patient pour fournir une exposition initiale à l'anticorps anti-CD20 suivie d'une deuxième exposition à l'anticorps anti-CD20, dans lequel l'exposition initiale et la deuxième exposition sont chacune de 600 mg de l'anticorps, et dans lequel l'intervalle entre l'exposition initiale et la deuxième exposition est de 20 à 24 semaines ou de 5 à 6 mois.

4. Anticorps anti-CD20 pour une utilisation selon la revendication 3, dans lequel l'anticorps anti-CD20 est administré au patient pour fournir une troisième exposition à l'anticorps anti-CD20, dans lequel la troisième exposition est de 600 mg de l'anticorps, et dans lequel l'intervalle entre la deuxième exposition et la troisième exposition est de 20 à 24 semaines ou de 5 à 6 mois.

5. Anticorps anti-CD20 pour une utilisation selon la revendication 4, dans lequel l'anticorps anti-CD20 est administré au patient pour fournir une quatrième exposition à l'anticorps anti-CD20, dans lequel la quatrième exposition est de 600 mg de l'anticorps, et dans lequel l'intervalle entre la troisième exposition et la quatrième exposition est de 20 à 24 semaines ou de 5 à 6 mois.

6. Anticorps anti-CD20 pour une utilisation selon l'une quelconque des revendications 3 à 5, dans lequel l'exposition initiale, qui est de 600 mg de l'anticorps anti-CD20, comprend une première dose et une seconde dose de l'anticorps anti-CD20, dans lequel chaque dose est de 300 mg et la première dose et la seconde dose sont séparées de deux semaines ou de 14 jours.

7. Anticorps anti-CD20 pour une utilisation selon la revendication 6, dans lequel les deuxième, troisième et/ou quatrième expositions comprennent une dose unique de 600 mg.

8. Anticorps anti-CD20 pour une utilisation selon l'une quelconque des revendications 3 à 5, dans lequel l'exposition initiale et les deuxième, troisième et/ou quatrième expositions supplémentaires comprennent une première dose et une seconde dose de l'anticorps anti-CD20, dans lequel chaque dose est de 300 mg et la première dose et la seconde dose sont séparées de deux semaines ou de 14 jours.

9. Anticorps anti-CD20 pour une utilisation selon l'une quelconque des revendications 1 à 8, dans lequel un second médicament est administré avec l'exposition initiale ou les expositions ultérieures, dans lequel l'anticorps anti-CD20 est un premier médicament.

10. Anticorps anti-CD20 pour une utilisation selon l'une quelconque des revendications précédentes, dans lequel l'anticorps anti-CD20 est dans une composition pharmaceutiquement acceptable.

11. Anticorps anti-CD20 pour une utilisation selon l'une quelconque des revendications précédentes, dans lequel l'anticorps anti-CD20 est administré par voie intraveineuse.

12. Anticorps anti-CD20 pour une utilisation selon la revendication 11, dans lequel l'anticorps anti-CD20 est administré par voie intraveineuse pour chaque exposition à l'anticorps.

13. Anticorps anti-CD20 pour une utilisation selon l'une quelconque des revendications 1 à 10, dans lequel l'anticorps est administré par voie sous-cutanée.

14. Anticorps anti-CD20 pour une utilisation selon la revendication 13, dans lequel l'anticorps anti-CD20 est administré par voie sous-cutanée pour chaque exposition à l'anticorps.
